# EUROPÄISCHE PATENTANMELDUNG

(11) **EP 3 287 528 A1**
(43) Veröffentlichungstag der Anmeldung: **28.02.2018**
(21) Anmeldenummer: 16185624.0
(22) Anmeldetag: 25.08.2016
(51) Int. Cl.: C12Q 1/68

(54) **VERFAHREN ZUR AMPLIFIKATION VON NUKLEINSÄUREN UND KIT ZU DESSEN DURCHFÜHRUNG**

(71) Anmelder: AGCT GmbH, 23562 Luebeck (DE)
(72) Erfinder: CHERKASOV, Dmitry, 35039 Marburg (DE); BASLER, Norbert, 22927 Groß Hansdorf (DE); BECKER, Claus, 76470 Ötigheim (DE); HEß, Hans-Jörg, 13467 Berlin (DE); MÜLLER-HERMANN, Andreas, 80796 München (DE)
(74) Vertreter: Keller, Günter

(57) **Zusammenfassung**

Offenbart wird ein Verfahren zur Amplifikation von Nukleinsäuren, bei dem im Wesentlichen davon Gebrauch gemacht wird, dass eine vordefinierte Nukleinsäurekette (Zielsequenz) in Anwesenheit eines zielsequenzspezifischen Aktivator-Oligonukleotides vervielfältigt / amplifiziert werden kann. Das zielsequenzspezifische Aktivator-Oligonukleotid bewirkt die Trennung von neu synthetisierten komplementären Primer-Verlängerungsprodukten mittels Strangverdrängung, so dass sich an dem jeweiligen Matrizenstrang ein neues Primer-Oligonukleotid anlagern kann. Der so gebildete Komplex aus einem Primer-Oligonukleotid und einem Matrizenstrang kann eine neue Primer-Verlängerungsreaktion initiieren. Die so gebildeten Primer-Verlängerungsprodukte dienen wiederum als Matrizen, so dass eine exponentiell ablaufende Amplifikationsreaktion resultiert.

Das Aktivator-Oligonukleotid dient dabei selbst nicht als Matrize und ist vorzugsweise gegenüber einer Primer-Verlängerungs-Reaktion inert. Das Aktivator-Oligonukleotid dient als Kofaktor in der Reaktion, welcher den für die Reaktion notwendigen einzelsträngigen Zustand der relevanten Strang-Segmente der Matrizen regeneriert.

Durch Verwendung eines Komponenten-Set, umfassend mindestens ein Primer-Oligonukleotid, mindestens eine Polymerase, ein Set von Substraten für Polymerase (z.B. dNTPs) und mindestens ein spezifisches Aktivator-Oligonukleotid kann eine zu amplifizierende Nukleinsäure linear amplifiziert werden.

Durch Verwendung eines solchen Sets und eines zweiten Primer-Oligonukleotids, kann eine zu amplifizierende Nukleinsäure exponentiell amplifiziert werden, wobei resultierende spezifische Primer-Verlängerungsprodukte für weitere Primer-Verlängerungsreaktionen als Matrizen verwendet werden.

## Beschreibung

Die Synthese von Nukleinsäureketten nimmt heute eine zentrale Rolle in der Biotechnologie ein. Verfahren wie PCR haben sowohl die Forschungslandschaft als auch industrielle Applikationsfelder, wie Diagnostik in der Medizin und Lebensmittelindustrie, signifikant weiterentwickelt. Die Verbindung der PCR mit anderen Technologien, wie Sequenzierung, Real-Time-Nachweis, Microarray-Technologie, Mikrofluidic-Managment etc. hat zur technologischen Entwicklung der Basistechnologie beigetragen und einige Barrieren der Grundtechnologie der PCR teilweise überwinden können. Es wurden auch weitere Amplifikations-Verfahren, wie isothermale Amplifikationstechniken (LAMP, HDA, RPA, TMA, SDA etc.) entwickelt. Deren Einsatz wurde besonders für Bereich von POCT vorgesehen.

Trotz enormer Fortschritte auf diesem Gebiet, nimmt PCR die zentrale Rolle ein und bestimmt somit die einzelnen technologischen Barrieren der Applikationen.

Eine Aufgabe der vorliegenden Erfindung ist es, Verfahren und Komponenten bereitzustellen, welche die enzymatische Synthese und Amplifikation von Nukleinsäureketten ermöglichen. Es soll ein neues enzymatisches Verfahren und Komponenten für die Synthese von Nukleinsäureketten, sowie die Amplifikation von Nukleinsäureketten bereitgestellt werden.

Mit dem erfindungsgemäßen Verfahren sollen Nukleinsäureketten mit definierter SequenzZusammensetzung synthetisiert bzw. amplifiziert werden können. Dies wird erfindungsgemäß durch Einsatz von Aktivator-Oligonukleotide mit vordefinierten Sequenzen erreicht, welche vorzugsweise mittels einer sequenzabhängigen nukleinsäurevermittelten Strangverdrängung einen neusynthetisierten Doppelstrang bestehend aus beiden Primer-Verlängerungsprodukten trennen. Die dabei entstehenden einzelsträngigen Segmente von Primer-Verlängerungsprodukten können als Bindungsstellen für weitere Primer-Oligonukleotide dienen, so dass eine exponentielle Amplifikation von zu amplifizierenden Nukleinsäureketten erreicht wird. Die Primer-Verlängerungsreaktionen und Strangverdrängungsreaktionen finden vorzugsweise im Ansatz gleichzeitig statt. Die Amplifikation erfolgt vorzugsweise unter Reaktionsbedinungen, welche eine spontane Trennung von beiden synthetisierten Primer-Verlängerungsprodukten nicht zulassen.

### Begriffe und Definitionen:

Im Rahmen der vorliegenden Erfindung haben die verwendeten Begriffe folgende Bedeutung:
Der Begriff "Oligonukleotid" wie er hier bezugnehmend auf Primer, Sonden, nachzuweisende oligomere Fragmente, Kontroll-Oligomere und unmarkierte Blockierungs-Oligomere verwendet wird, ist als ein Molekül definiert, das zwei oder mehr, bevorzugt mehr als drei Desoxyribonukleotide und / oder Ribonukleotide und / oder Nukleotid-Modifikationen und / oder Nicht-Nukleotid-Modifikationen enthält. Seine genaue Größe hängt von vielen Faktoren ab, die ihrerseits von der letztendlichen Funktion oder Verwendung der Oligonukleotide abhängen.

Der Begriff "Primer", wie er hier verwendet wird, betrifft ein Oligonukleotid, unabhängig davon, ob es natürlicherweise z. B. in einer gereinigten Restriktionsspaltung vorkommt oder synthetisch produziert wurde. Ein Primer ist fähig, als Initiationspunkt der Synthese zu wirken, wenn er unter Bedingungen eingesetzt wird, bei denen die Synthese eines zu einem Nukleinsäurestrang komplementären Primer-Verlängerungsproduktes induziert wird, d. h. in Gegenwart von Nukleotiden und einem induzierenden Agens, wie z. B. DNA- Polymerase bei einer geeigneten Temperatur und einem geeigneten pH-Wert. Der Primer ist für eine maximale Wirksamkeit bei der Amplifikation bevorzugt einzelsträngig. Der Primer muss genügend lang sein, um in Gegenwart des induzierenden Agens die Synthese des Verlängerungsproduktes zu initiieren. Die genaue Länge des Primers hängt von vielen Faktoren ab, einschließlich der Temperatur und der Primerquelle und der Anwendung des Verfahrens. Beispielsweise beträgt die Länge der Oligonukleotid-Primer bei diagnostischen Anwendungen, je nach Komplexität der Zielsequenz zwischen 5 bis 100 Nukleotide, vorzugsweise 6 bis 40 und besonders bevorzugt 7 bis 30 Nukleotide. Kurze Primer-Moleküle erfordern im allgemeinen niedrigere Temperaturen, um genügend stabile Hybridkomplexe mit der Matrize zu bilden, oder höhere Konzentrationen von anderen Reaktionskomponenten, beispielsweise von DNA-Polymerasen.

Die hier verwendeten Primer sind so ausgewählt, das sie "im wesentlichen" komplementär zu den verschiedenen Strängen jeder spezifischen zu amplifizierenden Sequenz sind. Das bedeutet, dass die Primer genügend komplementär sein müssen, um mit ihren betreffenden Strängen zu hybridisieren. Somit braucht die Primersequenz nicht die genaue Sequenz der Matrize wiederzuspiegeln. Zum Beispiel kann ein nicht-komplementäres Nukleotidfragment an dem 5'- Ende des Primers angeheftet sein, wobei der Rest der Primersequenz komplementär zu dem Strang ist. In einer anderen Ausführungsform können nichtkomplementäre Basen oder längere Sequenzen in den Primer eingefügt sein, vorausgesetzt, dass die Primersequenz eine genügend große Komplementarität mit der zu amplifizierenden Sequenz des Stranges aufweist, um damit zu hybridisieren und so eine Matrize zur Synthese des Verlängerungsproduktes des anderen Primers zu erzeugen.

Wenn die Ausgangsnukleinsäure die zu amplifizierende Sequenz darstellt, ist das hergestellte Primer-Verlängerungsprodukt vollständig zu den Strängen der Ausgangsnukleinsäure komplementär.

Wenn die komplementären Stränge der zu amplifizierenden Nukleinsäure voneinander getrennt sind, können die Stränge unabhängig davon, ob die Nukleinsäure ursprünglich doppel- oder einzelsträngig vorlag, zum Gebrauch als Matrize für die Synthese zusätzlicher Nukleinsäurestränge dienen.

Die Desoxyribonukleosid-Triphosphate (dNTPs) dATP, dCTP, dGTP und TTP werden in adäquaten Mengen ebenfalls zum Synthesegemisch gegeben. In einer Ausführungsform können zusätzlich zu dNTPs mindesten eine weitere Art von dNTP-Analoga zum Synthesegemisch zugegeben werden. Diese dNTP-Analoga umfassen in einer Ausführungsform beispielsweise eine charakteristische Markierung (z.B. Biotin oder Fluoreszenzfarbstoff), so dass wenn in Nukleinsäurestrang eingebaut, auch diese Markierung in den Nukleinsäurestrang integriert ist. In einer anderen Ausführungsform umfassen diese dNTP-Analoga mindestens eine Modifikation von Zucker-Phosphat-Anteil des Nukleotids, z.B. alpha-Phosphorothioat-2'-Desoxyribonukleosid-Triphosphate (oder andere Modifikationen, welche einem Nukleinsäurestrang eine Nuklease-Resistenz verleihen), 2',3'-Dideoxy-Ribonukleosid-Triphosphate, Azyklo-Nukleosid-Triophosphate (oder andere zu Termination einer Synthese führende Modifikationen). In einer weiteren Ausführungsform umfassen diese dNTP-Analoga mindestens eine Modifikation von Nukleobase, z.B. Iso-Cytosine, Iso-Guanosine (oder auch andere Modifikationen der Nukleobasen des erweiterten genetischen Alphabets), 2-Amino-Adenosine, 2-Thiouridine, Inosine, 7-deazy-adenosine, 7-deaza-guanosine, 5-Me-Cytosine, 5-Propyl-Uridine, 5-Propyl-Cytosine (oder auch andere Modifikationen von Nukleobasen, welche gegenüber natürlichen Nukleobasen durch eine Polymerase eingebaut werden können und zur Änderung der Strang-Stabilität führen). In einer weiteren Ausführungsform umfasst ein dNTP-Analog sowohl eine Modifikation der Nukleobase als auch eine Modifikation des Zucker-Phosphat-Anteils. In einer weiteren Ausführungsform wird statt mindestens eines natürlichen dNTP-Substrates mindesten einer weiteren Art von dNTP-Analoga zum Synthesegemisch zugegeben werden.

Das die Nukleinsäure-Synthese induzierende Agens kann eine Enzyme einschließende Verbindung oder ein System sein, das so wirkt, dass dadurch die Synthese der Primer-Verlängerungsprodukte bewirkt wird. Geeignete Enzyme für diesen Zweck beinhalten z. B. DNA-Polymerasen wie Bst Polymerase und ihre Modifikationen, Vent Polymerase und andere - bevorzugt hitzestabile DNA-Polymerasen, die die Einbau der Nukleotide in der korrekten Weise ermöglichen, wodurch die Primer-Verlängerungsprodukte gebildet werden, die zu jedem Nukleinsäurestrang komplementär sind. Im Allgemeinen wird die Synthese am 3'- Ende eines jeden Primers initiiert und schreitet dann in 5'- Richtung entlang des Matrizenstranges fort, bis die Synthese beendet ist.

### Reaktionsbedinungen

Reaktionsbedinungen umfassen unter anderem Puffer-Bedinungen, Temperatur-Bedingungen, Zeitdauer der Reaktion und Konzentrationen von jeweiligen Reaktionskomponenten.

Im Verlauf der Reaktion akkumuliert die Menge der spezifischen produzierten zu amplifizierenden Nukleinsäure in einer exponentiellen Weise. Die Reaktion umfassend die Synthese der Verlängerungsprodukte kann zur Produktion der gewünschten Menge der spezifischen Nukleinsäuresequenz so lange wie nötig durchgeführt werden. Das erfindungsgemäße Verfahren wird bevorzugt kontinuierlich durchgeführt. In bevorzugter Ausführungsform läuft die Reaktion bei gleicher Reaktionstemperatur ab, wobei die Temperatur bevorzugt zwischen 60°C und 70°C liegt., In einer anderen Ausführungsform kann die Temperatur auch variabel gesteuert werden. Die für die exponentielle Amplifikation benötigten Reagenzien sind vorzugsweise bereits zu Beginn einer Reaktion im gleichen Ansatz vorhanden. In einer anderen Ausführungsform können Reagenzien auch in späteren Stadien des Verfahrens zugegeben werden.

Bevorzugt werden keine Helicasen oder Recombinasen in der Reaktionsmischung zur Trennung der neu synthetisieren Doppelstränge der zu amplifizierenden Nukleinsäure verwendet.

In einer bevorzugten Ausführungsform beinhaltet die Reaktionsmischung keine biochemischen Energie-spendenden Verbindungen wie ATP.

Die Menge der zu Beginn der Reaktion vorliegenden zu amplifizierenden Nukleinsäure kann zwischen wenigen Kopien und mehreren Milliarden Kopien in einem Ansatz vorliegen. Bei diagnostischen Einsätzen kann die Menge der zu amplifizierenden Nukleinsäurekette unbekannt sein.

In der Reaktion können auch weitere, nicht zu amplifizierende Nukleinsäuren vorliegen. Diese Nukleinsäuren können von natürlichen DNA oder RNA oder ihren Äquivalenten abstammen. In einer Ausführungsform liegen Kontroll-Sequenzen im gleichen Ansatz vor, welche parallel zu der zu amplifizierenden Nukleinsäure ebenfalls amplifiziert werden sollen.

Bevorzugt wird ein molarer Überschuss von ungefähr 10³:1 bis ungefähr 10¹⁵:1 (Verhältnis Primer:Matrize) der eingesetzter Primer und des Aktivator-Oligonukleotides zu der Reaktionsmischung gegeben, die die getrennten Matrizen-Stränge enthält.

Es kann die Menge der Zielnukleinsäuren nicht bekannt sein, wenn das erfindungsgemäße Verfahren in diagnostischen Anwendungen benutzt wird, so dass die relative Menge des Primers und des Aktivator-Oligonukleotides bezüglich des komplementären Stranges nicht mit Sicherheit bestimmt werden kann. Die Menge des zugefügten Primers wird im Allgemeinen im molaren Überschuss bezüglich der Menge des komplementären Stranges (Matrize) vorhanden sein, wenn die zu amplifizierende Sequenz in einem Gemisch von komplizierten langkettigen Nukleinsäuresträngen enthalten ist. Ein großer molarer Überschuss wird bevorzugt, um die Effizienz des Verfahrens zu verbessern.

Die verwendeten Konzentration von Primer-1, Primer-2 und Aktivator-Oligonukleotide liegen beispielswiese in Bereichen zwischen 0,1 µmol/l und 100 µmol/l, bevorzugt zwischen 1 µmol/l und 50 µmol/l, besser zwischen 1 µmol/l und 20 µmol/l. Die hohe Konzentration von Komponenten soll das Gleichgewicht der Strangverdrängungsreaktion günstig beeinflussen und damit die Geschwindigkeit der Amplifikation erhöhen. Der Einsatz von hohen Konzentrationen einzelner Komponenten wird besonders bei Reaktionstemperaturen bevorzugt, welche eine hohe Sequezspezifität der Amplifikation begünstigen (s.u.).

Die Konzentration von Polymerase liegt in Bereichen zwischen 0,01 µmol/l und 50 µmol/l, vorzugsweise zwischen 0,1 µmol/l und 20 µmol/l, besser zwischen 0,1µmol/l und 10 µmol/l.

Die Konzentration von einzelnen dNTP-Substraten liegt in Bereichen zwischen 10 µmol/l und 10 mmol/l, vorzugsweise zwischen 50 µmol/l und 2 mmol/l, besser zwischen 100 µmol/l und 1 mmol/l. Die Konzentration von dNTP kann die Konzentration von divalenten Metal-Kationen beeinflußen. Gegebenenfalls wird diese entsprechend angepasst.

Als divalte Metal-Kationen werden beispielsweise Mg2+ verwendet. Als entsprechendes Anion können beispielsweise Cl, Acetat, Glutamat etc. verwendet werden.

Diese Synthese erfolgt im Allgemeinen in einer gepufferten wässrigen Lösung. Als PufferLösungen können gelöste konventionelle Puffer-Substanzen, wie Tris-HCl, Tris-Acetat, Kalium-Glutamat, Natrium-Glutamat in gängigen Konzentrationen verwendet werden. Der pH-Wert dieser Lösungen liegt üblicherweise zwischen 7 und 9,5, bevorzug etwa bei 8 bis 8,5. Die Puffer-Bedingungen können beispielsweise nach Empfehlung des Herstellers der verwendeten Polymerase angepasst werden.

Weitere Substanzen, wie Tm-Depressoren (z.B. DMSO, Betaine, TPAC) etc. können zum Puffer zugegeben werden. Auch Polymerase-stabilisierende Komponenten, wie Tween 20 oder Triton 100 können in üblichen Mengen zum Puffer zugegeben werden. EDTA oder EGTA kann zu Komplexierung von Schwermetallen in konventionellen Mengen zugegeben werden.

Vorzugsweise enthält die Reaktionsmischung keine Inhibitoren der Strangverdrängungsreaktion und keine Inhibitoren einer Polymerasenabhängigen Primer-Verlängerung.

In einer Ausführungsform enthält die Reaktionsmischung DNA-bindende Farbstoffe, bevorzugt interkalierenden Farbstoffe, wie z.B. EvaGreen oder SybrGreen.

Die Reaktionsmischung kann weiterhin Proteine bzw. andere Substanzen enthalten, welche beispielsweise aus einem Original-Material stammen und welche die Amplifikation vorzugsweise nicht beeinflussen.

### Temperatur-Bedingungen

Die Temperatur hat einen wesentlichen Einfluß auf die Stabilität der Doppelstränge. Vorzugsweise werden während der Amplifikationsreaktion keine Temperaturbedinungen verwendet, welche im Wesentlichen zur Trennung von Doppelsträngen der zu amplifizierenden Nukleinsäure in Abwesenheit von Aktivator-Oligonukleotid führen.

Bei Temperatur in Höhe von Tm der zu amplifizierenden Nukleinsäure kann es zu einer spontanen Trennung von beiden Strängen der zu amplifizierenden Nukleinsäure kommen, so dass Einfluss von Aktivator-Oligonukleotids auf die Sequenzspezifität der Amplifikation minimal bis begrenzt ist.

Bei einer Amplifikation, welche wenig sequenzspezifisch (d.h. wenig Aktivator-Oligonukleotid-abhängig) ablaufen muss, kann die Reaktionstemperatur beispielsweise um die Schmelztemperatur (d.h. Tm plus / minus 3°C) der zu amplifizierenden Nukleinsäure liegen, wobei die Tm in gleichem Reaktionspuffer gemessen wurde wie die Amplifikation.

Auch bei Temperatur-Bereich von ca. (Tm minus 3°C) bis ca. (Tm minus 10°C) kann es immer noch zu einer spontanen Strangtrennung kommen, obwohl mit geringerer Effizienz. Der Einfluss von Aktivator-Oligonukleotid auf die Sequenzspezifität der zu amplifizierenden Nukleinsäure ist höher als bei Temperaturbedinungen um die Tm der zu amplifizierenden Nukleinsäure. Zwar findet die Strangtrennung mit sinkender Reaktionstemperatur im Wesentlichen dank Interaktion des Doppelstranges mit Aktivator-Oligonukleotid statt, allerdings können Duplexe unter genannten Bedinungen auch spontan zerfallen, d.h. ohne sequenzabhängigen Strangverdrängung durch Aktivator-Oligonukleotid. Beispielsweise liegt die Reaktionstemperatur bei einer vermindert sequenzspezifischen Amplifikation in Bereichen zwischen ca. (Tm minus 3°C) und ca. (Tm minus 10°C), bevorzugt zwischen ca. (Tm minus 5°C) und ca. (Tm minus 10°C),

Eine hohe Sequenzspezifität der Amplifikation des Verfahrens wird vor allem dann erreicht, wenn die neu synthetisierten Stränge der zu amplifizierenden Nukleinsäure unter Reaktionsbedinungen nicht spontan in Einzelstränge dissoziieren können. In einem solchen Fall ist allein die sequenzspezifische Strangverdrängung durch Aktivator-Oligonukleoid für eine sequenzspezifische Strangtrennung verantwortlich. Dies kann im allgemeinen erreicht werden, wenn die Reaktionstemperatur deutlich unter der Schmelztemperatur von beiden Strängen der zu amplifizierenden Nukleinsäure liegt und keine weiteren Komponenten für eine Strangtrennung verwendet werden, beispielsweise keine Helicasen oder Recombinasen. Beispielsweise liegt die Reaktionstemperatur bei einer sequenzspezifischen Amplifikation in Bereichen zwischen ca. (Tm minus 10°C) und ca. (Tm minus 50°C), bevorzugt zwischen ca. (Tm minus 15°C) und ca. (Tm minus 40°C), besser zwischen ca. (Tm minus 15°C) und ca. (Tm minus 30°C).

Aufgrund einer Vielzahl von Faktoren, welche die Tm eines Doppelstranges beeinflussen können (z.B. Sequenzlänge, CG-Gehalt der Sequenz, Puffer-Bedinungen, Konzentration von divalenten Metal-Kationen etc.) sollte die Bestimmung der Tm einer zu amplifizierenden Nukleinsäure unter gleichen Bedinungen erfolgen, wie die beabsichtigte Amplifikationsreaktion. Die Tm kann bespielsweise gemessen werden, wenn beide komplementäre Primer-Verlängerungsprodukte in Konzentration von 1 µmol/l vorliegen.

Die Reaktionstemperatur der Amplifikationsreaktion kann im Bereich von 35°C bis 85°C, besser im Bereich von 50°C und 75°C, bevorzugt im Bereich von 55°C bis 70°C liegen.

Im nachfolgend beschriebenen Beispiel 2 und 3 wurde Reaktionstemperatur der Amplifikationsreaktion von 65°C verwendet, wobei die Tm der zu amplifizierenden Nukleinsäuren zwischen ca. 75°C und ca. 80°C lag. Somit war der Doppelstrang der zu amplifizizierenden Nukleinsäure unter Reaktionsbedinungen stabil und die Amplifikationsreaktion sequenzspezifisch (siehe Beispiel 3).

Bevorzugt wird ein Amplifikationsverfahren bei welchem die Strangverdrängung sowie Primer-Verlängerungsreaktion vorzugsweise gleichzeitig bzw. parallel stattfinden und unter gleichen Reaktionsbedinungen erfolgen.

In einer Ausführungsform wird das Amplifikationsverfahren unter isothermalen Bedingungen durchgeführt, d.h. dass keine oder keine großen Temperaturänderungen für die Ausführung des Verfahrens erforderlich sind.

Die im Rahmen der Erfindung verwendeten Begriffe werden nachfolgend näher erläutert:

### Erstes Primer-Oligonukleotid:

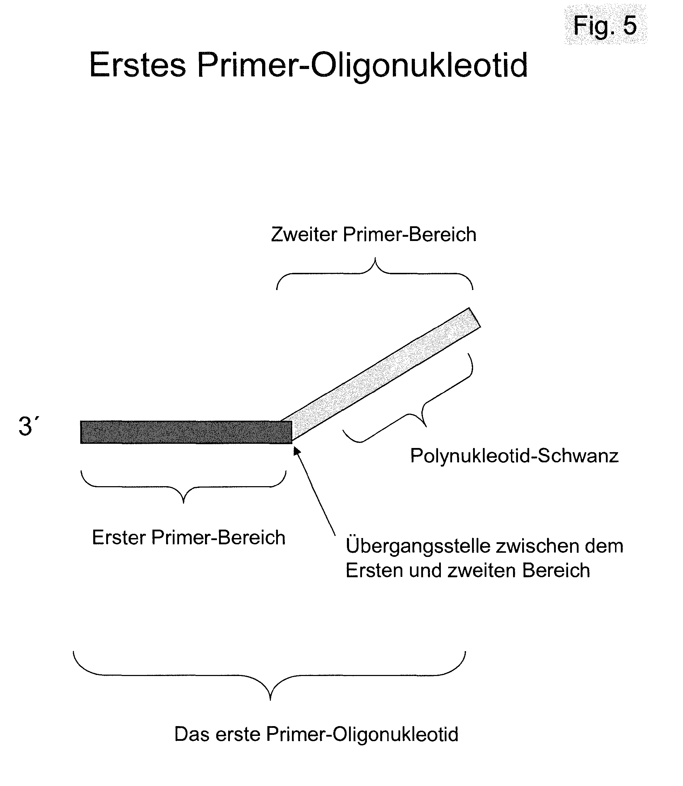

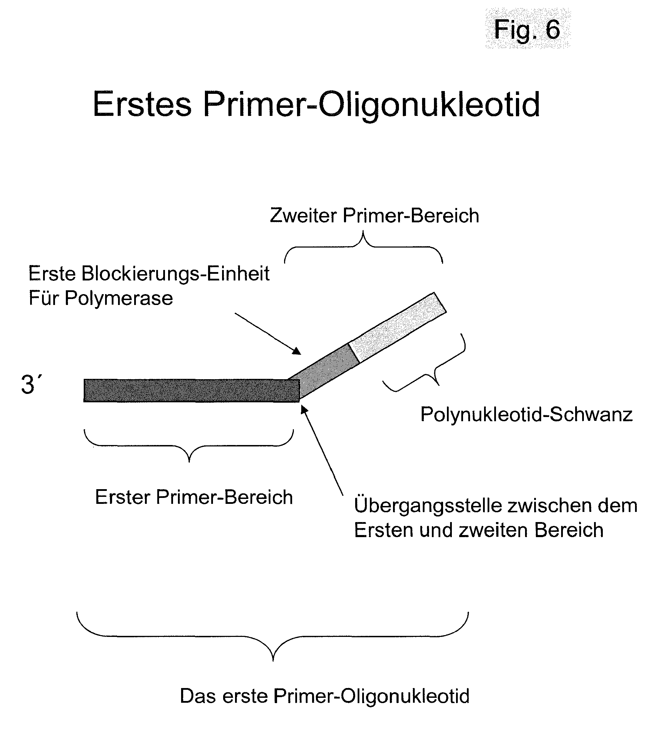

Das erste Primer-Oligonukleotid (Fig. 5 und 6) umfasst einen ersten Primer-Bereich und einen zweiten Bereich. Der erste Primer-Bereich ist in der Lage, an eine im wesentlichen komplementäre Sequenz innerhalb der zu amplifizierenden Nukleinsäure oder ihrer Äquivalente zu binden und eine Primer-Verlängerungsreaktion zu initiieren. Der zweite Bereich umfasst einen Polynukleotid-Schwanz, welcher in der Lage ist, ein Aktivator-Oligonukleotid zu binden und damit eine räumliche Nähe zwischen dem Aktivator-Oligonukleotid und den anderen Teilen des ersten Primer-Verlängerungsproduktes zu bewirken, welche ausreichend ist, um eine Strangverdrängung durch das Aktivator-Oligonukleotid zu initiieren. Der zweite Bereich des ersten Primer-Oligonukleotids umfasst weiterhin mindestens eine Modifikation (eine Nukleotid-Modifikation oder eine nicht-Nukleotid-Modifikation), welche die Polymerase am Kopieren des Polynukleotid-Schwanzes hindert, indem die Fortführung der Polymerasenabhängigen Synthese gehemmt wird. Diese Modifikation ist beispielsweise am Übergang zwischen dem ersten und dem zweiten Bereich des ersten Primer-Oligonukleotid lokalisiert. Der erste Primer-Bereich des ersten Primer-Oligonukleotides ist folglich durch eine Polymerase kopierbar, so dass eine komplementäre Sequenz zu diesem Bereich während der Synthese des zweiten Primer-Verlängerungsproduktes von der Polymerase erzeugt werden kann. Der Polynukleotid-Schwanz des zweiten Bereichs des ersten Primer-Oligonukleotides wird vorzugsweise durch die Polymerase nicht kopiert. In einer Ausführungsform wird dies durch die Modifikation im zweitem Bereich erreicht, welche die Polymerase vor dem Polynukleotid-Schwanz stoppt. In einer weiteren Ausführungsform wird dies durch Nukleotid-Modifikationen im zweitem Bereich erreicht, wobei der gesamte Polynukleotid-Schwanz im Wesentlichen aus solchen Nukleotid-Modifikationen besteht und somit für Polymerase unkopiertbar ist.

In einer Ausführungsform ist ein jedes erstes Primer-Oligonukleotid spezifisch für je eine zu amplifizierende Nukleinsäure.

In einer Ausführungsform ist ein jedes erstes Primer-Oligonukleotid spezifisch für mindestens zwei der zu amplifizierenden Nukleinsäuren, welche jeweils im Wesentlichen unterschiedliche Sequenzen umfassen.

### Zweites Primer-Oligonukleotid:

Ein Oligonukleotid, welches mit seinem 3'-Segment in der Lage ist, an eine im wesentlichen komplementäre Sequenz innerhalb der zu amplifizierenden Nukleinsäure oder ihrer Äquivalente zu binden und eine spezifische zweite Primer-Verlängerungsreaktion zu initiieren. Dieses zweite Primer-Oligonukleotid ist somit in der Lage, an das 3'-Segment eines ersten spezifischen Primer-Verlängerungsproduktes des ersten Primer-Oligonukleotids zu binden und eine Polymerase-abhängige Synthese eines zweiten Primer-Verlängerungsproduktes zu initiieren.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden.

In einer Ausführungsform ist ein jedes zweite Primer-Oligonukleotid spezifisch für je eine zu amplifizierende Nukleinsäure.

In einer Ausführungsform ist ein jedes zweite Primer-Oligonukleotid spezifisch für mindestens zwei der zu amplifizierenden Nukleinsäuren, welche jeweils im wesentlichen unterschiedliche Sequenzen umfassen.

### Primer-Verlängerungsprodukt:

Ein Primer-Verlängerungsprodukt (auch Primer-Extensionsprodukt genannt) entsteht durch enzymatische (Polymerase-abhängige) Verlängerung eines Primer-Oligonukleotides als Ergebnis einer matrizenabhängigen Synthese, welche durch eine Polymerase katalysiert wird.

Ein Primer-Verlängerungsprodukt umfasst die Sequenz des Primer-Oligonukleotid in seinem 5'-Segment und die Sequenz des Verlängerungsproduktes (auch ExtensionsProdukt genannt), welches durch eine Polymerase in matrizenabhängiger Form synthetisiert wurde. Das durch die Polymerase synthetisierte Verlängerungsprodukt ist komplementär zum Matrizenstrang, an welchem es synthetisiert wurde.

Die Länge des Verlängerungsproduktes kann zwischen 10 und 180 Nukleotiden liegen, bevorzugt zwischen 20 und 120 Nukleotiden, besser zwischen 30 und 80 Nukleotiden.

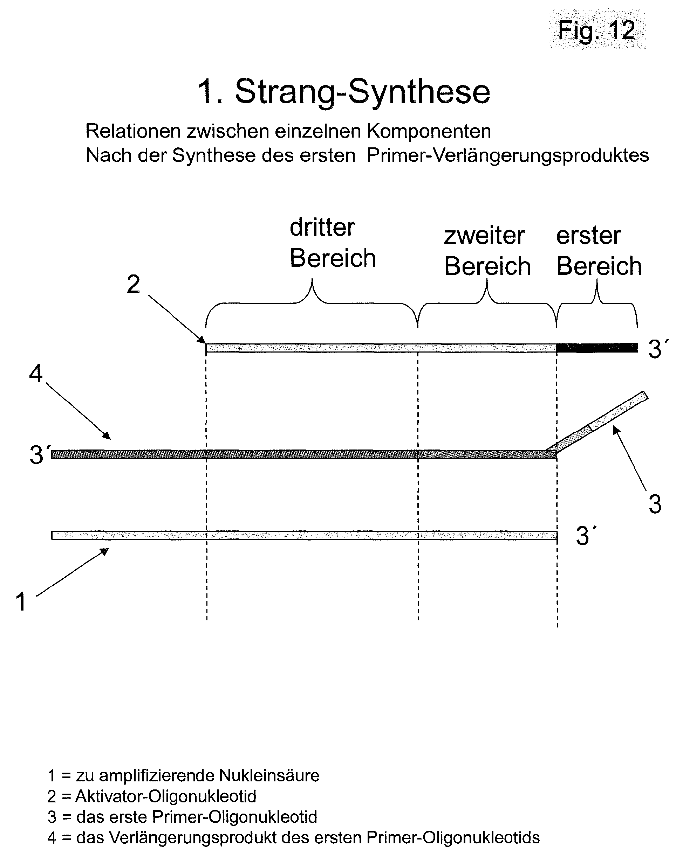

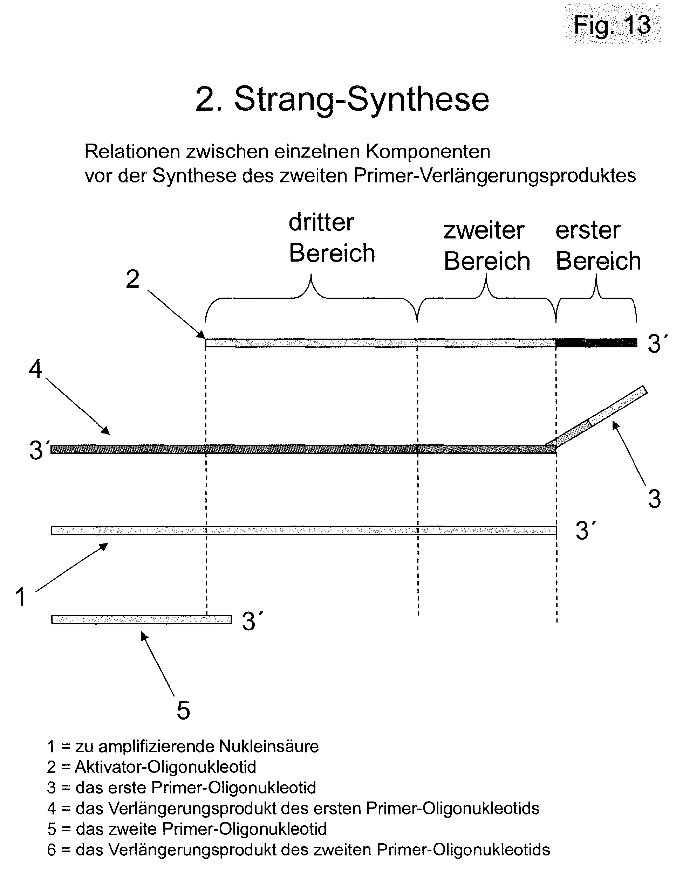

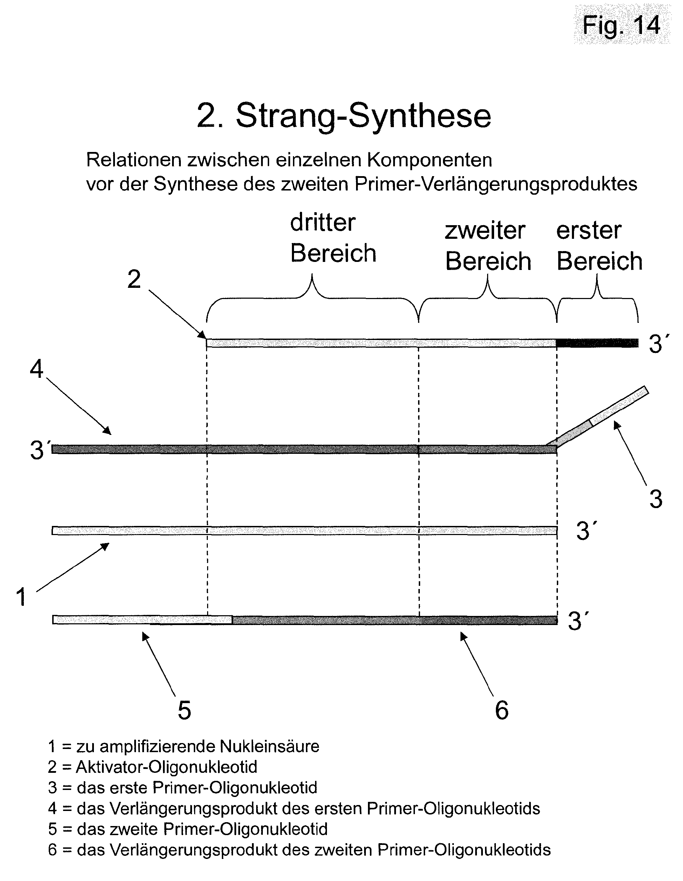

Ein spezifisches Primer-Verlängerungsprodukt (Fig. 12 bis 14) umfasst Sequenzen der zu amplifizierenden Nukleinsäure. Es ist Ergebnis einer spezifischen Synthese bzw. einer korrekten Ausführung einer beabsichtigten Primer-Verlängerungsreaktion bei welcher die spezifisch zu amplifizierende Nukleinsäure als Matrize dient. In einer bevorzugten Ausführungsform stimmt die Sequenz der synthetisierten Primer-Verlängerungsprodukte mit der zu erwartenden Sequenz einer zu amplifizierenden Nukleinsäure komplett überein. In einer anderen Ausführungsfrom können Abweichungen in der erhaltenen Sequenz von der theoretisch zu erwartenden Sequenz toleriert werden. Das Ausmaß der Übereinstimmung der erhaltenen Sequenz infolge einer Amplifikation mit der Sequenz der theoretisch zu erwartenden zu amplifizierenden Nukleinsäure liegt beispielsweise zwischen 90% und 100%, bevorzugt liegt die Übereinstimmung bei über 95%, idealerweise liegt die Übereinstimmung über 98%.

Ein unspezifisches Primer-Verlängerungsprodukt umfasst Sequenzen, welche als Ergebnis einer unspezifischen bzw. inkorrekten Primer-Verlängerungsreaktion entstanden sind. Diese schließen beispielsweise Primer-Verlängerungsprodukte ein, welche als Folge eines falschen Primings oder als Folge anderer Nebenreaktionen, einschließlich polymeraseabhängigen Sequenzveränderungen wie Basen-Substitution, Deletion etc., entstanden sind. Das Ausmaß an Sequenzabweichungen von unspezifischen Primer-Verlängerungsprodukten übersteigt die Toleranzgrenze. Beispiele für unspezifische Primer-Verlängerungsprodukte bilden Primer-Dimere oder Sequenzvarianten, welche nicht der zu amplifizierenden Nukleinsäure entsprechen.

Die Beurteilung einer hinreichenden Spezifität der Amplifikation hängt häufig mit der Aufgabenstellung zusammen. In vielen Amplifikationsverfahren kann ein gewisses Maß der Unspezifität der Amplifikationsreaktion toleriert werden, solange das gewünschte Ergebnis erreicht werden kann.

### Zu amplifizierende Nukleinsäure

Die zu amplifizierende Nukleinsäure stellt eine Nukleinsäure dar, welche sequenzspezifisch oder zumindest vorwiegend sequenzspezifisch mit Hilfe der exponentiellen Amplifikation unter Einsatz von Primern und Aktivator-Oligonukleoid durch die Polymerase amplifiziert wird. Die zu amplifizierende Nukleinsäure kann einzelsträngig oder doppelsträngig zu Beginn der Reaktion vorliegen.

Die Länge der zu amplifizierenden Nukleinsäure kann zwischen 30 und 200 Nukleotiden liegen, bevorzugt zwischen 40 und 150 Nukleotiden, besonders bevorzugt zwischen 50 und 100 Nukleotiden.

Die zu amplifizierende Nukleinsäurekette kann eine oder mehrere Zielsequenzen oder ihre Äquivalente umfassen. Weiterhin kann eine zu amplifizierende Nukleinsäure die im wesentlichen einer Zielsequenz komplementären Sequenzen umfassen. Zusätzlich zu einer Zielsequenz kann die zu amplifizierende Nukleinsäure weitere Sequenzsegmente einschließen, beispielsweise Primer-Bindungsstellen.

In einer Ausführungsform entspricht die zu amplifizierende Nukleinsäure der Zielsequenz.

In einer anderen Ausführungsform bildet die Zielsequenz einen Teil der Sequenz der zu amplifizierenden Nukleinsäure. Eine solche Zielsequenz kann von 3'-Seite und / oder von 5'-Seite von weiteren Sequenzen flankiert sein. Diese weiteren Sequenzen können beispielsweise als Bindungsstellen für Primer in der Amplifikation dienen.

Die zu amplifizierende Nukleinsäure kann einzelsträngig sein oder einen komplementären Doppelstrang bilden.

Äquivalente der zu amplifizierenden Nukleinsäure umfassen Nukleinsäuren mit im wesentlichen identischen Informationsgehalt. Beispielsweise haben komplementäre Stränge einer zu amplifizierenden Nukleinsäure identischen Informationsgehalt und können als äquivalent bezeichnet werden.

### Zielsequenz

Eine Zielsequenz ist ein Segment einer zu amplifizierenden Nukleinsäurekette, welches als charakteristische Sequenz der zu amplifizierenden Nukleinsäure dienen kann. Diese Zielsequenz kann als Marker für die Anwesenheit oder Abwesenheit einer anderen Nukleinsäure dienen. Diese andere Nukleinsäure kann beispielsweise eine genomische DNA oder RNA oder Teile der genomischen DNA oder RNA (z.B. mRNA), oder Äquivalente der genomischen DNA oder RNA eines Organismus sein (z.B. cDNA, modifizierte RNA wie rRNA, tRNA etc.), oder definierte Veränderungen der genomischen DNA oder RNA eines Organismus umfassen, beispielsweise Mutationen (z.B. Deletionen, Substitutionen, Additionen), Splice-Varianten, Rearrangement-varianten (z.B. T-Zell-Rezeptor Varianten) usw. Solche andere Nukleinsäure kann als Ausgangsnukleinsäure dienen. Eine solche Nukleinsäure schließt die Zielsequenz ein. Die Zielsequenz kann einzelsträngig oder doppelsträngig sein. Sie kann mit der zu amplifizierenden Nukleinsäure im Wesentlichen identisch sein oder nur einen Teil der zu amplifizierenden Nukleinsäure darstellen.

Äquivalente der Zielsequenz umfassen Nukleinsäuren mit im wesentlichen identischen Informationsgehalt. Beispielsweise haben komplementäre Stränge einer Zielsequenz identischen Informationsgehalt und können als äquivalent bezeichnet werden.

### Aktivator-Oligonukleotid:

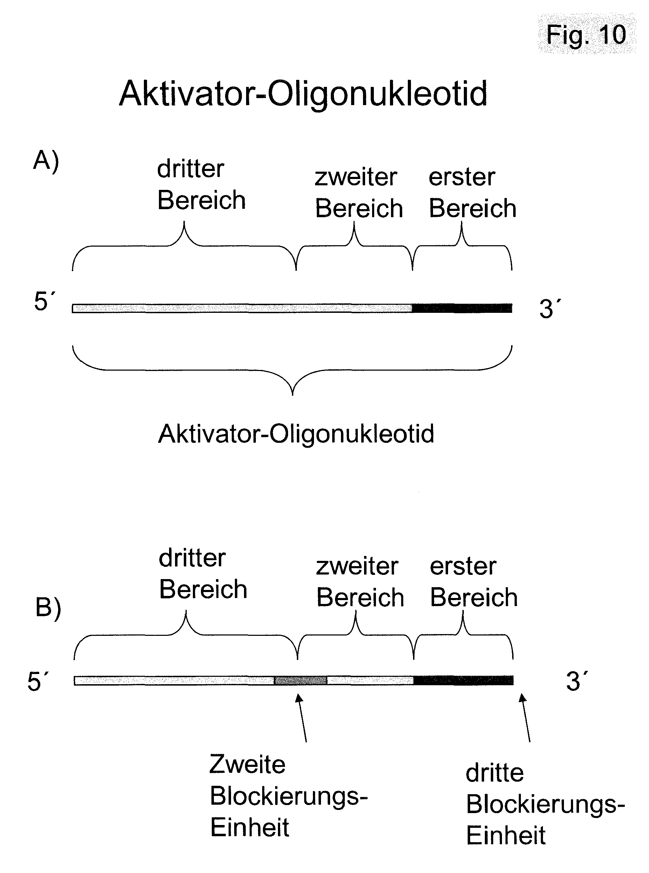

Das Aktivator-Oligonukleotid (Fig. 10) ist eine bevorzugt einzelsträngige Nukleinsäurekette, welche eine vordefinierte im wesentlichen komplementäre Sequenz einem Teil des ersten Primer-Verlängerungsprodukts einschließt, welches im Rahmen der Amplifikation der zu amplifizierenden Nukleinsäure spezifisch generiert wird. Dadurch kann das Aktivator-Oligonukleotid an das erste Primer-Oligonukleotid und mindestens an das 5'-Segment des spezifischen Verlängerungsprodukts des ersten Primer-Oligonukleotid im Wesentlichen komplementär binden. Das Aktivator-Oligonukleotid umfasst in seinen inneren Sequenzsegment in einer Ausführungsform Nukleotid-Modifikationen, welche die Polymerase daran hindern, einen komplementären Strang unter Verwendung des Aktivator-Oligonukleotides als Matrize zu synthetisieren, wenn das erste Primer-Oligonukleotid an das Aktivator-Oligonukleotid komplementär gebunden ist. Das Aktivator-Oligonukleotid ist weiterhin in der Lage, unter den gewählten Reaktionsbedingungen das zweite spezifische Primer-Verlängerungsprodukt vollständig oder teilweise aus der Bindung mit dem ersten spezifischen Primer-Verlängerungsprodukt via Strangverdrängung zu verdrängen. Dabei lagert sich das Aktivator-Oligonukleotid mit seinen komplementären Bereichen an das erste spezifische Primer-Verlängerungsprodukt an. Bei erfolgreicher Bindung zwischen dem Aktivator-Oligonukleotid und dem ersten spezifischen Primer-Verlängerungsprodukt führt dies zu Wiederherstellung eines einzelsträngigen Zustandes des 3'-ständigen Segments des zweiten spezifischen Primer-Verlängerungsproduktes, welches für die Bindung des ersten Primer-Oligonukleotides geeignet ist, so dass eine neue Primer-Verlängerungsreaktion stattfinden kann. Während der Synthese des zweiten Primer-Verlängerungsproduktes kann das Aktivator-Oligonukleotid aus der Bindung mit dem ersten Primer-Verlängerungsprodukt mittels Strangverdrängung beispielsweise durch Polymerase und/oder durch das zweite Primer-Oligonukleotid getrennt werden.

### Strangverdrängung (Strand Displacement):

Hiermit wird ein Vorgang bezeichnet, welcher durch Einwirkung eines geeigneten Mittels zu einer vollständigen oder partiellen Trennung eines ersten Doppelstranges (bestehend beispielsweise aus A1 und B1-Strang) führt, und zur gleichzeitigen / parallelen Ausbildung eines neuen zweiten Doppelstranges, wobei mindestens einer der Stränge (A1 oder B1) an der Ausbildung dieses neuen zweiten Stranges beteiligt sind. Man kann hier zwei Formen der Strangverdrängung unterschieden.

In einer ersten Form der Strangverdrängung kann die Ausbildung eines neuen zweiten Doppelstranges unter Verwendung eines bereits existierenden komplementären Stranges erfolgen, welcher zu Beginn der Reaktion im Allgemeinen in einzelsträngiger Form vorliegt. Dabei wirkt das Mittel der Strangverdrängung (beispielsweise ein vorgebildeter einzelsträngiger Strang C1, welcher eine komplementäre Sequenz zum Strang A1 aufweist, auf den ersten bereits ausgebildeten Doppelstrang (A1 und B1) und geht mit dem Strang A1 eine komplementäre Bindung ein, wodurch der Strang B1 aus der Bindung mit dem Strang A1 verdrängt wird. Falls die Verdrängung des B1 vollständig abläuft, so ist das Ergebnis der C1-Wirkung ein neuer Doppelstrang (A1:C1) und ein einzelsträngiger Strang B1. Falls die Verdrängung von B1 unvollständig abläuft, so hängt das Ergebnis von mehreren Faktoren ab. Beispielsweise kann ein Komplex aus partiell doppelsträngigen A1:B1 und A1:C1 als Zwischenprodukt vorliegen.

In einer zweiten Form der Strangverdrängung kann die Ausbildung eines neuen zweiten Doppelstranges unter einer gleichzeitig ablaufenden enzymatischen Synthese des komplementären Stranges erfolgen, wobei ein Strang des ersten vorgebildeten Doppelstranges als Matrize für die Synthese durch die Polymerase auftritt. Dabei wirkt das Mittel der Strangverdrängung (beispielsweise Polymerase mit einer Strangverdrängungs-Fähigkeit), auf den ersten bereits vorgebildeten Doppelstrang (A1 und B1) und synthetisiert einen zu Strang A1 neuen komplementären Strang D1, wobei gleichzeitig der Strang B1 aus der Bindung mit dem Strang A1 verdrängt wird.

In der wissenschaftlichen Literatur sind viele Beispiele aus der Natur und Molekular-Biologie / BioNanotechnologie beschrieben, welche den Vorgang der Strangverdrängung einschließen. Es wurden Beispiele für sowohl protein-vermittelte bzw. enzym-vermittelte Strangverdrängung als auch Bespiele für Nukleinsäureketten-vermittelte Strangverdrängung beschrieben. Ebenfalls wurden Beispiele für Strangverdrängungsvorgänge mit bereits vorgebildeten einzelsträngigen Nukleinsäuren beschrieben als auch Strangverdrängungsvorgänge, bei welchen ein neuer Strang während der Strangverdrängung erst neu synthetisiert wird.

Bei dem protein- bzw. enzym-vermittelten Strangverdrängungs-Vorgang (z.B. durch Polymerase oder Helicase vermittelt) erfolgt die Trennung einer Duplex unter Mithilfe eines Proteins oder Enzyms. Strangverdrängung mit Hilfe einer DNA-Polymerase findet beispielsweise unter gleichzeitiger Synthese eines komplementären Stranges während Strand-Displacement Amplification (SDA) statt (US Pat No.: 5,455,166 und Walker et al. PNAS 89, 1992, 392-396). Die verwendete Polymerase muss die Nukleinsäure Polymerisation bewirken und den stromabwärts gelegenen Strang verdrängen, jedoch nicht durch die eine 5'→3' Exonuklease Aktivität abbauen. Derartige Eigenschaften haben beispielsweise das Klenow-Fragment der E.coli Polymerase I, Bsu-Polymerase oder das große Fragment von Bacillus stearothermophilus (Bst Polymerase und ihre Modifikationen), sowie Vent Polymerase, DeepVent-Polymerase und andere Polymerase mit Fähigkeit zu Strangverdrängung.

Ein anderes Beispiel für Polymerase-assoziierte Strangverdrängung ist die Amplifikations-Methode LAMP (Loop mediated isothermal Amplification). Ein anderes Beispiel für protein-vermittelte bzw. enzym-vermittelte Strangverdrängung stellt die Recombinase-Reaktion dar, z.B. mit RecA. Hier führt eine Interaktion zwischen einem Protein-Nukleinsäurestrang-Komplex und einem Doppelstrang zu einer partiellen Strang-Verdrängung des Doppelstranges und Dissoziation des Protein-Nukleinsäurestrang-Komplexes. Eine Recombinase-Polymerase Amplification (RPA) macht Gebrauch von diesem Vorgang und ist beschrieben in Patentanmeldung WO 2008035205. Ein protein- bzw. enzym-vermittelter Strangverdrängungs-Vorgang geht häufig mit einem Energie-Verbrauch einher, z.B. in Form von ATP bei RPA.

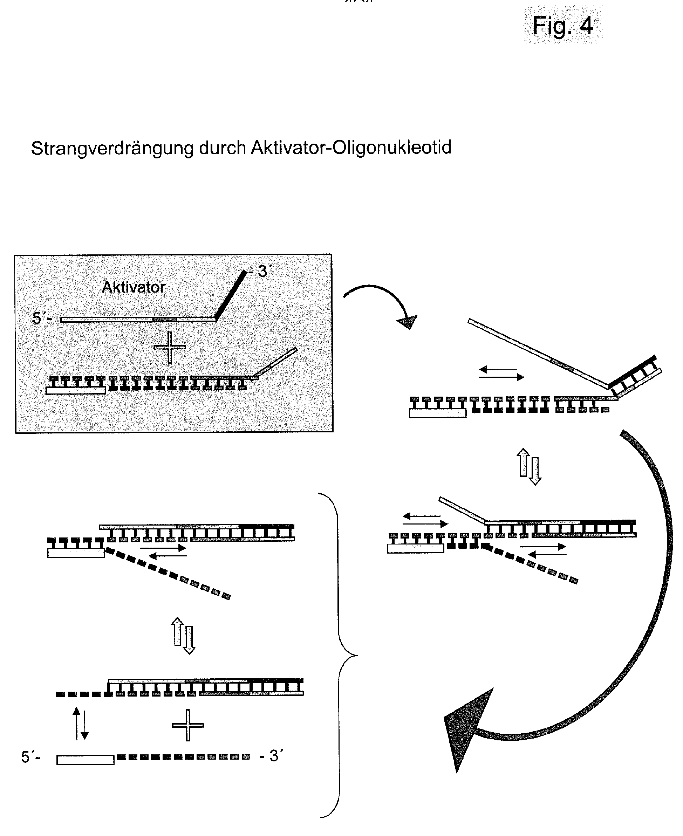

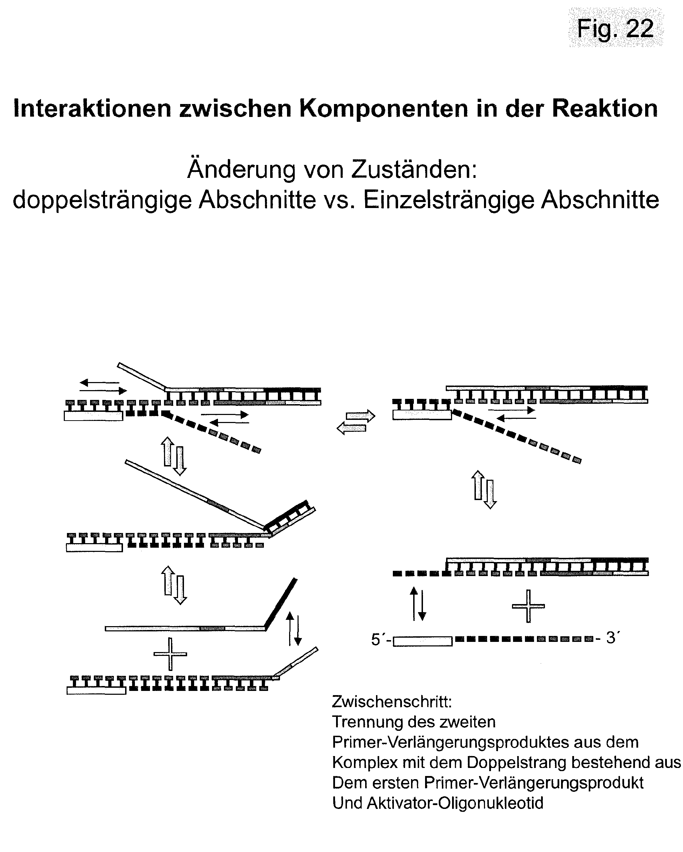

Bei einer nukleinsäurevermittelten Strangverdrängung (Fig. 4 und 22) erfolgt ein Austausch von identischen oder ähnlichen Strängen unter gleichzeitiger / parallelen Ausbildung neuer Doppelstränge. In der Literatur wird dieser Vorgang synonym als Strangersatzreaktion (engl. Strand-Replacement) bezeichnet. Dieses Phänomen ist involviert / maßgeblich beteiligt in Zweigmigration (engl. Branch-Migration) von homologen Duplexen, Überwindung von intramolekularen Haarnadel-Strukturen mittels einzelsträngiger Oligonukleotide, DNA-Klonierung, DNA-basierte "Toehold"-vermittelte Kaskadenreaktionen und SNP-Analysen. Die Natur des Phänomens der nukleinsäurebasierten bzw. nukleinsäurevermittelten Strangverdrängung wurde seit über 40 Jahren ausführlich studiert und ist in vielen Artikel abgebildet ("The Kinestics of Oligonukleotide Replacement", Reynaldo et al. J.Mol.Biol. 2000, 297, 511-; "Formation of a single base mismatch impedes spontaneous DNA branch migration" Panyutin et al. J.MoLBiol. 1993, 230, 413-; "The kinetics of spontaneous DNA branch migration" Panyutin et al. PNAS 1994, 91,2021-; "Programmable energy landscapes for Kinetic control of DNA strand displacement" Machinek et al. Nature Commun 2014, 5, 5324-; "Remote Toehold: A mechanism for flexiblecontrol of DNA hybridization Kinetics" Genot et al. JACS, 2011, 133, 2177-; "A new class of homogeneous nucleic acid probes based on specific displacement hybridization" Li et al. NAR, 20102, v30, No.2 e5; "On the biophysics and kinetics of toehold-mediated DNA strand displacement", Srinivas et al. NAR 2013, 10641-58; "Modelling toehold-mediated RNA strand displacement" Sulc et al. Biophysical Journal, 2015, 1238-; "Branch Migration through DNA sequence heterology" Biswas et al. J.Mol.Biol. 1998, 279, 795-; "Toehold-mediated nonenzymatic DNA strand displacement as a platform for DNA genotyping" Khodakov et al. JACS 2013, 135, 5612-; "Protected DNA Strand Displacement for enhanced single nucleotide discrimination in double stranded DNA" Khodakov et al. Nature, Scientific Reports, 2015, 5, 8721-; "Allele-Specific Holliday Junction Formation: a new mechanism of allelic discrimination for SNP Scoring" Yang et al. Genome Research 2003, 1754-; "Conditionally fluorescent molecular probes for detecting single base changes in double-stranded DNA" Chen et al. Nature Chemistry, 2013, 782-; "Optimizing the specificity of nucleic acid hybridization" Zhang et al. Nature Chemistry, 2012, 208-). In diesen und anderen Quellen des Standes der Technik sind strukturelle Voraussetzungen von beteiligten Nukleinsäuresträngen für einen erfolgreichen Strangverdrängungs-Vorgang, die Initiierung der Strangverdrängung, die Kinetik der Strangverdrängung, Reaktionsbedingungen sowie der Einfluss von Sequenzunterschieden in einzelnen Abschnitten der beteiligten Strukturen auf Strangverdrängung detailliert studiert und beschrieben worden. Dabei kommt der Sequenzabhängigkeit des Strangverdrängungsvorgangs eine besonders große Rolle zu. Dank der Möglichkeit, vordefinierte, spezifische Nukleinsäureketten für eine nukleinsäurevermittelte Strangverdrängung zu verwenden, ist das Ergebnis dieses Vorgangs ebenfalls sequenzspezifisch. Diese Sequenzabhängigkeit führte in Vergangenheit zum Einsatz beim Nachweis von Unterschieden in Nukleinsäureketten, unter Verwendung von Branch-Migration (dieser Vorgang ist verwandt mit Strangverdrängung und ist in Patentanmeldung beschrieben: Lishanski et al. US Pat No: 6,232,104. Verwendung von modifizierten Nukleinsäuresträngen beim Einsatz in Branch-Migration ist beschrieben in Wetmur et al US Pat No: 5,958,681.)

Nukleinsäurevermittelte Strangverdrängung wird in der Literatur auch als ein Beispiel für kompetitor-induzierte Dissoazion von molekularen Komplexen angesehen, siehe "A general mechanism for competitor-induced dissociation of molecular complexes", Paramanathan et al. Natur Commun, 2014, 5207-).

Einem Fachmann sind mehrere Formen des nukleinsäurevermittelten Strangverdrängungs-Vorgangs sind bekannt, z.B. "Toehold-Mediated" Strand Displacement oder "3-Branch-Migration".

Unter dem Begriff "nukleinsäurevermittelten Strang-Verdrängung" wird eine Summe / Reihe von Zwischenschritten zusammengefasst, welche untereinander im Gleichgewicht stehen können, und im Ergebnis zur vorübergehenden oder dauerhaften Öffnung einer ersten vorgeformten Duplex (bestehend aus komplementären Strängen A1 und B1) und Ausbildung einer neuen zweiten Duplex (bestehend aus komplementären Strängen A1 und C1), wobei A1 und C1 einander komplementär sind. Dieser Vorgang ist im Beispiel 1 veranschaulicht (s.u.).

Es ist bekannt, dass eine wesentliche strukturelle Voraussetzung für die Initiierung einer Strangverdrängung die Herbeiführung einer räumlichen Nähe zwischen einem Duplex-Ende (vorgeformter erster Duplex aus A1 und B1) und einem einzelsträngigen Strang (C1) ist, welcher die Strangverdrängung initiiert (wobei A1 und C1 einen komplementären Strang bilden können). Eine solche räumliche Nähe kann vorzugsweise mittels eines einzelsträngigen Überhanges (in der Literatur sind Beispiele mit kurzen Überhängen bekannt, im Englischen genannt "Toehold", siehe Literatur oben) herbeigeführt werden, welcher den einzelsträngigen Strang (C1) vorübergehend oder dauerhaft komplementär bindet, und somit komplementäre Seqmente des Stranges C1 und A1 in hinreichende Nähe bringt, so dass eine erfolgreiche Strangverdrängung des Stranges B1 initiiert werden kann. Die Effizienz der Initiierung der nukleinsäurevermittelten Strang-Verdrängung ist im Allgemeinen um so höher, je näher die komplementäre Segmente des Stranges A1 und C1 zueinander positioniert werden.

Eine weitere wesentliche strukturelle Voraussetzung für die effiziente Fortführung einer nukleinsäurevermittelten Strangverdrängung in inneren Segmenten liegt in hoher Komplementarität zwischen Strängen (z.B. zwischen A1 und C1), welche einen neuen Doppelstrang ausbilden müssen. So können beispielsweise einzelne Nukleiotid-Mutationen (in C1) zur Unterbrechung einer Strangverdrängung (beschrieben z.B. für Branch-Migration) führen.

Die vorliegende Erfindung macht Gebrauch von der Fähigkeit komplementärer Nukleinsäuren zu einer sequenzabhängigen nukleinsäurevermittelten Strangverdrängung.

Bevorzugte Ausführungsformen der Erfindung werden in den Figuren und Beispielen näher erläutert.

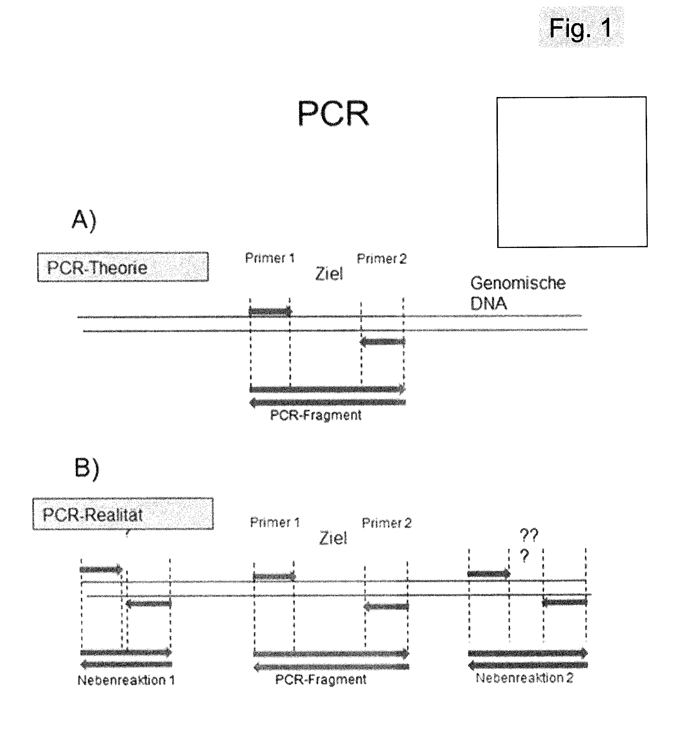

Figur 1 zeigt die Problematik bei der Nukleinsäureamplifikation durch PCR. Theoretisch sollte nur die gewünschte Zielsequenz amplifiziert werden. Da die Primer aber auch an anderen, nicht relevanten Teilen der in der Reaktionsmischung vorhandenen Nukleinsäure binden können, entstehen aufgrund von (unerwünschten) Nebenreaktionen Nebenprodukte, die das Ergebnis stören können.

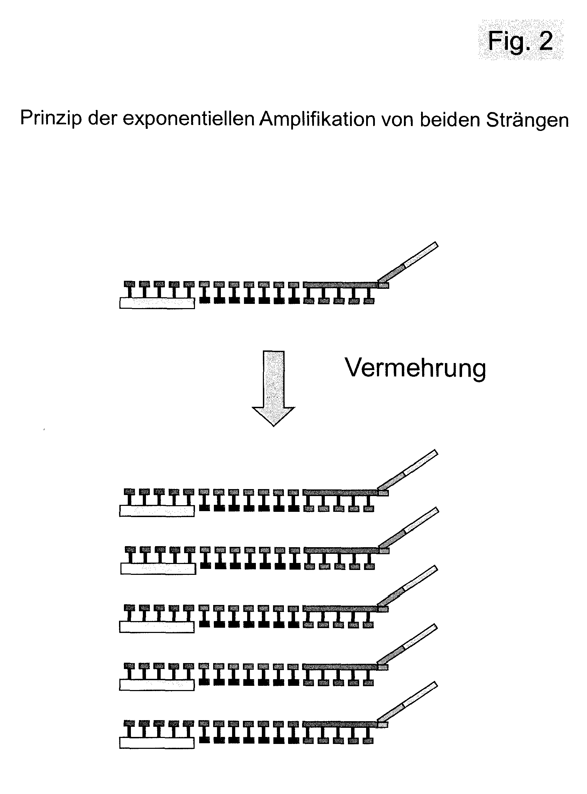

Figur 2 zeigt die Amplifikation einer zu amplifizierenden Nukleinsäure unter Einsatz des ersten Primer-Oligonukleotids und des zweiten Primer-Oligonukleotids.

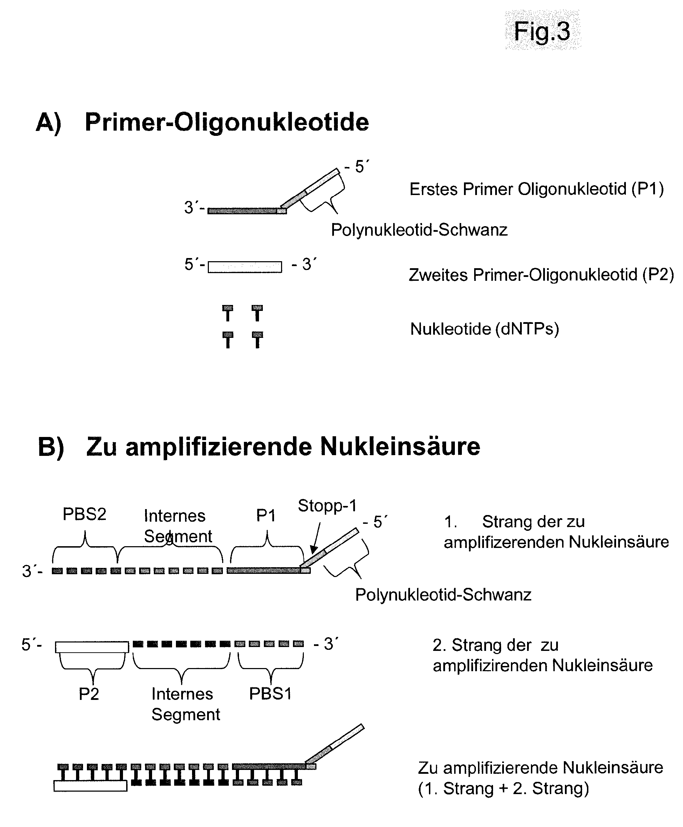

Figur 3 beschreibt Komponenten der in Fig. 2 dargestellten Strukturen
Figur 4 zeigt schematisch den Strangverdrängungsmechanismus
Figur 5 - 6 zeigt schematisch die Struktur des ersten Primer-Oligonukleotids.

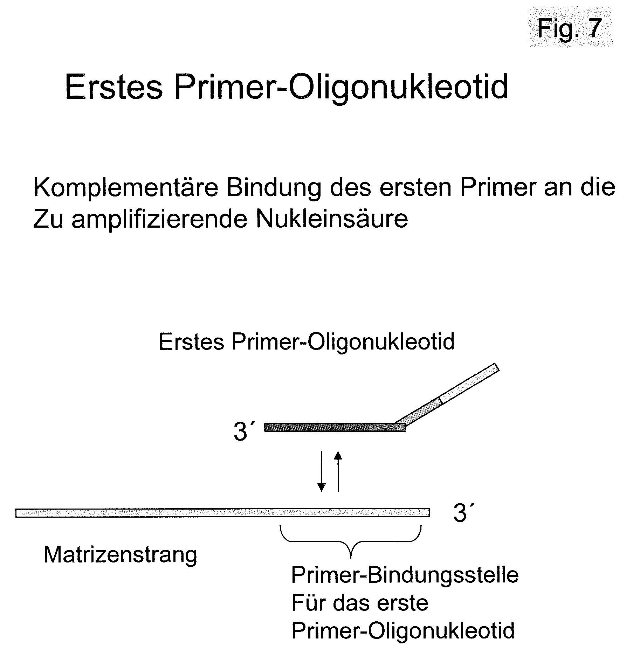

Figur 7 - 8 zeigt schematisch die Interaktion zwischen dem ersten Primer-Oligonukleotid und der Matrize, sowie die Synthese des ersten Primer-Verlängerungsprodukts.
Figur 9 zeigt schematisch die Struktur des Primer-Verlängerungsprodukts des ersten Primer-Oligonukleotids.
Figur 10 zeigt schematisch die Struktur des Aktivator-Oligonukleotids.
Figur 11 - 12 zeigt schematisch die Interaktion zwischen Strukturen während der Primer-Verlängung des ersten Primer-Oligonukleotids.
Figur 13 - 14 zeigt schematisch die Interaktion zwischen Strukturen während der Primer-Verlängung des zweiten Primer-Oligonukleotids.
Figur 15 - 21 zeigt schematisch die Interaktion zwischen einzelnen Bereichen von Komponenten während der Amplifikation.
Figur 22 zeigt schematisch das Zusammenwirken der Strukturen bei Strangverdrängung.
Figur 23 zeigt schematisch das Zusammenwirken der Strukturen während der Nukleinsäureamplifikation durch einerseits die Amplifikation von erstem Primer-Oligonukleotid und zweitem Primer-Oligonukleotid und weiterhin die Einwirkung des Aktivator-Oligonukleotids und dabei resultierende Strangverdrängung.
Fig. 24 - 29 zeigt die Ergebnisse der Strangverdrängung im Beispiel 1.
Figur 30 zeigt die Entwicklung der Signalintensität im Verlauf einer exponentiellen Amplifikation wie in Beispiel 2 beschrieben.
Figur 31 zeigt den Verlauf einer Kapillarelektrophorese nach Ablauf einer exponentiellen Amplifikation wie in Beispiel 2 beschrieben.
Figur 32 zeigt die Entwicklung einer Signalintensität im Verlauf einer erfindungsgemäßen Amplifikation, wie in Beispiel 3 beschrieben.
Figur 33 zeigt die Entwicklung einer Signalintensität im Verlauf einer erfindungsgemäßen Amplifikation, wie in Beispiel 4 beschrieben.

Die mit → 1 gekennzeichnete Reaktion entspricht dem Einsatz einer Matrize mit Perfect Match (SEQ ID NO:26) in einer Konzentration von 10 µmol/l. →2 zeigt die Reaktion mit einer Mismatch-Matrize (SEQ ID NO:27) in einer Menge von 10 µmol/l und die mit →3 gekennzeichnete Reaktion stellt die Negativkontrolle dar.

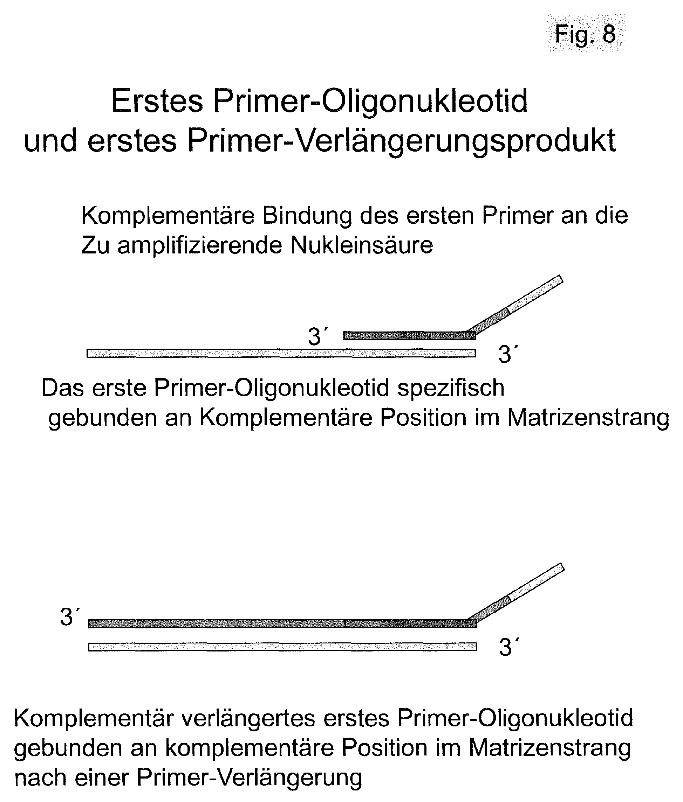

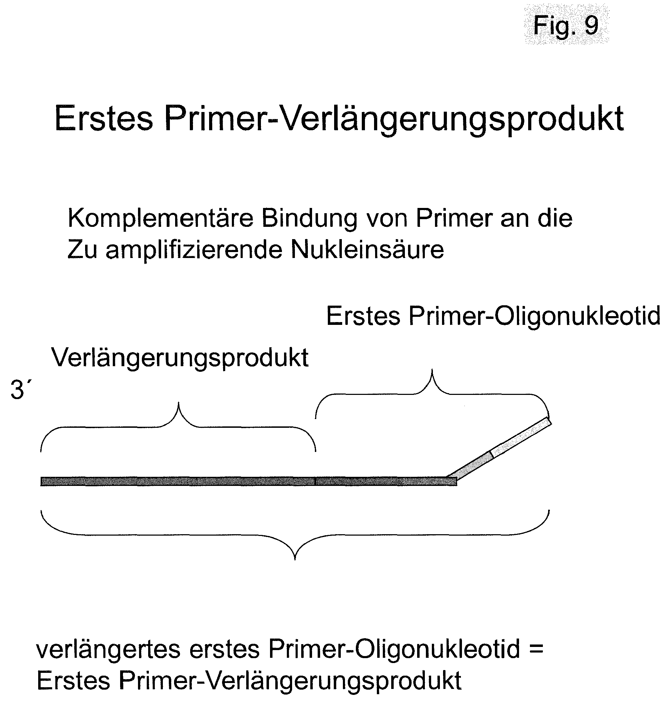

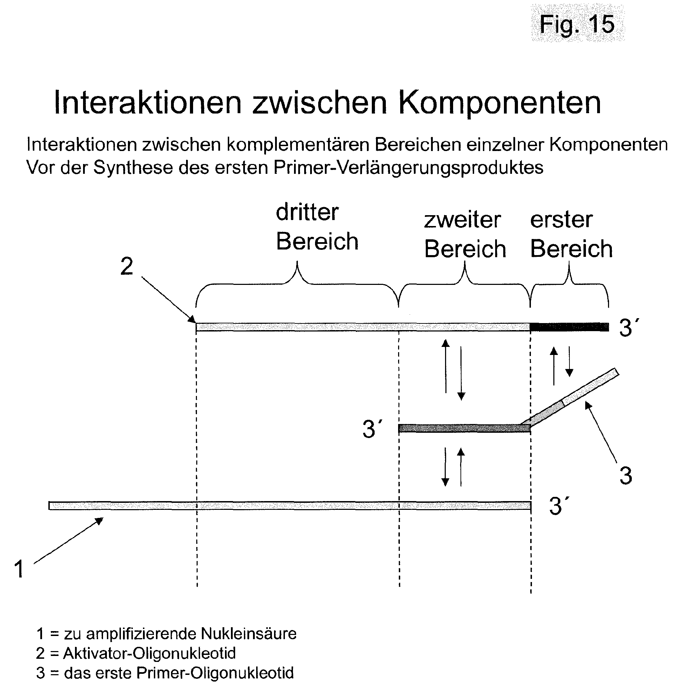

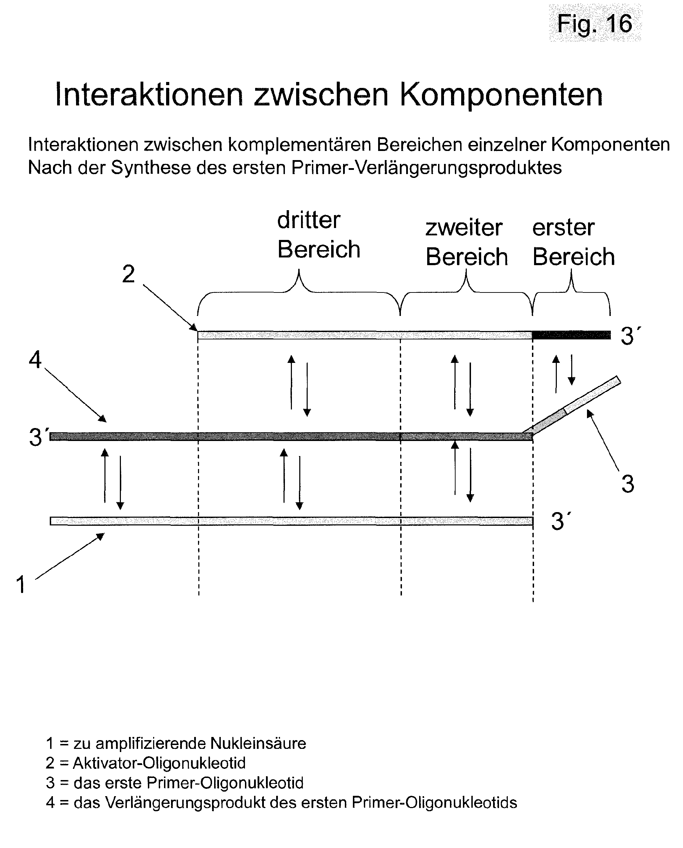

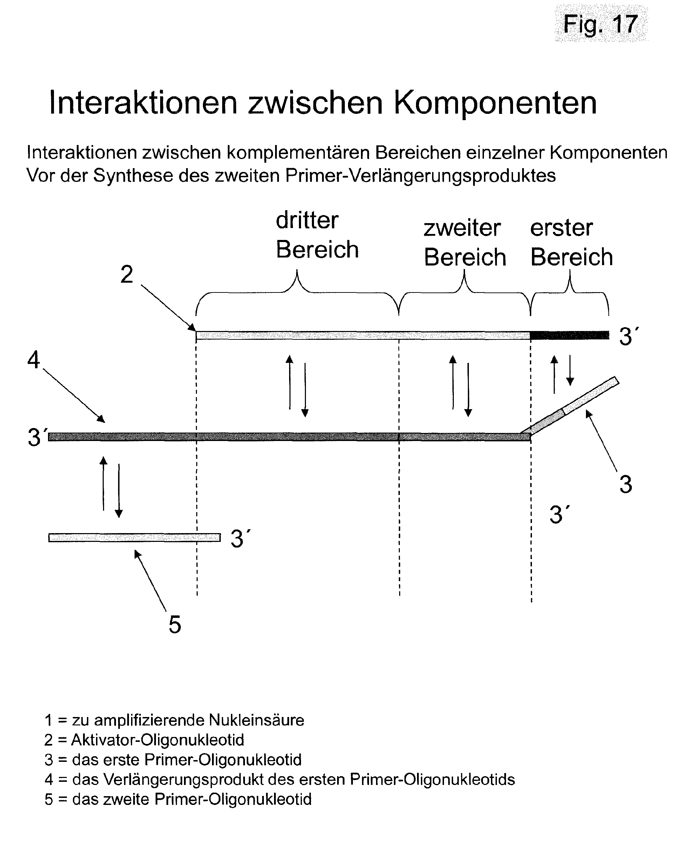

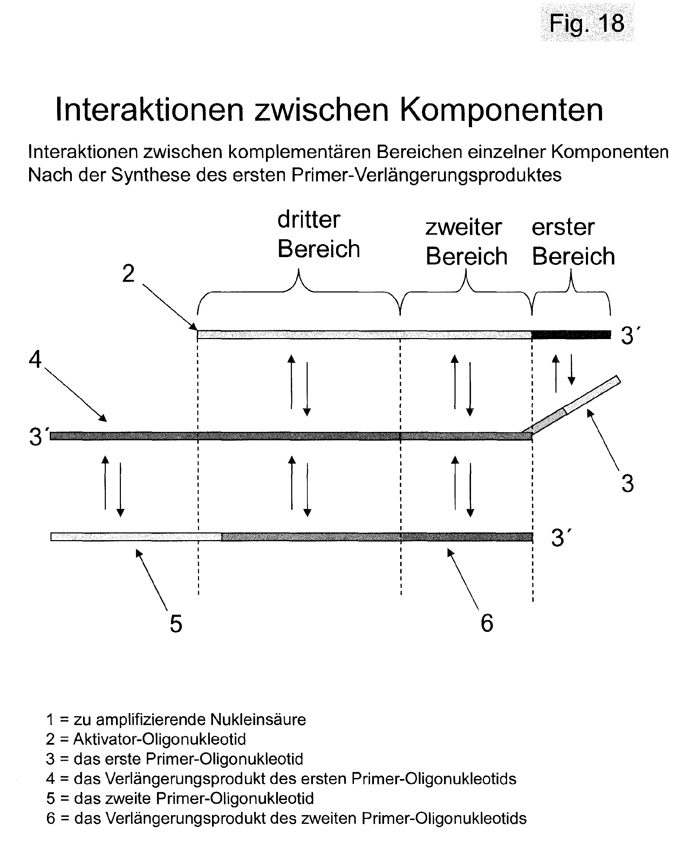

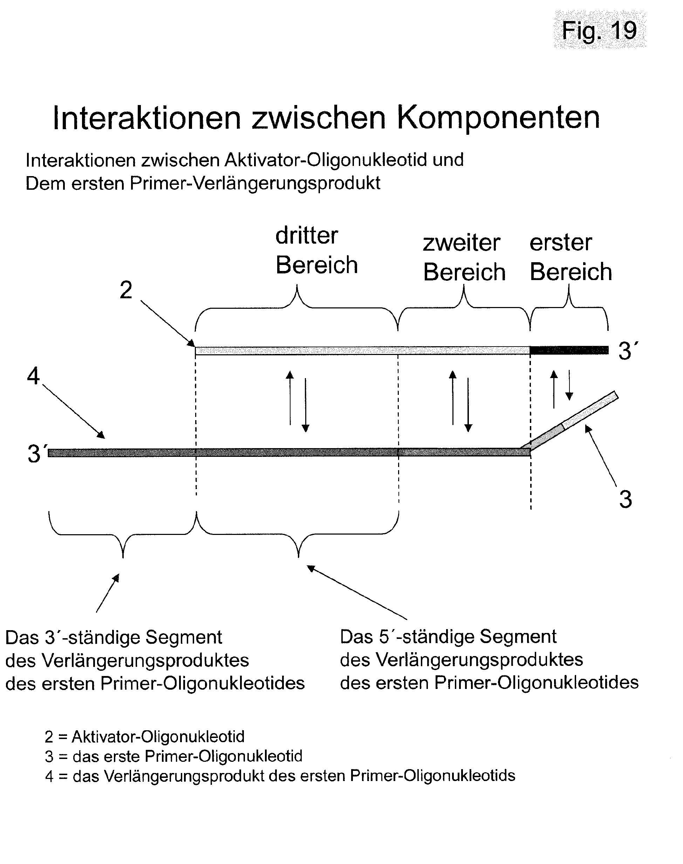

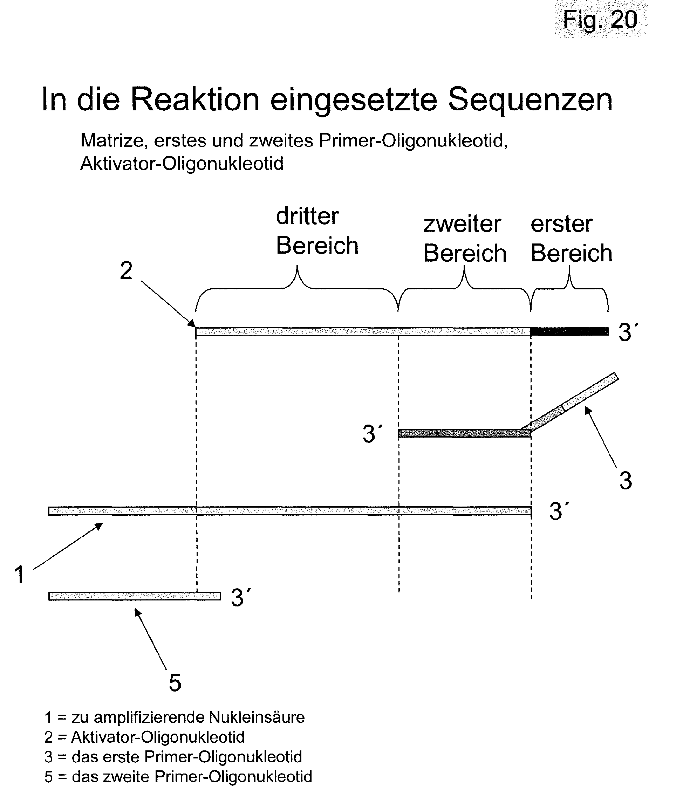

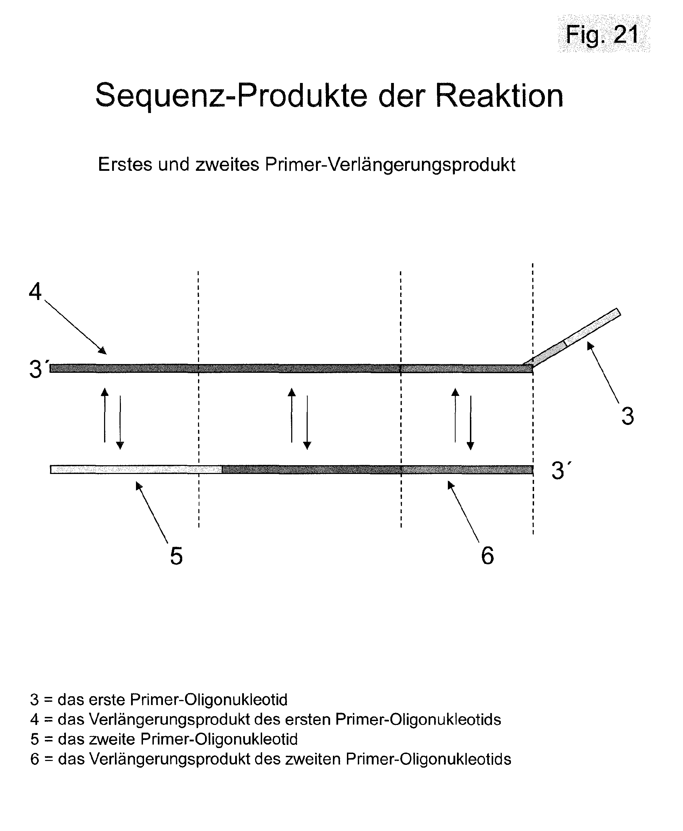

In dem erfindungsgemäßen Verfahren (Fig. 8 bis 21) kann als Ausgangsmaterial eine einzelsträngige Nukleinsäurekette dienen oder eine doppelsträngige Nukleinsäure, welche in einzelsträngige Form überführt wurde. Die Amplifikation erfolgt bevorzugt sequenzspezifisch, d.h. vorzugsweise wird die zu amplifizierende Nukleinsäure vervielfältigt.

Als Komponenten des Amplifikationssystems dienen eine zu amplifizierende Nukleinsäurekette, ein erstes spezifisches Primer-Oligonukleotid, ein zweiter Primer und ein Aktivator-Oligonukleotid, welches bei der Trennung der neu synthetisierten Stränge mitwirkt, sowie eine geeignete Polymerase und Substrate wie dNTP's. Die Amplifikation verläuft in einer Puffer-Lösung unter Bedingungen, welche eine Primer-Verlängerungsreaktion von beiden Primern zulassen, sowie welche eine Strangverdrängung durch Aktivator-Oligonukleotid zur Trennung von beiden Primer-Verlängerungsprodukten unterstützen.

In einer Ausführungsform sollen alle Verfahrensschritte unter Bedinungen verlaufen, welche keine Trennung von synthetisierten Primer-Verlängerungsproduken in Abwesenheit eines geeigneten Aktivator-Oligonukleotides zulassen. Beispielsweise ist die Temperatur der Reaktionslösung dermaßen gewählt, dass die Tm eines Doppelstranges aus beiden Primer-Verlängerungsprodukten signifikant über der Reaktionstemperatur liegt.

Unter diesen Voraussetzungen erfolgt eine Trennung von beiden Primer-Verlängerungsprodukten in Abhängigkeit von der Wirkung des Aktivator-Oligonukleotides. Dieses Aktivator-Oligonukleotid ist in der Lage komplementär an das erste Primer-Verlängerungsprodukt zu binden und dadurch das zweite Primer-Verlängerungprodukt aus seiner Bindung mit dem ersten Primerverlängerungsprodukt zu verdrängen. Um diese Strangverdrängungsreaktion zu initiieren ist das erste Primer-Oligonukleotid mit einem Polynukleotid-Schwanz in seinem zweiten Bereich versehen, welcher in der Lage ist, das Aktivator-Oligonukleotid unter Reaktionsbedingungen vorübergehend zu binden und damit eine räumliche Nähe zu anderen Bereichen des ersten Primer-Verlängerungsnukleotides herbeizuführen. Nach der Initiierung der Strangverdrängung durch das Aktivator-Oligonukleotid wird das zweite Primer-Verlängerungsprodukt aus seiner Bindung mit dem ersten Primer-Verlängerungsprodukt verdrängt. Sein 3'-ständiges Segment wird damit frei und steht für weitere Bindung eines ersten Primer-Oligonukleotides zur Verfügung.

Der Polynukleotid-Schwanz des ersten Primer-Oligonukleotides ist vorzugsweise nicht von einer Polymerase kopierbar. Das kann entweder durch Verwendung entsprechender Modifikationen in diesem Bereich erreicht werden oder durch Einführung einer ersten Blockierungseinheit zwischen dem ersten Primer-Bereich und dem zweiten Primer-Bereich des ersten Primer-Oligonukleotides.

Die Synthese des zweiten Primer-Verlängerungsproduktes erfolgt nach der Bindung des zweiten Primer-Oligonukleotides an das erste Primer-Verlängerungsprodukt in seinem 3'-ständigen Segment. Dieses Segment geht vorzugsweise keine Bindung mit dem Aktivator-Oligonukleotid ein und ist hinreichend lang, um das zweite Primer-Oligonukleotid zu binden und eine erfolgreiche Primer-Verlängerungsreaktion zu unterstützen. Die Synthese des zweiten Primer-Verlängerungsproduktes erfolgt unter Verdrängung des Aktivator-Oligonukleotides aus der Bindung mit dem ersten Primer-Verlängerungsprodukt.

Beide Primer-Verlängerungsprodukte schließen kopierbare Bereiche ein und dienen gegenseitig als Matrize. Das Aktivator-Oligonukleotid dient nicht als Matrize. Dies kann bevorzugt durch Verwendung von Nukleotid-Modifikationen erreicht werden, welche zwar eine komplementäre Bindung mit dem ersten Primer-Verlängerungsprodukt eingehen können, nicht aber von der Polymerase als Matrize akzeptiert werden. Beispiele für solche Nukleotid-Modifikationen stellen Nukleotid-Verbindungen mit modifizierten Rückgrad-Anteilen, z.B. 2'-O-Alkyl-RNA Modifikationen, LNA-Modifikationen oder Morpholino-Modifkationen dar. Im Allgemeinen hindert die Anwesenheit solcher Modifikationen in einem Strang eine DNA-Abhängige Polymerase beim Ablesen eines solchen Stranges. Die Anzahl von solchen Modifikationen kann unterschiedlich groß sein, in der Regel können wenige Modifikationen (zwischen 1 und 20) hinreichend sein, um eine Polymerase beim Ablesen eines solchen Stranges zu hindern. Solche Nukleotid-Modifikationen können beispielsweise an oder um die Bindungsstelle des ersten Primer-Oligonukleotides an das Aktivator-Oligonukleotid verwendet werden und / oder als Bestandteile des zweiten Bereichs des ersten Primer-Oligonukleotides.

Dank der Verwendung solcher Modifikationen kommt es zu einer lokalen Hinderung der Polymerase-Funktion, so dass bestimmte Segmente der verwendeten Strukturen nicht durch die Polymerase kopiert werden können und vorwiegend einzelsträngig bleiben. In dieser einzelsträngigen Form können sie weitere Reaktionskomponenten binden und somit ihre Funktion ausführen.

Dank der Verwendung der sequenzabhängigen nukleinsäurevermittelten Strangverdrängung zur Trennung der beiden Primer-Verlängerungsprodukte während der Amplifikationsreaktion ist das beschriebene Verfahren sequenzspezifisch: eine hinreichende Komplementarität zwischen neusynthetisierten Verlängerungsfragmenten der Primer-Oligonukleotide mit der zu Beginn einer Amplifikation vorgegebenen Sequenz eines Aktivator-Oligonukleotides stellt eine Voraussetzung für eine erfolgreiche Strangverdrängung dar und kann somit Einfluss auf die Effizienz der Strangtrennung eines Doppelstranges (bestehend aus dem ersten und dem zweiten Primer-Verlängerungsprodukt) haben. Bei geringfügigen Abweichungen kommt es lediglich zu einer Verlangsamung der Strangverdrängung und somit auch zur Verlangsamung der Strangtrennung. Diese kann sich in einer Verlangsamung der Gesamtreaktion auswirken. Mit steigender Differenz in der Sequenz der neusynthetisierten Verlängerungsprodukte und der zu Beginn der Reaktion vorgegebenen Sequenz des Aktivator-Oligonukleotides kommt es zu einer immer höheren Hinderung der Strangverdrängung, welche im Endeffekt keine ausreichende Trennung von beiden Primer-Verlängerungsprodukten mehr leisten kann. Die beiden neu synthetisierten Stränge können nicht mehr ausreichend voneinander getrennt werden, so dass ihre Bindungsstellen für Primer-Oligonukleotide nicht mehr zugängig sind. Das führt im Allgemeinen zum Abbruch einer Amplifikation von Sequenzen mit Sequenzabweichungen.

Zusammengefasst können nicht nur die Spezifitäten der Bindung von beiden Primern mit ihren Matrizen, sondern auch die Beschaffenheit der Sequenzsegmente zwischen den Primern auf die Amplifikation Auswirkung haben, nämlich in dem diese Abschnitte eine hinreichende Strangverdrängung zulassen oder nicht, je nach ihrer Übereinstimmung in der zu Beginn der Reaktion vorgegebenen Sequenz des Aktivator-Oligonukleotides. Das beschrieben Verfahren kann dadurch unter Umständen insgesamt eine höhere Spezifität vorweisen, als die herkömmlichen Amplifikations-Verfahren.

Das erfindungsgemäße Verfahren verwendet vorzugsweise keine spezifischen Proteine, welche einen Strang-Austausch zwischen Aktivator-Oligonukleotid und einem Doppelstrang bewirken, z.B. RecA. Das erfindungsgemäße Verfahren macht vielmehr Gebrauch von der zeitlichen Kopplung zwischen einer Strangverdrängung mittels einer zu Beginn der Reaktion vorgegebenen Nukleinsäurekette (Aktivator-Oligonukleotid) und einer gleichzeitig stattfindenden matrizenabhängigen Strang-Polymerisation mittels Polymerase. Diese gleichzeitige Kopplung von beiden Prozessen wird zum einen durch sequenzspezifische Strukturen von Komponenten (sequenzspezifische Aktivator-Oligonukleotid und sequenzspezifische Primer) sowie ihre spezifische Kombination ermöglicht. Zum anderen wird die Aktivität der Polymerase sequenzspezifisch kontrolliert: beispielsweise dient Aktivator-Oligonukleotid nicht als Matrize und die Synthese des zweiten Primer-Verlängerungsproduktes wird am ersten Primer-Oligonukleotid positionsspezifisch gestoppt. Die spezifische Amplifikation resultiert weiterhin durch Verwendung der Komponenten bei Reaktionsbedinungen, welche eine spontane Trennung von neu synthetisierten Primer-Verlängerungsprodukte nicht zulassen.

Überraschenderweise ist es gelungen, diese Vorgänge vorteilhaft miteinander zu verknüpfen, und zwar in einer Art und Weise, dass beide Vorgänge (sequenzspezifische Strangverdrängung und Primer-Verlängerung) vorzugsweise in einem Ansatz ablaufen können. Dadurch kann ein homogenes Amplifikationssystem zusammengestellt werden, welches sequenzspezifische Primer-Oligonukleotide, ein sequenzspezifisches Aktivator-Oligonukleotid, Polymerase und Nukleotid-Substrate (dNTP) umfasst. Mit einem solchen Amplfikationssystem kann eine zu amplifizierende Nukleinsäure vervielfältigt werden. Die Sequenzen der beiden Primer-Oligonukleotide und die Sequenz des Aktivator-Oligonukleotides sind auf die zu amplifizierende Nukleinsäure abgestimmt.

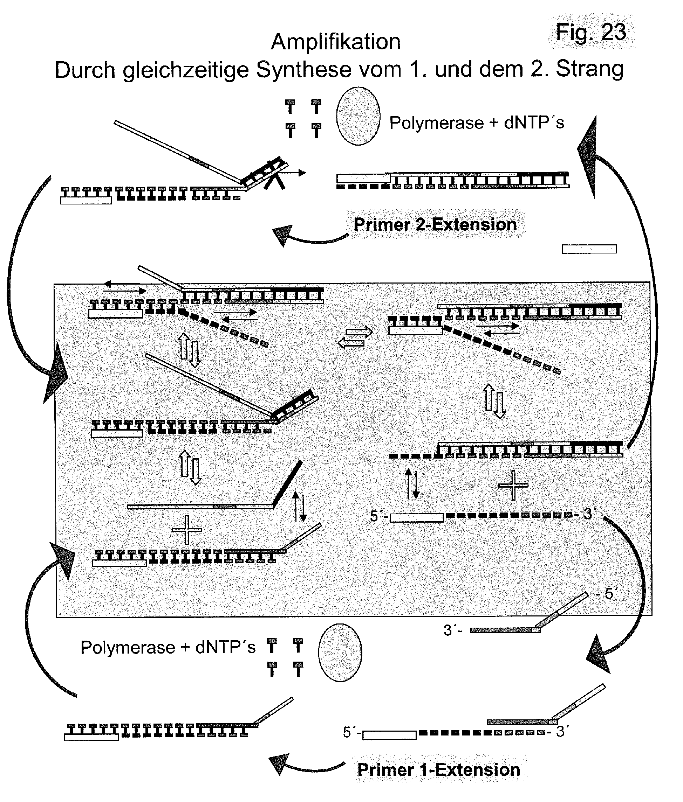

Das Verfahren (Fig. 23) umfasst mehrere Vorgänge, welche nachfolgend beschrieben sind. Diese Vorgänge können in einem Ansatz oder in getrennten Ansätzen ausgeführt werden. Wenn die Vorgänge in einem Ansatz durchgeführt werden sollen, so können diese unter gleichen Bedingungen, z.B. isothermal, oder unter unterschiedlichen Bedingungen, z.B. beim Thermocycling, ausgeführt werden. Vorzugsweise sind Primer-Oligonukleotide und Aktivator-Oligonukleotid zu Beginn der Reaktion präsent. Eine sequenzielle Zugabe einzelner Reagenzien ist allerdings auch möglich.

Ebenfalls sind Kombinationen mit anderen Amplifikationsverfahren möglich, z.B. mit PCR, wobei die PCR beispielsweise erst über 1 bis 10 Zyklen durchgeführt werden und anschließend wird unter isothermalen Bedingungen weitergearbeitet.

In einer vorteilhaften Form umfasst das Verfahren zur spezifischen Amplifizierung von Nukleinsäureketten folgende Schritte:
a) Hybridisierung eines ersten Primer-Oligonukleotids an das 3'-Ende eines Stranges einer zur amplifizierenden Nukleinsäurekette, wobei die zu amplifizierende Nukleinsäurekette eine Zielsequenz umfasst,
   wobei das erste Primer-Oligonukleotid umfasst:
   - Einen ersten Primer-Bereich im 3'-Segment des ersten Primer-Oligonukleotides, der an einen Strang einer zu amplifizierenden Nukleinsäurekette sequenzspezifisch binden kann
   - Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt.
b) Extension des ersten Primer-Oligonukleotids mit Hilfe einer Polymerase zur Bildung eines ersten Primer-Verlängerungsproduktes, das zur Zielsequenz der zu amplifizierenden Nukleinsäurekette (a) komplementär ist,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
   - Einen ersten einzelsträngigen Bereich, welcher an den Polynukleotid-Schwanz des zweiten Bereichs des ersten Primer-Oligonukleotides binden kann
   - Einen zweiten einzelsträngigen Bereich, welcher zu dem ersten Bereich des ersten Primer-Oligonukleotides im Wesentlichen komplementär ist und an diesen binden kann
   - Einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
   wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette bis dieser komplementärer Strang der zu amplifizierenden Nukleinsäure vom ersten Primer-Verlängerungsprodukt abgelöst wird, wobei das 3'-Segment des ersten Primer-Verlängerungsproduktes einzelsträngig wird
f) Hybridisierung eines zweiten Oligonukleotid-Primers an das erste Primer-Verlängerungsprodukt, wobei das 3'-Segment des zweiten Oligonukleotid-Primers eine Ziel-Bindungssequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist.

In einer Ausführungsform wird das Verfahren unter Bedinugen ausgeführt, welche keine Trennung von komplementären Strängen der zu amplifizierenden Nukleinsäure in Abwesenheit von Aktivator-Oligonukleotid zulassen.

In einer Ausführungsform wird das Kopieren des Polynukleotid-Schwanzes im zweiten Primer-Bereich durch einen Stopp Bereich für die Polymerase bewirkt, der zwischen dem ersten und dem zweiten Bereich angeordnet ist.

In einer Ausführungsform ist der dritte einzelsträngige Bereich des Aktivator-Oligonukleotid zum Segment des von Polymerase synthetisierten Extensionsproduktes des ersten Primer-Extensionsprodukt, welches unmittelbar an den ersten Primer-Bereich anschließt, im Wesentlichen komplementär ist.

In einer weiteren Ausführungsform des Verfahrens wird Schritt (f) des Verfahrens weiterhin modifiziert und umfasst:
Hybridisierung eines zweiten Oligonukleotid-Primers an das erste Primer-Verlängerungsprodukt, wobei gleichzeitige zumindest partielle Verdrängung des Aktivator-Oligonukleotides aus der Bindung mit dem ersten Verlängerungsprodukt durch Strangverdrängung erfolgt.

In einer weiteren Ausführungsform des Verfahrens wird Schritt (g) des Verfahrens weiterhin modifiziert und umfasst eine Verdrängung des Aktivator-Oligonukleotides aus der Bindung mit dem ersten Primer-Verlängerungsprodukt unter Mitwirkung der Polymerase.

In einer weiteren Ausführungsform des Verfahrens wird Schritt (h) des Verfahrens weiterhin modifiziert und umfasst: ggf. Bindung des Aktivator-Oligonukleotides an den unkopierten Polynukleotid-Schwanz des ersten verlängerten Primer-Oligonukleotid und Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oligonukleotides.

In einer weiteren Ausführungsform des Verfahrens wird Verfahren weiterhin modifiziert und umfasst: h) Fortführung der Reaktion unter Bedinungen, welche Wiederholung von Schritten (a) bis (g) zulassen.

In einer weiteren Ausführungsform des Verfahrens wird des Verfahrens weiterhin modifiziert und umfasst: gleichzeitige Amplifizierung des ersten und zweiten Primer-Verlängerungsproduktes in einer exponentiellen Reaktion unter Verwendung der ersten und zweiten Primer-Oligonukleotid und des Aktivator-Oligonukleotides, wobei die gebildeten Primer-Verlängerungsprodukte als Matrizen für gegenseitige Synthese auftreten.

### Bevorzugte Ausführungsformen des ersten Primer-Oligonukleotids (Primer-1)

**Das erste Primer-Oligonukleotid** (Primer-1) ist eine Nukleinsäurekette welche mindestens folgende Bereiche einschließt (Fig. 5 und 6):
- Einen ersten Primer-Bereich im 3'-Segment des ersten Primer-Oligonukleotides, der an einen Strang einer zu amplifizierenden Nukleinsäurekette im Wesentlichen sequenzspezifisch binden kann
- Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz unter Reaktionsbedingungen im wesentlichen einzelsträngig bleibt, d.h. keine Hairpin-Struktur oder ds-Strukturen ausbildet, und vorzugsweise nicht von Polymerase kopiert wird.

Die Gesamtlänge des ersten Primer-Oligonukleotides liegt zwischen 10 und 80, vorzugsweise zwischen 15 und 50, besser zwischen 20 und 30 Nukleotiden oder ihren Äquivalenten (z.B. Nukleotid-Modifikationen). Die Struktur des ersten Primer-Oligonukleotids ist dermassen angepasst, dass es unter gewählten Reaktionsbedinungen eine reversible Bindung an Aktivator-Oligonukleotid eingehen kann. Weiterhin ist die Struktur des ersten Primer-Oligonukletides an seine Primer-Funktion angepasst. Weiterhin ist die Struktur so angepasst, dass eine Strangverdrängung mittels Aktivator-Oligonukleotid ausgeführt werden kann. Insgesamt, sind Strukturen des ersten und des zweiten Bereichs aneinander angepasst, so dass exponentielle Amplifikation ausgeführt werden kann.

In einer vorteilhaften Ausführungsform der Erfindung sind der erste und der zweite Bereich des Primers in einer konventionellen 5'- 3' Anordnung gekoppelt. In einer weiteren Ausführungsform der Erfindung erfolgt die Kopplung von beiden Abschnitten über eine 5'-5'-Bindung, so dass der zweite Bereich eine umgekehrte Richtung hat als der erste Bereich.

Die Kopplung von Bereichen zwischen einander / untereinander erfolgt vorzugsweise kovalent. In einer Ausführungsform ist die Kopplung zwischen dem ersten und zweiten Bereich eine für DNA konventionelle 5'-3'-Phosphodiester-Kopplung. In einer weiteren Ausführungsform ist es eine 5'-5'-Phosphodiester-Kopplung. In einer weiteren Ausführungsform ist es eine 5'-3'-Phosphodiester-Kopplung, wobei zwischen benachbarten endständigen Nukleotiden bzw. Nukleotid-Modifikationen der beiden Bereiche mindestens ein Linker (z.B. ein C3, C6, C12 oder ein HEG-Linker oder eine abasische Modifikation) positioniert ist.

Einzelne Bereiche können unterschiedliche Nukleotid-Modifikationen einschließen. Dabei können individuelle Elemente von Nukleotiden modifiziert sein: Nukleobase und Rückgrad (Zucker-Anteil und / oder Phosphat-Anteil). Weiterhin können Modifikationen verwendet werden, welchen mindestens eine Komponente der Standard-Nukleotid-Bausteinen fehlt oder modifiziert ist, z.B. PNA.

In einer weiteren Ausführungsform umfasst ein zweiter Bereich des ersten Primer-Oligonukleotides weitere Sequenzen, die nicht an das Aktivator-Oligonukleotid binden. Diese Sequenzen können zu anderen Zwecken verwendet werden, z.B. zu Bindung an die feste Phase. Diese Sequenzen sind vorzugsweise am 5'-Ende des Polynukleotid-Schwanzes lokalisiert.

In einer weiteren Ausführungsform kann ein erstes Primer-Oligonukleotid charakteristische Markierung umfassen. Beispiele für eine solche Markierung stellen Farbstoffe dar (z.B. FAM, TAMRA, Cy3, Alexa 488 etc.) oder Biotin bzw. andere Gruppen, die spezifisch gebunden werden können, z.B. Digoxigenin.

### Der erste Primer-Bereich des ersten Primer-Oligonukleotides

Die Sequenz-Länge liegt zwischen ca. 3-30 Nukleotiden, vorzugsweise zwischen 5 und 20 Nukleotiden, wobei die Sequenz vorwiegend komplementär zum 3'-Segment der zu amplifizierenden Sequenz ist. Im Einzelnen, muss dieser Primer-Bereich in der Lage sein, an das komplementäre 3'-Segment eines zweiten Primer-Verlängerungsproduktes spezifisch zu binden. Dieser erste Bereich soll bei Rücksynthese kopiert werden können und dient auch als Matrize für 2. Strang. Die Nukleotid-Bausteine sind vorzugsweise untereinander via übliche 5'-3' Phosphodieser-Bindung oder Phosphothioester-Bindung verknüpft.

Der erste Primer-Bereich schließt bevorzugt Nukleotid-Monomere ein, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung.

Das 3'-OH-Ende dieses Bereichs ist vorzugsweise frei von Modifikationen und hat eine funktionsfähige 3'-OH Gruppe, welche von Polymerase erkannt werden kann. Der erste Primer-Bereich dient als Initiator der Synthese des ersten Primer-Verlängerungsproduktes in der Amplifikation.

Die Sequenz des ersten Bereichs des ersten Primer-Oligonukleotids und die Sequenz des zweiten Bereichs des Aktivator-Oligonukleotides sind vorzugsweise einander komplementär.

### Der zweite Bereich des ersten Primer-Oligonukleotides

Der zweite Bereich des ersten Primer-Oligonukleotides ist vorzugsweise eine Sequenz, welche mindestens einen Polynukleotid-Schwanz umfasst, welcher vorzugsweise während der Synthesereaktion von Polymerase unkopiert bleibt und welcher zur Bindung mit dem ersten Bereich des Aktivator-Oligonukleotides fähig ist. Das Segment des zweiten Bereichs, welches überwiegend diese Bindung mit dem Aktivator-Oligonukleotid eingeht, kann als Polynukleotid-Schwanz bezeichnet werden.

Weiterhin muss der zweite Bereich des ersten Primer-Oligonukleotids nicht nur das Aktivator-Oligonukleotides unter Reaktionsbedinungen spezifisch binden, sondern auch beim Vorgang der Strangverdrängung mittels Aktivator-Oligonukleotides mitwirken. Die Struktur des zweiten Bereichs muß folglich für Herbeiführung einer räumlichen Nähe zwischen dem Aktivator-Oligonukleotid und dem entsprechenden Doppelstrang-Ende (im Einzelnen, dem 3'-Ende des zweiten Primer-Verlängerungsproduktes) geeignet sein.

Die Gestaltung der Struktur des zweiten Bereichs des ersten Primer-Oligonukleotids wird in mehreren Ausführungsformen genauer dargestellt. Dabei werden Anordnung der Oligonukleotid-Segmente und verwendeten Modifikationen berücksichtigt, welche zu einem Stopp in der Polymerase-katalysierten Synthese führen.

Die Länge des zweiten Bereichs liegt zwischen 3 und 60, vorzugsweise zwischen 5 und 40, bevorzugt zwischen 6 und 15 Nukleotiden oder ihren Äquivalenten.

Die Sequenz des zweiten Bereichs kann willkürlich gewählt sein. Vorzugsweise ist sie nicht komplementär mit der zu amplifizierenden Nukleinsäure und / oder mit dem zweiten Primer-Oligonukleotid und / oder mit dem ersten Bereich des ersten Primer-Oligonukleotid. Weiterhin enthält sie vorzugsweise keine selbstkomplementären Segmente, wie Hairpins oder Stemmloops.

Die Sequenz des zweiten Bereichs ist vorzugsweise auf die Sequenz des ersten Bereichs des Aktivator-Oligonukleotids abgestimmt, so dass beide Sequenzen unter Reaktionsbedinungen eine Bindung eingehen können. In einer bevorzugten Ausführungsform ist diese Bindung unter Reaktionsbedinungen reversibel: es besteht somit ein Gleichgewicht zwischen aneinander gebundenen Komponenten und nicht-gebundenen Komponenten.

Die Sequenz des zweiten Bereichs des ersten Primer-Oligonukleotides ist vorzugsweise dermaßen gewählt, dass die Anzahl von komplementären Basen, welche mit dem ersten Bereich des Aktivator-Oligonukleotid binden können, zwischen 1 und 40, besser zwischen 3 und 20, bevorzugt zwischen 6 und 15 liegt.

Die Funktion des zweiten Bereichs besteht unter anderem in Bindung des Aktivator-Oligonukleotides. In einer Ausführungsform ist diese Bindung spezifisch, so dass ein zweiter Bereich eines ersten Primer-Oligonukleotides ein spezifisches Aktivator-Oligonukleotid binden kann. In einer anderen Ausführungsform kann ein zweiter Bereich mehr als nur ein Aktivator-Oligonukleotid unter Reaktionsbedinungen binden.

Es besteht im Allgemeinen keine Notwendigkeit in einer perfekten Übereinstimmung in der Sequenz zwischen dem zweiten Bereich des ersten Primer-Oligonukleotides und dem ersten Bereich des Aktivator-Oligonukleotides. Das Maß der Komplementarität zwischen dem zweiten Bereich des ersten Primer-Oligonukleotides und dem ersten Bereich des Aktivator-Oligonukleotdies kann zwischen 20% und 100%, besser zwischen 50% und 100%, bevorzugt zwischen 80% und 100% liegen. Die jeweils komplementären Bereiche können unmittelbar aneinander anschließend positioniert sein oder auch nichtkomplementäre sequenz-Segmente dazwischen umfassen.

Der zweite Bereich des ersten Primer-Oligonukleotides kann in einer Ausführungsform mindestens eine Tm-Modifizierende Modifikation einschließen. Durch Einführung solcher Modifikationen kann die Stabilität der Bindung zwischen dem zweiten Bereich des ersten Primer-Oligonukleotides und dem ersten Bereich des Aktivator-Oligonukleotides modifiziert werden. Beispielsweise können Tm-erhöhende Modifikationen (Nukleotid-Modifikationen oder nicht-Nukleotid-Modifikationen) verwendet werden, wie LNA-Nukleotide, 2-Amino-Adenosine oder MGB-Modifikationen. Andererseits können auch Tm-Senkende Modifikationen verwendet werden, wie beispielsweise Inosine-Nukleotid. In der Struktur des zweiten Bereichs können auch Linker (z.B. C3, C6, HEG-Linker) intergriert werden.

Für die Strangverdrängung muss das Aktivator-Oligonukleotid in räumliche Nähe des Doppelstrang-Endes der zu amplifizierenden Nukleinsäure gebraucht werden. Dieses Doppelstrang-Ende besteht aus Segmenten des ersten Primer-Bereich des ersten Primer-Verlängerungsproduktes und einem entsprechend komplementären dazu 3'-Segment des zweiten Primer-Verlängerungsproduktes.

Der Polynukleotid-Schwanz bindet vorwiegend komplementär das Aktivator-Oligonukleotides unter Reaktionsbedinungen und bewirkt damit eine vorübergehende Annäherung des zweiten Bereichs des Aktivator-Oligonukleotides und des ersten Bereichs eines verlängerten Primer-Verlängerungsproduktes, so dass eine komplementäre Bindung zwischen diesen Elementen im Rahmen eines Strangverdrängungsvorgangs initiiert werden kann.

In einer Ausführungsform führt die Bindung des Aktivator-Oligonukleotides an den Polynukleotid-Schwanz des ersten Primer-Oligonukleotides unmittelbar zu einem solchen Kontakt. Das bedeutet, dass der Polynukleotid-Schwanz und der erste Primer-Bereich des ersten Primer-Oligonukleotides unmittelbar aneinander gekoppelt sein müssen. Dank einer solchen Anordnung kann nach einer Bindung eines Aktivator-Oligonukleotids im seinem ersten Bereich unmittelbar zu einem Kontakt zwischen komplementären Basen des zweiten Bereichs des Aktivator-Oligonukleotides und zu entsprechenden Basen des ersten Primer-Bereichs kommen, so dass eine Strangverdrängung initiiert werden kann.

In einer weiteren Ausführungsform liegen zwischen Strukturen des Polynukleotid-Schwanzes und dem ersten Primer-Bereich andere Strukturen des zweiten Bereichs des ersten Primer-Oligonukleotides. Nach einer Bindung eines Aktivator-Oligonukleotids an den Polynukleotid-Schwanz ist dieser somit nicht unmittelbar an den ersten Primer-Bereich positioniert, sondern in einer gewissen Distanz dazu. Die Strukturen zwischen dem unkopierbarem Polynukleotid-Schwanz und dem kopierbaren ersten Primer-Bereich des Primer-Oligonukleotides können einen solchen Abstand generieren. Dieser Abstand hat einen Wert, welcher zwischen 0,1 und 20 nm, vorzugsweise zwischen 0,1 und 5 nm, besser zwischen 0,1 und 1 nm liegt.

Solche Strukturen stellen beispielsweise Linker (z.B. C3 oder C6 oder HEG-Linker) oder nicht zu Aktivator-Oligonukleotid komplementäre Segmente dar (z.B. in Form von nicht-komplementären, nicht-kopierbaren Nukleotid-Modifikationen). Die Länge dieser Strukturen kann im Allgemeinen in Ketten-Atomen ausgemessen werden. Diese Länge begrägt zwischen 1 und 200 Atomen, vorzugsweise zwischen 1 und 50 Kettenatomen, bevorzugt zwischen 1 und 10 Kettenatomen.

Damit der Polynukleotid-Schwanz von Polymerase unter Amplifikationsbedinungen unkopierbar bleibt, umfasst der zweite Bereich des ersten Primer-Oligonukleotides im allgemeinen Sequenz-Anordnungen bzw. Strukturen, welche zu einem Stopp der Polymerase in der Synthese des zweiten Primer-Verlängerungsproduktes bewirken, nachdem die Polymerase den ersten Primer-Bereich erfolgreich kopiert hat. Diese Strukturen sollen das Kopieren des Polynukleotid-Schwanzes des zweiten Bereichs verhindern. Der Polynukleotid-Schwanz bleibt somit vorzugsweise von der Polymerase unkopiert.

In einer Ausführungsform liegen solche Strukturen zwischen dem ersten Primer-Bereich und dem Polynukleotid-Schwanz.

In einer weiteren Ausführungsform kann die Sequenz des Polynukleotid-Schwanzes Nukleotid-Modifikationen einschließen, welche zum Stopp der Polymerase führen. Dadurch kann ein Sequenzsegment des zweiten Bereichs des ersten Primer-Oligonukleotids beide Funktionen umfassen: es ist sowohl ein Polynukleotid-Schwanz als auch eine zum Stopp der Polymerase führende Sequenz aus Nukleotid-Modifikationen.

Modifikationen im zweiten Bereich des ersten Primer-Oligonukleotid, welche zu einem Synthese-Stopp führen und somit den Polynukleotid-Schwanz unkopierbar lassen, werden in dieser Anmeldung unter dem Begriff "erste Blockierungs-Einheit oder einen Stopp-Bereich" zusammengefasst.

### Nachfolgend sind weitere Ausführungsformen von Strukturen angeführt, welche zum Stopp in der Synthese des zweiten Stranges führen können.

Mehrere Bausteine bei der Oligonukleotid-Synthese sind bekannt, welche die Polymerase beim Ablesen der Matrize hindern und zur Termination von Polymerase-Synthese führen. Beispielsweise sind nicht-kopierbare Nukleotid-Modifikationen oder Nicht-Nukleotid-Modifikationen bekannt. Es gibt auch Synthese-Arten / Anordnungen von Nukleotid-Monomeren innerhalb eines Oligonukleotides, welche zum Stopp der Polymerase führen (z.B. 5'-5-Anordnung oder 3'-3'Anordnung). Primer-Oligonukleotide mit einem nicht kopierbaren Polynukleotid-Schwanz sind ebenfalls im Stand der Technik bekannt (z.B. Scorpion-Primer-Strukturen bzw. Primer für Bindung an die feste Phase). Beide Primer-Varianten beschreiben Primer-Oligonukleotid-Strukturen, welche in der Lage sind, einerseits die Synthese eines Stranges zu initiieren, so dass eine Primer-Verlängerungsreaktion stattfinden kann. Es resultiert ein erster Strang, welcher im Primer-Verlängerungs-Produkt auch die Primer-Struktur mit Schwanz integriert. Bei der Synthese eines komplementären Stranges zum ersten Primer-Verlängerungs-Produkt, z.B. im Rahmen einer PCR-Reaktion, wird der zweite Strang bis zur "Blockierungs-Einheit/Stopp-Struktur" der Primer-Struktur verlängert. Beide beschriebene Primer-Strukturen sind dermaßen konzipiert, dass der 5'-Anteil des Primer-Oligonukleotides einzelsträngig bleibt und nicht von der Polymerase kopiert wird.

In einer weiteren Ausführungsform umfasst der zweite Bereich des Primer-Oligonukleotides einen Polynukleotid-Schwanz, welche in seiner gesamten Länge eine konventionelle Anordnung von 5'- nach 3'- aufweist und nicht-kopierbare Nukleotid-Modifikationen einschließt. Zu solchen nicht-kopierbaren Nukleotid-Modifikationen zählen beispielsweise 2'-O-Alkyl-RNA Modifikationen, PNA, Morpholino. Diese Modifikationen können unterschiedlich im zweiten Primer-Bereich verteilt sein.

Der Anteil von nicht-kopierbaren Nukleotid-Modifikationen kann im Polynukleotid-Schwanz zwischen 20% und 100% liegen, vorzugsweise beträgt er mehr als 50% der Nukleotid-Bausteinen. Vorzugsweise liegen diese Nukleotid-Modifikationen im 3'-Segment des zweiten Bereichs und grenzen somit an den ersten Bereich des ersten Primer-Oligonukleotides.

In einer Ausführungsform ist die Sequenz der nicht-kopierbaren Nukleotid-Modifikationen zumindest teilweise komplementär zu der Sequenz im Matrizenstrang, so dass die Primer-Bindung an die Matrize unter Einbeziehung von zumindest einem Teil dieser Nukleotid-Modifikationen stattfindet. In einer weiteren Ausführungsform ist die Sequenz der nicht-kopierbaren Nukleotid-Modifikationen nicht-komplementär zu der Sequenz im Matrizenstrang.

Die nicht-kopierbaren Nukleotid-Modifikationen werden vorzugsweise aneinander kovalent gekoppelt und stellen somit ein Sequenz-Segment im zweiten Bereich dar. Die Länge dieses Segments liegt zwischen 1 und 20 Nukleotid-Modifikationen, bevorzugter zwischen 3 und 10 Nukleotid-Modifikationen.

In einer weiteren Ausführungsform umfasst der zweite Bereich des ersten Primer-Oligonukleotides einen Polynukleotid-Schwanz, welcher in seiner gesamten Länge eine konventionelle Anordnung von 5'-3' aufweist und nicht-kopierbare Nukleotid-Modifikationen einschließt (z.B. 2'-O-Alkyl-Modifikationen) und mindestens einen Nicht-Nukleotid-Linker (z.B. C3, C6, HEG-Linker) einschließt. Ein Nicht-Nukleotid-Linker hat die Funktion, benachbarte Nukleotide oder Nukleotid-Modifikationen kovalent zu verbinden und gleichzeitig die Synthese-Funktion der Polymerase ortspezifisch zu unterbrechen.

Ein solcher Nicht-Nukleotid-Linker soll die Strukturen des Polynukleotid-Schwanzes und des ersten Primer-Bereichs nicht zu weit weg bringen. Vielmehr sollte der Polynukleotid-Schwanzes in einer räumlichen Nähe vom ersten Primer-Bereich sein. Unter einem Nicht-Nukleotid-Linker werden Modifikationen zusammengefasst, welche in ihrer Länge nicht länger sind als 200 Ketten-Atome, noch vorteilhafter nicht länger als 50 Ketten-Atome, besonders bevorzugt nicht länger als 10 Ketten-Atome umfassen. Die Minimal-Länge eines solchen Linkers kann ein Atom betragen. Ein Beispiel für solche Nicht-Nukleotid-Linker stellen gerade oder verzweigte Alkyl-Linker mit einer Alkyl-Kette dar, welche mindestens ein Kohlenstoff-Atom einschließt, vorteilhafter mindestens 2 bis 30, bevorzugter 4 bis 18. Solche Linker sind in der Oligonukleotid-Chemie hinreichend bekannt (z.B. C3, C6 oder C12 Linker) und können während einer Festphasen-Synthese von Oligonukleotiden zwischen der Sequenz des Polynukleotid-Schwanzes und der Sequenz des ersten Bereichs des ersten Primer-Oligonukleotides eingeführt werden. Ein anderes Beispiel für solche Nicht-Nukleotid-Linker stellen Lineare oder verzweigte Polyethylen-Glykol-Derivate dar. Ein bekanntes Beispiel in der Oligonukleotid-Chemie stellt Hexaethylenglycol (HEG) dar. Ein weiteres Beispiel für solche Nicht-Nukleotid-Linker stellen abasische Modifikationen dar.

Wenn eine oder mehrere solche Modifikationen in einen zweiten Bereich integriert sind, können sie eine Polymerase in ihrer Kopier-Funktion während ihrer Synthese des zweiten Primer-Verlängerungsprodukts effektiv stören, so dass stromabwärts gelegene Segmente nach einer solchen Modifikation unkopiert bleiben. Die Anzahl von solchen Modifikationen im zweiten Bereich kann zwischen 1 und 100 liegen, vorzugsweise zwischen 1 und 10, bevorzugt zwischen 1 und 3.

Die Position eines solchen Nicht-Nukleotid-Linker kann am 3'-Ende des zweiten Bereichs liegen und somit den Übergang zum ersten Bereich und dem zweiten Bereich des Primer-Oligonukleotid darstellen

Die Lage des Nicht-Nukleotid-Linker im Mittleren Segment des zweiten Bereichs kann ebenfalls verwendet werden. Damit wird ein Polynukleotid-Schwanz in mindestens zwei Segmente geteilt. In dieser Ausführungsform schließt das 3'-Segment des Polynukleotid-Schwanzes mindestens eine, besser mehrere, z.B. zwischen 2 und 20, vorzugsweise zwischen 2 und 10 nicht-kopierbaren Nukleotid-Modifikationen ein. Diese nicht-kopierbaren Nukleotid-Modifikationen liegen vorzugsweise am Übergang zwischen dem ersten und dem zweiten Bereich des Primer-Oligonukleotides.

In einer weiteren Ausführungsform umfasst der zweite Bereich des Primer-Oligonukleotides einen Polynukleotid-Schwanz, welcher in seiner gesamten Länge eine Anordnung von 5'-nach 3'- aufweist und zumindest ein Nukleotid-Monomer in einer "umgekehrten" Anordnung von 3'-nach 5'- einschließt und welche am Übergang zwischen dem ersten und dem zweiten Bereich des ersten Primer-Oligonukleotides positioniert sind.

In einer weiteren Ausführungsform umfasst der zweite Bereich des Primer-Oligonukleotides einen Polynukleotid-Schwanz, wobei ein solcher Polynukleotid-Schwanz vollständig aus Nukleotiden besteht, welche in umgekehrten Anordnung direkt an den ersten Bereich des ersten Primer-Oligonukleotides angrenzen, so dass die Kopplung des ersten und des zweiten Bereichs durch 5'-5' Position erfolgt. Ein Vorteil einer solchen Anordnung ist, dass die Polymerase nach einer Kopierung des ersten Bereichs an eine "umgekehrte" Anordnung von Nukleotiden trifft, was typischerweise zu Termination der Synthese an dieser Stelle führt.

Bei einer "umgekehrten" Anordnung von Nukleotiden in der Gesamtlänge des Polynukleotid-Schwanzes soll vorzugsweise das 3'-terminales Nukleotid des Polynukleotid-Schwanzes an seinem 3'-OH-Ende blockiert sein, um Nebenreaktionen vorzubeugen. Alternativ kann auch ein terminales Nukleotid verwendet werden, welches gar keine 3'-OH Gruppe aufweist, z.B. ein Dideoxy-Nukleotid.

In einer solchen Ausführungsform soll natürlich auch die entsprechende Nukleotid-Anordnung im Aktivator-Oligonukleotid angepasst werden. In einem solchen Fall müssen der erste und der zweite Bereich des Aktivator-Oligonukleotids in 3'-3' Anordnung verknüpft werden.

In einer weiteren Ausführungsform umfasst der zweite Bereich des Primer-Oligonukleotides einen Polynukleotid-Schwanz, welche in seiner gesamten Länge eine konventionelle Anordnung von 5'-nach 3'- aufweist und mindestens eine Nukleotid-Modifikation einschließt, welche für die Polymerase keine komplementäre Nukleobase darstellt, wenn die Synthese mit ausschließlich natürlichen dNTP (dATP, dCTP, dGTP, dTTP oder dUTP) durchgeführt wird.

Beispielsweise können Iso-dG bzw. Iso-dC Nukleotid-Modifikationen als einzelne, vorzugsweise aber mehrere (mindestens 2 bis 20) Nukleotid-Modifikationen im zweiten Bereich des ersten Primer-Oligonukleotids integriert sein. Weitere Beispiele für Nukleobasen-Modifikationen stellen verschiedene Modifikationen des erweiterten genetischen Alphabets dar. Solche Nukleotid-Modifikationen unterstützen vorzugsweise keine komplementäre Basen-Paarung mit natürlichen Nukleotiden, so dass eine Polymerase (zumindest theoretisch) kein Nukleotid aus der Reihe (dATP, dCTP, dGTP, dTTP oder dUTP) einbaut. In der Realität kann es dennoch zum rudimentären Einbau kommen, insbesondere bei höheren Konzentrationen von dNTP-Substraten und prolongierten Inkubationszeiten (z.B. 60 min oder länger). Daher sollen bevorzugt mehrere solche Nukleotid-Modifikationen positioniert an benachbarten Stellen zum Einsatz kommen. Der Stopp der Polymerase-Synthese wird durch Fehlen von passenden komplementären Substraten für diese Modifikationen bewirkt. Oligonukleotide mit Iso-dC bzw. Iso-dG können mit Standardverfahren synthetisiert werden und sind von mehreren kommerziellen Anbietern erhältlich (z.B. Trilink-Technologies, Eurogentec, Biomers GmbH). Wahlweise kann auch die Sequenz des ersten Bereichs des Aktivator-Oligonukleotids an die Sequenz eines solchen zweiten Primer-Bereichs angepasst werden. Dabei können komplementäre Nukleobasen des erweiterten genetischen Alphabets in den ersten Bereich des Aktivator-Oligonukleotides entsprechend während der chemischen Synthese intergriert werden. Beispielsweise kann Iso-dG im zweiten Bereich des ersten Primer-Nukleotids integriert sein, sein komplementäres Nukleotid (Iso-dC-5-Me) kann an der passenden Stelle im ersten Bereich des Aktivator-Oligonukleotids platziert werden.

Zusammenfassend, kann die Termination der Synthese von Polymerase im zweiten Bereich auf auf verschiedene Art und Weise erreicht werden. Diese Blockade erfolgt bevorzugt allerdings erst wenn Polymerase den ersten Bereich des ersten Primer-Oligonukleotides kopiert hat. Damit wird sichergestellt, dass ein zweites Primer-Verlängerungsprodukt eine passende Primer-Bindungsstelle in seinem 3'-Segment besitzt. Diese Primer-Bindungsstelle wird im Rahmen der Strangverdrängung freigelegt und steht somit für eine erneute Bindung eines weiteren ersten Primer-Oligonukleotids zur Verfügung.

Bei der Synthese des komplementären Stranges zum ersten Primer-Verlängerungsprodukt bleibt die Primer-Verlängerungsreaktion vor dem Polynukleotid-Schwanz stehen. Da dieser Polynukleotid-Schwanz für die Interaktion mit dem Aktivator-Oligonukleotid dadurch einzelsträngig bleibt und somit für Bindung zur Verfügung steht, unterstützt dieser die Initiierung der Strangverdrängungs-Reaktion durch das Aktivator-Oligonukleotid, indem er den entsprechenden komplementäre Segmente des Aktivator-Oligonukleotids in unmittelbare Nähe des passenden Duplex-Endes bringt. Der Abstand zwischen dem komplementären Teil des Aktivator-Oligonukleotides (zweite Bereich) und dem komplementären Teil des verlängerten Primer-Oligonukleotid (erste Bereich) wird dadurch auf ein Minimum reduziert. Solche räumliche Nähe erleichtert die Initiierung der Strangverdrängung.

Im Rahmen einer schematischen Darstellung einer nukleinsäurevermittelten Strangverdrängungs-Reaktion liegt nun eine komplementäre Sequenz von Aktivator-Oligonukleotid unmittelbar in der Nähe des passenden Duplex-Endes. Dabei kommt es zu Konkurrenz um die Bindung an den ersten Bereich des ersten Primer-Oligonukleotids zwischen dem Strang des Aktivator-Oligonukleotides und dem zum Primer komplementären Matrizenstrang. Durch repetetive Schließung und Formierung von Basenpaarung zwischen dem Primer-Bereich und dem komplementären Segment des Aktivator-Oligonukleotids (zweiter Bereich des Aktivator-Nukleotides) bzw. dem komplementären Segment des Matrizenstranges kommt es zu Initiierung des nukleinsäurevermittelten Strangverdrängungs-Vorgangs.

Im Allgemeinen ist die Ausbeute der Initiierung der Stang-Verdrängung ist umso höher, je näher der entsprechende komplementäre Sequenzabschnitt des Aktivator-Oligonukleotides zum komplementären Segment des Primer-Bereichs liegt. Bei Vergrößerung dieses Abstandes sinkt dagegen die Ausbeute der Initiierung der Strangverdrängung.

Im Rahmen der vorliegenden Erfindung ist es nicht zwingend notwendig, dass die Initiierung der Strangverdrängung mit maximaler Ausbeute funktioniert. Daher können Abstände zwischen dem 5'-Segment des ersten Primer-Bereichs des ersten Primer-Oligonukleotid, welcher eine Bindung mit einem komplementären Strang der Matrize eingeht und eine komplementäre Duplex bildet, und einem entsprechend komplementären Sequenzabschnitt im Aktivator-Oligonukleotides, wenn gebunden an Polynukleotid-Schwanz des zweiten Bereichs des ersten Primer-Oligonukleotides in folgenden Bereichen liegen: zwischen 0,1 und 20 nm, besser zwischen 0,1 und 5 nm, noch besser zwischen 0,1 und 1 nm. Im bevorzugten Fall beträgt diese Distanz weniger als 1 nm. In anderen Einheiten ausgedruckt entspricht dieser Abstand einer Strecke von weniger als 200 Atomen, noch besser weniger als 50 Atomen, noch besser weniger als 10 Atomen. Im bevorzugten Fall ist diese Distanz ein Atom. Die Angaben zur Distanz sollen lediglich zur Orientierung dienen und veranschaulichen, dass kürzere Distanzen zwischen diesen Strukturen bevorzugt werden. Die Messung dieser Distanz ist in vielen Fällen nur durch Analyse der genauen Strukturen von Oligonukleotiden und Ausmessung der Messung von Sequenzabständen bzw. von Linker-Längen möglich.

### Bevorzugte Ausführungsformen des Aktivator-Oligonukleotids

Ein Aktivator-Oligonukleotid (Fig. 10) umfasst:
- Einen ersten einzelsträngigen Bereich, welcher an den Polynukleotid-Schwanz des zweiten Bereichs des ersten Primer-Oligonukleotides binden kann
- Einen zweiten einzelsträngigen Bereich, welcher an den ersten Bereich des ersten Primer-Oligonukleotides im wesentlich komplementär binden kann
- Einen dritten einzelsträngigen Bereich, welcher zumindest zu einem Segment des Verlängerungsprodukts des ersten Primer-Verlängerungsprodukts im Wesentlichen komplementär ist
Und das Aktivator-Oligonukleotid dient nicht als Matrize für Primerverlängerung des ersten oder des zweiten Primer-Oligonukleotids

Im Allgemeinen wird die Sequenz des dritten Bereichs des Aktivator-Oligonukleotides an die Sequenz der zu amplifizierenden Nukleinsäure angepasst, da diese als Matrize für die Reihenfolge der Nukleotide im Verlängerungsprodukt eines ersten Primers maßgeblich ist. Die Sequenz des zweiten Bereichs von Aktivator-Oligonukleotid wird an die Sequenz des ersten Primer-Bereiches angepasst. Die Struktur des ersten Bereichs des Aktivator-Oligonukleotides wird an die Sequenz des zweiten Bereichs des ersten Primer-Oligonukleotides angepasst, vor allem an die Beschaffenheit des Polynukleotid-Schwanzes.

Ein Aktivator-Oligonukleotid kann auch weitere Sequenz-Segmente einschließen, welche nicht zum ersten, zweiten oder dritten Bereich gehören. Diese Sequenzen können beispielsweise als flankierende Sequenzen am 3'- und am 5'-Ende angebracht werden. Vorzugsweise stören diese Sequenzsegmente die Funktion des Aktivator-Oligonukleotids nicht.

Die Struktur des Aktivator-Oligonukleotids weist bevorzugt folgende Eigenschaften auf:
Die einzelnen Bereiche sind untereinander kovalent gebunden. Die Bindung kann beispielsweise via konventionelle 5'-3' Bindung erfolgen. Es kann eine beispielsweise eine Phospho-Diesterbindung oder eine nuklease resistente Phospho-Thioester Bindung verwendet werden.

Ein Aktivator-Oligonukleotid kann mittels seines ersten Bereichs an den Polynukleotid-Schwanz des ersten Primer-Oligonukleotides binden, wobei die Bindung vorwiegend durch Hybridisierung komplementärer Basen vermittelt wird. Die Länge dieses ersten Bereichs beträgt 3 - 80 Nukleotide, vorzugsweise 4 - 40 Nukleotide, besonders bevorzugt 6 - 20 Nukleotide. Das Ausmaß an Sequenzübereinstimmung zwichen der Sequenz des ersten Bereichs des Aktivator-Oligonukleotids und der Sequenz des zweiten Bereichs des ersten Primer-Oligonukleotide kann zwischen 20% und 100%, vorzugsweise zwischen 50% und 100%, besonders bevorzugt zwischen 80% und 100% liegen. Die Bindung des ersten Bereichs des Aktivator-Oligonukleotids sollte vorzugsweise spezifisch an den zweiten Bereich des ersten Primer-Oligonukleotid unter Reaktionsbedinungen erfolgen.

Die Sequenz des ersten Bereichs des Aktivator-Oligonukleotids ist vorzugsweise dermaßen gewählt, dass die Anzahl von komplementären Basen, welche mit dem zweiten Bereich des ersten Primer-Oligonukleotids komplementär binden können, zwischen 1 und 40, besser zwischen 3 und 20, bevorzugt zwischen 6 und 15 liegt.

Da das Aktivator-Oligonukleotid keine Matrize für Polymerase darstellt, kann es Nukleotid-Modifikationen einschließen, welche die Polymerase-Funktion nicht unterstützen. Das Aktivator-Oligonukleotid kann in seinem ersten Bereich Nukleotide und/oder Nukleotid-Modifikationen einschließen, welche aus der folgenden Liste ausgewählt sind: DNA, RNA, LNA, PNA, PTO (phosphorothioate), Morpholino, 2'-O-Alkyl-RNA, 2'-Halogen-RNA, 2'-Amino-RNA etc. Diese Nukleotide oder Nukleotid-Modifikationen sind untereinander beispielsweise durch konventionelle 5'-3'- Bindung verknüpft. Es kann beispielsweise eine Phospho-Diesterbindung oder eine nuklease resistente Phospho-Thioester Bindung verwendet werden.

Das Aktivator-Oligonukleotid kann in seinem ersten Bereich Nukleotide und/oder Nukleotid-Modifikationen einschließen, wobei die Nukleobasen aus der folgenden Liste ausgewählt sind: Adenine und seine Analoga, Guanine und seine Analoga, Cytosine und seine Analoga, Uracil und seine Analoga, Thymine und seine Analoga, Inosine und seine Analoga, 2-Amino-Adenin und seine Analoga, Iso-Cytosine und seine Analoga, Iso-Guanine und seine Analoga.

Das Aktivator-Oligonukleotid kann in seinem ersten Bereich Nicht-Nukleotid-Verbindungen einschließen, welche aus der folgenden Liste ausgewählt sind: Interkallierende Stoffe, welche Bindungsstärke zwischen dem Aktivator-Oligonukleotid und dem ersten Primer-Oligonukleotid beeinflussen können, z.B. MGB, Naphthalene etc.

Das Aktivator-Oligonukleotid kann in seinem ersten Bereich Nicht-Nukleotid-Verbindungen, z.B. Linker wie C3, C6, HEG Linker, welche einzelne Segmente des ersten Bereichs untereinander verknüpfen.

Das Aktivator-Oligonukleotid kann mittels seines zweiten Bereichs an den ersten Primer-Bereich des ersten Primer-Oligonukleotids binden, wobei die Bindung im Wesentlichen durch Hybridisierung komplementärer Basen vermittelt wird.

Die Länge des zweiten Bereichs des Aktivator-Oligonukleotids ist an die Länge des ersten Bereichs des ersten Primer-Oligonukleotid angepasst und stimmt mit dieser vorzugsweise überein. Sie liegt zwischen ca. 3-30 Nukleotiden, vorzugsweise zwischen 5 und 20 Nukleotiden. Die Sequenz des zweiten Bereichs von Aktivator-Oligonukleotid ist vorzugsweise komplementär zum ersten Bereich des ersten Primer-Oligonukleotid. Das Maß der Übereinstimmung in Komplementarität liegt vorzugsweise zwischen 95% und 100%, bevorzugt bei 100%. Der zweite Bereich von Aktivator-Oligonukleotid schließt vorzugsweise Nukleotid-Modifikationen ein, welche Polymerase die Verlängerung des ersten Primer-Oligonukleotides verhindern, beispielsweise 2'-O-Alkyl RNA-Analoga (z.B. 2'-O-Me Nukleotid-Modifikatioien), PNA oder Morpholino.

Die Sequenz-Länge und ihre Beschaffenheit des ersten und des zweiten Bereichs des Aktivator-Oligonukleotides sind vorzugsweise dermassen gewählt, dass die Bindung dieser Bereiche an das erste Primer-Oligonukleotid unter Reaktionsbedinungen reversibel ist. Das bedeutet, dass Aktivator-Oligonukleotid und das erste Primer-Oligonukleotid zwar spezifisch an einander binden können, diese Bindung führt allerdings nicht zu Ausbildung eines unter Reaktionsbedinungen stabilen Komplexes aus beiden Elementen. Vielmehr wird ein Gleichgewicht zwischen einer gebunden Komplex-Form aus Aktivator-Oligonukleotid und dem ersten Primer-Oligonukleotid und einer freien Form von einzelnen Komponenten unter Reaktionsbedingungen angestrebt. Damit soll sichergestellt werden, dass zumindest ein Teil der ersten Primer-Oligonukleotide unter Reaktionsbedinungen in freier Form vorliegt mit der Matrize interagieren kann, um eine Primer-Verlängerungsreaktion zu initiieren.

Die Sequenz-Länge und ihre Beschaffenheit des ersten und des zweiten Bereichs des Aktivator-Oligonukleotides sind in einer Ausführungsform so gewählt, dass das Verhältnis zwischen einem Anteil eines freien Aktivator-Oligonukleotids und einem Anteil eines Aktivator-Oligonukleotid im Komplex mit einem ersten Primer-Oligonukleotid von 1:100 bis 100:1, vorzugsweise von 1:30 bis 30:1, besonders bevorzugt von 1:10 bis 10:1 liegt. Das Verhältnis zwischen einem Anteil eines freien ersten Primer-Oligonukleotid und einem Anteil eines ersten Primer-Oligonukleotids im Komplex mit einem Aktivator-Oligonukleotid liegt dabei in Bereichen von 1:100 bis 100:1, vorzugsweise von 1:30 bis 30:1, besonders bevorzugt von 1:10 bis 10:1.

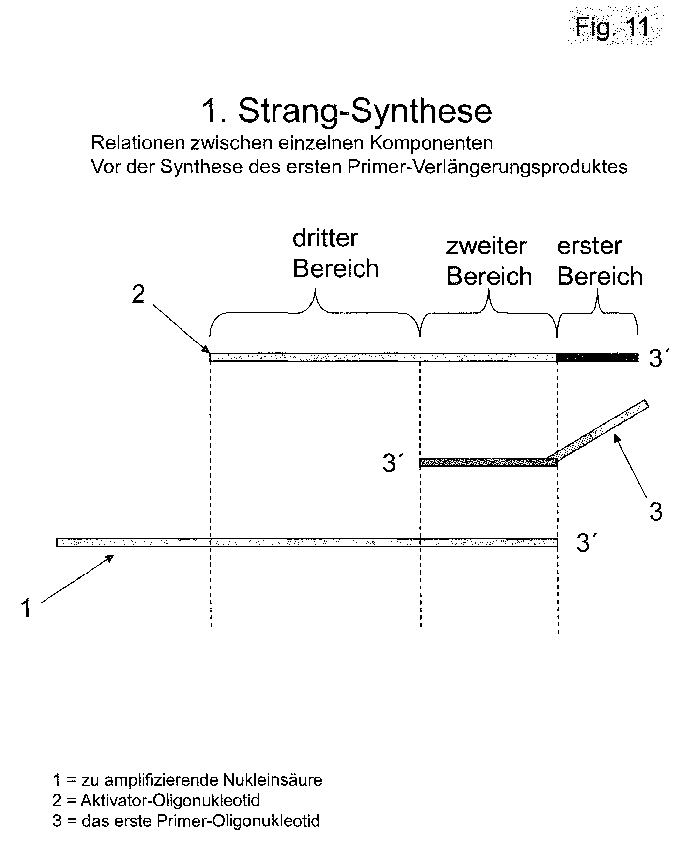

Das Aktivator-Oligonukleotid kann mittels seines dritten Bereichs an mindestens ein Segment des spezifisch synthetisierten Verlängerungsprodukts des ersten Primer-Oligonukleotids binden (Fig. 11 bis 21). Die Bindung erfolgt im Wesentlichen durch Hybridisierung komplementärer Basen zwischen dem Aktivator-Oligonukleotid und dem von Polymerase synthetisierten Verlängerungsprodukt.

Zu Unterstützung der Strangverdrängungsreaktion soll die Sequenz des dritten Bereichs vorzugsweise eine hohe Komplementarität zum Verlängerungsprodukt aufweisen. In einer Ausführungsform ist die Sequenz des dritten Bereichs zu 100% dem Verlängerungsprodukt komplementär.

Die Bindung des dritten Bereichs erfolgt vorzugsweise an das Segment des Verlängerungsproduktes, welches unmittelbar an den ersten Bereich des ersten Primer-Oligonukleotides anschließt. Somit liegt das Segment des Verlängerungsprodukts vorzugsweise im 5'-Segment des gesamten Verlängerungsprodukts des ersten Primer-Oligonukleotids.

Die Bindung des dritten Bereichs des Aktivator-Oligonukleotids erfolgt vorzugsweise nicht über die gesamte Länge des Verlängerungsprodukts des ersten Primer-Oligonukleotides. Vorzugsweise bleibt ein Segment am 3'-Ende des Verlängerungsprodtes ungebunden. Dieses 3'-ständige Segment ist für die Bindung des zweiten Primer-Oligonukleotides notwendig.

Die Länge des dritten Bereichs ist entsprechend dermassen angepasst, dass der dritte Bereich zum einen an das 5'-ständige Segment des Verlängerungsproduktes bindet, andererseits das 3'-ständige Segment des Verlängerungsproduktes nicht bindet.

Die Gesamtlänge des dritten Bereichs des Aktivator-Oligonukleotids beträgt von 2 bis 100, vorzugsweise von 6 bis 60, besonders bevorzugt von 10 bis 40 Nukleotide oder ihre Äquivalente. Über diese Länge kann das Aktivator-Oligonukleotid mit dem Segment des Verlängerungsproduktes komplementäre Bindung eingehen und damit dieses 5'-ständige Segment des Verlängerungsprodukts aus der Bindung mit dem komplementären MatrizenStrang verdrängen.

Die Länge des 3'-ständigen Segments des Verlängerungsproduktes, welches nicht vom Aktivator-Oligonukleotid gebunden wird, beträgt beispielsweise Bereiche zwischen 5 und 60 Nukleotide, vorzugsweise zwischen 10 und 40, bevorzugt zwischen 15 und 30 Nukleotide.

Dieses 3'-ständige Segment des Verlängerungsprodukts wird nicht von Aktivator-Oligonukletid aus der Bindung mit dem Matrizenstrang verdrängt. Auch bei vollständig gebundenem dritten Bereich des Aktivator-Oligonukleotid an sein komplementäres Segment des Verlängerungsproduktes bleibt das Verlängerungsprodukt via sein 3'-ständiges Segment mit dem Matrizenstrang gebunden. Die Bindungsstärke dieses Komplexes ist vorzugsweise so gewählt, dass er unter Reaktionsbedinungen spontant dissoziieren kann. Dies kann bespielsweise dadurch erreicht werden, dass die Schmelztemperatur dieses Komplexes aus dem 3'-ständigen Segment des Verlängerungsprodukts des ersten Primer-Oligonukleotid und seinem Matrizenstrang etwa im Bereich der Reaktionstemperatur liegt oder unterhalb der Reaktionstemperatur. Bei geringer Stabilität deses Komplexes führt eine vollständige Bindung des dritten Bereichs des Aktivator-Oligonukleotid an das 5'-ständige Segment des Verlängerungsproduktes zu einer schnellen Dissoziation der ersten Primer-Verlängerungsprodukts von seinem Matrizenstrang.

Das Aktivator-Oligonukleotid hat insgesamt eine passende Struktur, um seine Funktion auszuführen: unter entsprechenden Reaktionsbedinungen ist es in der Lage, das verlängerte erste Primer-Oligonukleotid aus der Bindung mit dem Matrizenstrang sequenzspezifisch zu verdrängen, wodurch der Matrizenstrang in einzelsträngige Form überführt wird und somit für weitere Bindungen mit einem neuen ersten Primer-Oligonukleotid und deren zielsequenzspezifische Verlängerung durch Polymerase zur Verfügung steht.

Um die Funktion der Strangverdrängung zu erfüllen, sollten Bereiche ein, zwei und drei des Aktivator-Oligonukleotids vorwiegend in einzelsträngiger Form unter Reaktionsbedingungen vorliegen. Daher sollten doppelsträngige selbstkomplementäre Strukturen (z.B. Hairpins) in diesen Bereichen sollten nach Möglichkeit vermieden werden, da sie Funktionalität des Aktivator-Oligonukleotides herabsetzen können.

Das Aktivator-Oligonukleotid soll im erfindungsgemäßen Verfahren nicht als Matrize auftreten, daher soll das erste Primer-Oligonukleotid, wenn angelagert an das Aktivator-Oligonukleotid unter Reaktionsbedingungen, nicht von Polymerase verlängert werden.

Dies wird vorzugsweise durch Verwendung von Nukleotid-Modifikationen erreicht, welche die Polymerase am Kopieren des Stranges hindern. Vorzugsweise bleibt das 3'-Ende des ersten Primer-Oligonukleotid nicht verlängert, wenn das erste Primer-Oligonukleotid an das Aktivator-Oligonukleotid unter Reaktionsbedinungen bindet.

Das Ausmaß der Blockade / Hinderung / Verlangsamung / Erschwerung der Reaktion kann zwischen vollständiger Ausprägung dieser Eigenschaft (z.B. 100% Blockade unter gegebenen Reaktionsbedingungen) und einer partiellen Ausprägung dieser Eigenschaft liegen (z.B. 30-90% Blockade unter gegebenen Reaktionsbedingungen). Bevorzugt werden Nukleotid-Modifikationen, welche einzeln oder zusammen mehr als 70%, bevorzugt mehr als 90%, bevorzugter mehr als 95%, und besonders bevorzugt 100% die Verlängerung eines Primers verhindern können.

Die Nukleotid-Modifikationen können Basen-Modifikationen und / oder Zucker-Phosphat-Rest Modifikationen umfassen. Die Zucker-Phosphat-Modifikationen sind bevorzugt, da durch Kombination mit konventionellen Nukleobasen eine beliebige komplementäre Sequenz eines Aktivator-Oligonukleotides zusammengestellt werden kann. Die Nukleotide mit Modifikationen im Zucker-Phosphat-Rest, welche zu Hinderung bzw. Blockade der Synthese der Polymerase führen, einschließen beispielsweise ein: 2'-O-Alkyl-Modifikationen (z.B. 2'-O-Methyl Nukleotid-Modifikationen), 2'-Amino-2'-Deoxy-Nukleotid-Modifikationen, 2'-Amino-Alkyl-2'-Deoxy-Nukleotid-Modifikationen, PNA, Morpholino-Modifikationen.

Die Blockade kann sowohl durch eine einzelne Nukleotid-Modifikation erfolgen oder erst durch Kopplung von mehreren Nukleotid-Modifikationen in Reihe erfolgen. Beispielsweise können mindestens 2, vorzugsweise mindestens 5, besonders bevorzugt mindestens 10 solcher Nukleotid-Modifikationen nebeneinander im Aktivator-Oligonukleotid gekoppelt sein. Ein Aktivator-Oligonukleotid kann eine einheitliche Art von Nukleotid-Modifikationen aufweisen oder auch mindestens zwei verschiedene Arten der Nukleotid-Modifizierung umfassen.

Die Position dieser solcher Nukleotid-Modifikationen im Aktivator-Oligonukleotid soll vorzugsweise die Polymerase daran hindern, das 3'-Ende eines am Aktivator-Oligonukleotid gebundenen ersten Primer-Oligonukleotids zu verlängern.

In einer Ausführungsform sind solche Nukleotid-Modifikationen im zweiten Bereich des Aktivator-Oligonukleotids lokalisiert. In einer Ausführungsform sind solche Nukleotid-Modifikationen im dritten Bereich des Aktivator-Oligonukleotids lokalisiert. In einer Ausführungsform sind solche Nukleotid-Modifikationen im zweiten und im dritten Bereich des Aktivator-Oligonukleotids lokalisiert.

Beispielsweise besteht der zweite Bereich des Aktivator-Oligonukleotids zu mindestens 20% seiner Positionen aus solchen Nukleotid-Modifikationen, vorzugsweise zu mindestens 50%.

Beispielsweise besteht der dritte Bereich des Aktivator-Oligonukleotids zu mindestens 20% seiner Positionen aus solchen Nukleotid-Modifikationen, vorzugsweise zu mindestens 50%.

Die Sequenz der Nukleobasen von diesen Nukleotid-Modifikationen sind den Anforderungen an die Sequenz im jeweiligen Bereich angepasst.

Den restlichen Anteil können beispielsweise natürliche Nukleotide ausmachen, oder Nukleotid-Modifikationen, welche die Polymerasen-Funktion gar nicht oder nur unwesentlich hindern z.B. DNA-Nukleotide, PTO-Nukleotide, RNA-Nukleotide. Dabei können weitere Modifikationen wie Inosine, 2-Amino-Adenosin, 7-Deaza-Adenosin, 7-Deaza-Guanosin, 5-Propyl-Cytosin, 5-Propyl-Uridin oder Nicht-Nukleotid-Modifikationen wie MGB-M etc. z.B. zur Anpassung von Bindungsstärken von einzelnen Bereichen der Aktivator-Oligonukleotide verwendet werden.

Ein Segment des Aktivator-Oligonikleotids mit Nukleotid-Modifikationen, welche Verlängerung von 3'-Ende eines an Aktivator-Oligonukleotid gebundenen ersten Primer-Oligonukleotids durch Polymerase verhindern, wird als "zweite Blockierungs-Einheit" bezeichnet. Die Länge dieses Segments kann zwischen 1 bis 50 Nukletid-Modifikationen einschließen, vorzugsweise zwischen 4 und 30. Dieses Segment kann beispielsweise dermassen im Aktivator-Oligonukleotid lokalisiert sein, dass das 3'-Ende des gebundenen ersten Primer-Oligonukleotids in diesem Segment liegt. Somit kann dieses Segment die Bereiche zwei und drei überspannen.

In einer Ausführungsform werden vorzugsweise keine Linker-Strukturen, wie C3, C6, HEG-Linker zur Verhinderung der Verlängerung des 3'-Ende eines an das Aktivator-Oligonukleotid gebundenen ersten Primer-Oligonukleotids verwendet.

### Beispiele:

### Material und Methoden:

Reagenzien wurden von folgenden kommerziellen Anbietern bezogen:
- nicht modifizierte und modifizierte Oligonukleotide (Eurofins MWG, Eurogentec, Biomers, Trilink Technologies, IBA Solutions for Life Sciences)
- Polymerasen NEB (New England Biolabs)
- dNTP's: Jena Bioscience
- interkallierender EvaGreen Farbstoff: Jena Bioscience
- Puffer-Substanzen und andere Chemikalien: Sigma-Aldrich
- Plastikware: Sarstedt

Lösung 1 (Puffer-Lösung 1):
- Kalium Glutamat, 50 mmol/l, pH 8,0
- Magnesium Acetat, 10 mmol/l
- Triton X-100, 0,1% (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

Lösung 2 (Amplifikationsreaktion Lösung 2) (Standard-Reaktion):
- Kalium Glutamat, 50 mmol/l, pH 8,0
- Magnesium Acetat, 10 mmol/l
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1% (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).
- Primer 1: 10 µmol/l
- Primer 2: 10 µmol/l
- Aktivator-Oligonukleotid: 10 µmol/l
- Matrizenstrang - variable Konzentrationen, wie in Beispielen indiziert.

Alle Konzentrationen sind Angaben der Endkonzentrationen in der Reaktion. Abweichungen von der Standard-Reaktion werden entsprechend angegeben.

Die Schmelztemperatur (Tm) von beteiligten Komponenten wurde bei Konzentration von 1 µmol/l von jeweiligen Komponenten in Lösung 1 bestimmt. Abweichende Parameter sind jeweils angegeben.

### Allgemeine Informationen zu Reaktionen

Primer-Verlängerungsreaktionen und Amplifikation wurden standardmäßig bei einer Reaktionstemperatur von 65°C durchgeführt. Abweichungen werden angegeben.

Der Reaktionsstart erfolgte durch Erhitzen der Reaktionslösungen auf Reaktionstemperatur, da Bst 2.0 Polymerase Warmstart bei niedrigeren Temperauren größtenteils in ihrer Funktion durch ein temperatur-sensitives Oligonukleotid gehemmt ist (laut Hersteller-Angaben). Die Polymerase wird zunehmend aktiver ab einer Temperatur von ca. 45°C, bei einer Temperatur von 65°C konnten keine Unterschiede zwischen Polymerase Bst 2.0 und Bst 2.0 Warmstart festgestellt werden. Um der extensiven Bildung von Nebenprodukten (z.B. Primer-Dimeren) während der Vorbereitungsphase einer Reaktion vorzubeugen, wurde Polymerase Bst 2.0 Warmstart verwendet. Abweichungen davon werden speziell angegeben.

Reaktionsstopp erfolgte durch Erhitzen der Reaktionslösung auf über 80°C, z.B. 10 min bei 95°C. Bei dieser Temperatur wird Polymerase Bst 2.0 irreversible denaturiert und das Ergebnis der Synthese-Reaktion kann nachträglich nicht geändert werden.

Reaktionen wurden in einem Thermostat mit einer Fluoreszenz-Messvorrichtung ausgeführt. Zu diesem Zweck wurde ein kommerzielles Real-Time PCR Gerät verwendet, StepOne Plus (Applied Biosystems, Thermofischer). Das Reaktionsvolumen betrug standardmäßig 10 µl. Abweichungen davon werden angegeben.

Sowohl Endpunkt-Bestimmung als auch kinetische Beobachtungen wurden vorgenommen. Bei Endpunktbestimmungen wurde das Signal beispielsweise von an Nukleinsäuren gebundenen Farbstoffen registriert, z.B. von TMR (Tetramethyl-Rhodamine, auch TAMRA genannt) oder von FAM (Fluorescein). Die Wellenlängen zur Anregung und Messung der Fluoreszenzsignale von FAM und TMR sind als Werkeinstellungen bei StepOne Plus Real-Time PCR Gerät gespeichert. Ebenfalls wurde ein interkallierender Farbstoff (EvaGreen) bei Endpunktmessungen vorgenommen, z.B. bei Messung einer Schmelzkurve). EvaGreen ist ein interkallierender Farbstoff und ist ein Analogon des häufig eingesetzten Farbstoff Sybrgreen, allerdings mit etwas geringerer Inhibierung von Polymerasen. Die Wellenlängen zur Anregung und Messung der Fluoreszenzsignale von SybreGreen und EvaGreen sind identisch und sind als Werkeinstellungen bei StepOne Plus Real-Time PCR Gerät gespeichert. Die Fluoreszenz kann mittels eingebauter Detektoren fortlaufend, d.h. "online" oder "Real-Time" detektiert werden. Da die Polymerase während ihrer Synthese einen Doppelstrang synthetisiert, konnte diese Technik zu kinetischen Messungen (Real-Time Monitoring) der Reaktion verwendet werden. Aufgrund von einem gewissen Cross-Talk zwischen Farb-Kanälen bei StepOne Plus Gerät wurde teilweise erhöhte basale Signal-Intensität bei Messungen beobachtet, bei welchen z.B. TMR-markierte Primer in Konzentration von über 1 µmol/l (z.B. 10 µmol/l) eingesetzt waren. Es wurde beobachtet, dass TMR-Signal in Sybre-Green-Kanal zu erhöhten Grundwerten führt. Diese erhöhten Grundwerte wurden bei Berechnungen berücksichtigt.

Die kinetischen Beobachtungen von Reaktionsverläufen wurden routinemäßig mittels Fluoreszenzsignalen von Fluoreszein (FAM-TAMRA Fret-Paar) bzw. interkallierenden Farbstoffen (EvaGreen) aufgenommen. Zeitabhängigkeit des Signal-Verlaufs wurde registriert (Real-Time Signal Erfassung bei StepOne plus PCR Gerät). Ein Anstieg des Signals während einer Reaktion vergleichen zu einer Kontroll-Reaktion wurde je nach Aufbau des Ansatzes interpretiert. Beispielsweise, eine Zunahme des Signals bei Verwendung von Evagreen-Farbstoff wurde als Hinweise auf Zunahme der Menge an doppelsträngigen Nukleinsäureketten während der Reaktion interpretiert, und somit als Ergebnis einer stattfindenden Synthese durch DNA-Polymerase gewertet.

Bei einigen Reaktionen wurde im Anschluss an die Reaktion eine Schmelzkurvenbestimmung durchgeführt. Solche Messungen erlauben Rückschlüsse auf das Vorliegen von Doppelsträngen, welche beispielsweise interkallierende Farbstoffe aufnehmen können und dadurch die Signalintensität von Farbstoffen deutlich verstärken. Mit der steigenden Temperatur sinkt der Anteil von Doppelsträngen und die Signal-Intensität sinkt ebenfalls. Das Signal ist von der Länge von Nukleinsäureketten und von der Sequenzzusammensetzung abhängig. Diese Technik ist einem Fachmann hinreichend bekannt.

Bei Verwendung der Schmelzkurven-Analyse im Zusammenhang mit Reaktionen, welche signifikante Anteile von modifizierten Nukleinsäureketten (z.B. Aktivator-Oligonukleotide oder Primer) enthielten, wurde festgestellt, dass sich das Signal von Farbstoff EvaGreen beispielsweise unterschiedlich zwischen B-Form der DNA und A-Form von modifizierten Nukleinsäureketten verhalten kann. Beispielsweise wurde bei B-Form der doppelsträngigen Nukleinsäureketten (gewöhnlich angenommen für klassische DNA-Abschnitte) eine höhere Signal-Intensität beobachtet, als bei doppelsträngigen Nukleinsäureketten mit der gleichen Sequenz von Nukleobasen, welche eine A-Form ähnliche Konformation annehmen können (z.B. durch mehrere 2'-O-Me Modifikationen von Nukleotiden). Diese Beobachtung wurde beim Einsatz von interkallierenden Farbstoffen berücksichtigt.

Bei Bedarf wurde die Reaktion mittels Kapillarelektrophorese analysiert und die Länge von gebildeten Fragmenten mit einem Standard verglichen. Als Vorbereitung auf die Kapillarelektrophorese wurde die Reaktionsmischung in einem Puffer (Tris-HCl, 20 mmol/l, pH 8,0, und EDTA, 20 mmol/l, pH 8,0) dermaßen verdünnt, dass die Konzentration von markierten Nukleinsäuren ca. 20 nmol/l betrug. Die Kapillar-Elektrophorese wurde bei Firma GATC-Biotech (Konstanz, Deutschland) als Auftragsleistung durchgeführt. Nach Angaben des Anbieters wurde die Kapillarelektrophorese auf einem ABI 3730 Cappilary Sequencer unter Standard-Bedingungen für eine Sanger-Sequenzierung unter Verwendung von POP7 Gelmatrix, bei ca. 50°C durchgeführt und konstanter Spannung (ca. 10 kV) durchgeführt. Die verwendeten Bedinungen führten zu Denaturierung von Doppelsträngen, so dass in der Kapillarelektrophorese die einzelsträngige Form von Nukleinsäureketten separiert wurde. Die Elektrophorese ist eine Standardtechnik in der genetischen Analyse. Die automatisierte Kapillar-Elektrophorese wird heute routinemäßig bei Sanger-Sequenzierung eingesetzt. Das Fluoreszenz-Signal wird während der Kapillar-Elektrophorese kontinuierlich aufgezeichnet (gewöhnlich unter Verwendung von virtuellen Filtern), so dass ein Elektrophorogramm entsteht, bei welchem die Signal-Intensität mit der Dauer der Elektrophorese korreliert. Bei kürzeren Fragmenten, z.B. nicht verbrauchte Primer, beobachtet man einen frühren Signal-Peak, bei verlängerten Fragmenten kommt es zu einer zeitlichen Verschiebung der Signale proportional der Länge des extendierten Bereichs. Dank mitgeführten Kontrollen mit bekannten Längen lässt sich die Länge von extendierten Fragmenten ausmessen. Diese Technik ist einem Fachmann bekannt und wird ebenfalls standardmäßig bei Fragment-Längen-Polymorphismus eingesetzt.

### Beispiel 1:

### Nukleinsäureabhängige Strangverdrängung unter isothermalen Bedingungen.

Das Beispiel demonstriert die grundsätzliche Fähigkeit von einzelsträngigen komplementären Nukleinsäureketten zu einer nukleinsäureabhängigen, vorwiegend sequenzspezifischen Strangverdrängung in Abwesenheit von strangtrennenden Proteinen bzw. Enzymen (z.B. Helicasen oder Recombinase) und ohne Verbrauch von chemisch gespeicherten Energie in Form von ATP. Es wird demonstriert, dass ein unter Reaktionsbedinungen stabiler Doppelstrang (gebildet aus Strängen, hier genannt A1 und B1, so dass ein A1:B1-Doppelstrang resultiert) von einer einzelsträngigen Nukleinsäurekette (hier genannt C1-Oligonukleotid), welche im wesentlichen zu bestimmten Segmenten des Strang A1 komplementär ist, geöffnet werden kann. Als Ergebnis dieser Doppelstrang-Öffnung wird Strang B1 in einzelsträngige Form überführt, gleichzeitig entsteht ein neuer A1:C1-Doppelstrang. Die Sequenz der in diesem Beispiel verwendeten C1-Nukleinsäureketten wurde dermaßen konstruiert, dass eine nukleinsäureketten-vermittelte sequenzspezifische Strangverdrängungsreaktion an einem doppelsträngigen DNA-Fragment unter isothermalen Bedinungen stattfinden kann. In diesem Beispiel wurde keine Polymerase zur Reaktion zugegeben, es wurden somit keine neuen Nukleinsäurestränge synthetisiert und es fand keine Amplifikation von Nukleinsäureketten statt.

Zu Detektion der Strangverdrängung und Trennung eines vorgebildeten Doppelstranges wurden beide zu trennende Stränge mit Fluorophoren markiert, so dass ein FRET-Paar (bestehend aus Fluorescein und TAMRA) gebildet wurde. Die Fluorophore wurden an einem Ende des A1:B1-Doppelstranges, d.h. an komplementären Enden der beiden Stränge, positioniert. Die hier verwendeten Nukleinsäurestränge wurden chemisch synthetisiert, die Fluorophore wurden während der chemischen Synthese an die entsprechenden Strangenden chemisch gekoppelt. Die Distanz zwischen Fluophoren, wenn beide Stränge aneinander komplementär gebunden sind, wurde dermassen gewählt, dass ein effektives Quenching der Fluorescein-Fluoreszenz durch TAMRA-Farbstoff erfolgen kann. Bei Trennung der Stränge durch irgendeine Einwirkung, z.B. durch Temperatur oder durch Strangverdrängung, kommt es zu Vergößerung der Distanz zwischen einzelnen Fluorophoren, so dass Intensität des Fluoreszenz-Signals des Fluorescein-Markers zunimmt. Die maximale Intensität des Fluorescein-Markers wird erreicht, wenn beide Stränge (A1 und B1) vollständig getrennt sind. Durch Beobachtung der Intensität der Fluoreszenz und ihre Abhängigkeit von Einwirkung verschiedener Parameter (z.B. Temperatur, Zugabe von komplementären oder nicht-komplementären Sequenzen zwecks Strangverdrängung etc.) kann man Rückschlüsse auf die Menge der noch aneinander gebundenen markierten Stränge ziehen.

Im Beispiel 1 wird das Prinzip der nukleinsäureabhängigen sequenzspezifischen Strangverdrängungsreaktion veranschaulicht. Sowohl die Kinetik der Strangverdrängungsreaktion als auch Temperatur-Abhängigkeit der Öffnung des vorgebildeten Doppelstranges (A1:B1) wurde beobachtet, indem das Fluoreszenz-Signal während des Reaktionsablaufs aufgezeichnet wurde. Sowohl isothermale Reaktionsbedinungen als auch Bedingungen mit einem Temperatur-Gradient wurden ausgetestet.

### Vorbereitung des Ausgangsmaterials:

### Doppelstrang (A1:B1)

Die Sequenzen für Doppelstrang A1:B1 sind künstliche Sequenzen, die zufällig ausgewählt wurden, wobei auf Abwesentheit von extensiven Hairpins und langen G-Segmenten, bzw. C-Segmenten geachtet wurde. Beide Stränge sind in der Lage, einen komplementären Doppelstrang auszubilden. Ein Doppelstrang hat eine Tm von ca. 75°C (ein weiterer Doppelstrang hat eine Tm von ca. 77°C s.u.), welche höher ist als die Reaktionstemperatur von 65°C. Damit ist solcher Doppelstrang unter Reaktionsbedinungen stabil. Strang A1 ist länger als jeweilige Strang B1, so dass ein 5'-Überhang entsteht. Dieser 5'-Überhang am A1-Strang wurde in Analogie zum Polynukleotid-Schwanz eines ersten Primer-Verlängerungsproduktes konzepiert. Zur Vereinfachung der Synthese enthielt dieser 5'-Überhang aber keine Modifikationen, welche Polymerase-Aktivität blockieren. Da im vorliegenden Beispiel keine Polymerase in der Reaktion vorhanden war, spielte die Ab- / Anwesentheit solcher Modifikationen eine untergeordnete Rolle. Dieser 5'-Überhang ist komplementär zum ersten Bereich der einzelsträngigen Nukleinsäurekette (C1), so dass eine spezifische Bindung der C1-Nukleinsäurekette an diesen Überhang unter Reaktionsbedinungen stattfinden kann. Dank dieser Bindung wird eine hinreichende räumliche Nähe zwischen einem Ende des A1:B1-Doppelstranges und entsprechenden komplementären Segmenten der einzelsträngigen C1-Nukleinsäureketten herbeigeführt, so dass eine Strangverdrängungsreaktion stattfinden kann. Als Ergebnis dieser Reaktion kann ein neuer Doppelstrang A1:C1 entstehen und B1-Strang kann in seine einzelstränige Form überführt werden.
**A1-Strang** SEQ ID NO: 1 ES-10-3504 5' CCGAAGCTCGCAGGAACTCAGAGTGTGGAGAGGACGATAGCTAGTCAGATGATGAAGTT 3'TMR

Die Sequenz besteht aus DNA. Eine 3'-endständige TAMRA-Modifikation (endständige TMR-Modifikation) wurde bereits während Synthese angebracht.
**B1-Strang** SEQ ID NO: 2 T-A6P-10-3506 5'FAM 5' CATCATCTGACTAGCTATCGTCCTCTCCACACTCTGAGTTCCTGCGAG 3'
**B1-Strang** SEQ ID NO: 3 T-A6P-10-3507 5'FAM 5' CATCATCTGACTAGCTATCGTCCTCTCCACACTCTGAGTTCCTGC 3'

Beide Stränge bestehen aus DNA. Eine 5'-endständige Fluorescein-Farbstoff-Modifikation (endständige FAM-Modifikation) wurde als Reporter ausgewählt und wurde bereits während Synthese an den jeweiligen Strang mit konventionellen Methoden der Oligonukleotid-Chemie angebracht.

Es wurden zwei Varianten des Doppelstranges generiert. Variante 1 enthälgt einen Doppelstrang mit einem kürzeren Überhang in 5'-Segment des A1-Stranges (6 Nukleotide), dieser Doppelstrang besteht aus SEQ ID NO: 1 und SEQ ID NO: 2), Variante 2 enthälgt einen Doppelstrang mit einem längeren Überhang in 5'-Segment des A1-Stranges (9 Nukleotide), dieser Doppelstrang besteht aus SEQ ID NO: 1 und SEQ ID NO: 3), die jeweils komplementäre Segmente sind unterstrichen. Überhänge am 3'-Segment des A1-Stranges wurden aus synthetischen Gründen designed und spielen in diesem Beispiel eine untergeordnete Rolle.
A1:B1-Doppelstrang (Variante 1) 
TMR 3´ ttGAAGTAGTAGACTGATCGATAGCAGGAGAGGTGTGAGACTCAAGGACGCTCGAAGCC SEQ ID NO: 1
FAM 5´ CATCATCTGACTAGCTATCGTCCTCTCCACACTCTGAGTTCCTGCGAG SEQ ID NO: 2
A1:B1-Doppelstrang (Variante 2)

TMR 3´ ttGAAGTAGTAGACTGATCGATAGCAGGAGAGGTGTGAGACTCAAGGACGCTCGAAGCC SEQ ID NO: 1
FAM 5´ CATCATCTGACTAGCTATCGTCCTCTCCACACTCTGAGTTCCTGC SEQ ID NO: 3

### Einzelsträngige Nukleinsäureketten (C1-Oligonukleotide):

Eine einzelsträngige Nukleinsäurekette (in diesem Beispiel als C1-Oligonukleotid genannt) kann bei bestimmten Voraussetzungen zu einer Strangverdrängungs-Reaktion am Strang A1:B1 führen unter gleichzeitiger Ausbildung eines neuen Doppelstrangs mit A1 (A1:C1-Doppelstrang). Dabei wird Strang B1 in seine einzelsträngige Form zumindest teilweise überführt. Im Extremfall kann eine Strangverdrängung zu einer vollständigen Dissoziation von A1:B1 führen, so dass B1-Strang von A1-Strang komplett getrennt ist.

C1-Oligonukleotide und Aktivator-Oligonukleotide haben eine ähnliche Funktion: sie vermitteln nukleinsäureabhängige Strangverdrängung am entsprechenden Doppelstrang. Die Bezeichnung C1-Oligonukleotid wird im vorliegenden Beispiel verwendet. Wegen Abwesenheit der Polymerase in diesem Beispiel können zwar auch C1-Oligonukleotide Polymerase-Blockierende Nukleotid-Modifikationen umfassen, die Anwesenheit dieser Modifikationen stellt allerdings keine Voraussetzung für die Demonstration der Strangverdrängung dar (s.u.). Die Bezeichnung Aktivator-Oligonukleotid wird in Beispielen verwendet, in welchen die Reaktionsmischung eine Polymerase umfasst und welche eine exponentielle Amplifikation demonstrieren. Im Unterschied zu C1-Oligonukleotiden müssen Aktivator-Oligonukleotide Polymerase-blockierende Nukleotid-Modifikationen einschließen.

Aufgrund ähnlicher Funktion von C1-Oligonukleotiden und den Aktivator-Oligonukleotiden, wird die Sequenzzusammensetzung von C1-Oligonukleotiden ähnlich dem Aufbau von Aktivator-Oligonukleotide gestaltet. Die C1-Oligonukleotide müssen die zu A1-Strang komplementären Segmente umfassen, damit die Strangverdrängung ablaufen kann. C1-Oligonukleotide verfügen über einen einzelsträngigen ersten, zweiten und dritten Bereich (siehe Struktur von Aktivator-Oligonukleotid). Mit dem ersten Bereich können C1-Oligonukleotide an den einzelsträngigen 5'-Überhang des A1-Stranges binden, Bereiche zwei und drei binden die inneren Segmente des A1-Stranges, ähnlich wie bei Bindung eines Aktivator-Oligonukleotides und eines ersten Primer-Verlängerungsproduktes.

In diesem Beispiel soll auch die Fähigkeit von Aktivator-Oligonukleotiden-Strukturen zu einer effektiven Strangverdrängung demonstriert werden. Aus diesem Grund wurden einige C1-Nukleinsäureketten identisch zu Aktivator-Oligonukleotiden (z.B. SEQ ID NO: 4, 5, 6 und 7) gestaltet. Andere C1-Nukleinsäureketten (SEQ ID NO: 8, 9 und 10) wurden nur in diesem Beispiel zu Demonstrationszweicken der Strangverdrängung eingesetzt, sie bestehen aus DNA und enthalten keine internen Modifikationen, welche Polymerase-Aktivität blockieren können. Da im vorliegenden Beispiel keine Polymerase in der Reaktion vorhanden war, spielte die Ab- / Anwesentheit solcher Modifikationen in diesem Beispiel eine untergeordnete Rolle. Die Strukturen von jeweiligen einzelsträngigen Nukleinsäureketten (C1-Oligonukleotide) sind nachfolgend zusammengefasst:

### C1-Oligonukleotide (Gruppe 1, Oligonukleotide mit Struktur von Aktivator-Oligonukleotiden):

Folgende vier C1-Oligonukleotide können ggf. als Aktivator-Oligonukleotide auftreten.
SEQ ID NO: 4 A6 8504-001 5' -[CGUCCUCUCCACACUCUGAGUUCCUG]CGAGCTTCGGTACAAG -3'
SEQ ID NO: 5 A6 8504-002 
5′- [AAAACAAACUAGCUAUCGUCCUCUCCACACUCUGAGUUCCUG]CGAGCTTC
GGTACAAG - 3′
SEQ ID NO: 6 A6 8507 5'- CTAGCTATCGTCCTCTCCACA[CUCUGAGUUCCUG]CGAGCTTCGGTACAAG -3'
SEQ ID NO: 7 A26-1000-103 (negativ-kontrolle) 
5′- [AUUCAAAUGUGUUCUCAACGUCCUCUACUCAUGUUCCUG]CGAGCTTCGGTAC
AAAA – 3′
 Nummerierung von 5' zu 3'

Die Sequenz mit SEQ ID NO 4, 5, 6 und 7 wurden als Aktivator-Oligonukleotide konstruiert. Sie umfassen 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides war durch Phosphat-Rest blockiert. Oligonukleotide wurden chemisch von einem kommerziellen Anbieter synthetisiert. Das unterstrichene Segment der Sequenz kann komplementär an den A1-Strang binden. Die Nukleinsäuren mit SEQ ID NO 4, 5 und 6 sind in ihren Bereichen ein, zwei und drei mit der Sequenz des Stranges A1 komplementär (komplementäre Sequenz des zweiten Bereichs gehen nahtlos in die komplementere Sequenzen des dritten Bereichs über). Solche durchgehende Anordnung von komplementären Nukleobasen soll eine Strangverdrängung unterstützen.

Die Nukleinsäure mit SEQ ID NO 7 ist komplementär im Bereich eins und zwei (von Position 33 bis 49), allerdings enthält sie eine zu A1-nicht komplementäre Sequenz (Positionen 1-18 und 27-32). Ein Teil dieser nicht-komplementären Sequenz ist platziert direkt am 5'-Segment des zweiten Bereichs, um das Fortschreiten der Strangverdrängung über diesen Bereich hinaus zu verhindern. Die Nukleinsäurekette diente als Negativ-Kontrolle.

### C1-Oligonukleotide (Gruppe 2, keine Aktivator-Oligonukleotide):

Folgende drei Oligonukleotide wurden ebenfalls in Strangverdrängungsreaktion eingesetzt.
SEQ ID NO: 8 A6 8510 5'-TTTTTCTAGCTATCGTCCTCTCCACACTCTGAGTTCCTGCGAGCTTCGGTACAAG 3'
SEQ ID NO: 9 A6 8512 5'TTTTTCTAGCTATCGTC*A*TCTCCACACTCTGAGTT*A*CTGCGAGCTTCGGTACAAG 3'
SEQ ID NO: 10 A6 8514-1 5' TTTTTCTAGCTATCGTCCTCTCCACA CTGAGTTCCTGCGAGCTTCGGTACAAG 3' Nummerierung von 5' zu 3'

Die Sequenz mit SEQ ID NO 8 - 10 wurde in ihrer Nukleobasenzusammensetzung ähnlich wie oben genannte Aktivator-Oligonukleotide konstruiert. Es wurden allerdings keine zu Polymerase-Blockade führenden Gruppe an entsprechender Stellen im inneren Segment der Oligonukleotide eingeführt: es fehlen Modifikationen des Zucker-Phosphat-Rückgrades (Polymerase-blockierender Modifikationen) im inneren Bereich der Sequenz, sowie eine 3'-blockierenden Gruppe. Diese C1-Oligonukleotide bestehen vollständig aus DNA-Nukleotiden. Oligonukleotide wurden chemisch von einem kommerziellen Anbieter synthetisiert. Das unterstrichene Segment der Sequenz kann komplementär an den A1-Strang binden. Dabei enthält die SEQ ID NO 8 einen durchgehend komplementeren Segment. Dieses Segment entspricht einem ersten, zweiten und dritten Bereich in einem Aktivator-Oligonukleotid. Die Nukleinsäuren mit SEQ ID NO 9 und 10 umfassend je zwei Sequenzabweichungen von durchgehend komplementären Sequenzen zu A1-Strang. SEQ ID NO 9 enthält an Positionen 18 und 36 je ein A-Nukleotid statt eines C-Nukleotids. Nukleotide an diesen Positionen stellen keine komplementäre Nukleobase zu entsprechenden Positionen in Strang A1 dar. Nukleinsäurekette mit SEQ ID NO 10 hat eine Abweichung in Komplementarität in Form einer Deletion: zwischen Positionen 26 und 27 fehlen 2 Nukleotide (CT). Diese Abweichung führt zu einer Unterbrechung einer komplementären Reihe bei Ausbildung von A1:C1 Doppelstrang.

### Erstes Primer-Oligonukleotid (Primer-1).

Das erste Primer-Oligonukleotid wurde in der Reaktion eingesetzt, um einen temperaturabhängigen Start der Strangverdrängung zu initiieren.
SEQ ID NO: 11 A6P-10-1001 5' - GTA[CCGAAGC]T*[CG]CAGGAAC - 3' Nummerierung von 5' zu 3'

Das erste Primer-Oligonukleotid umfasst 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides ist frei. Das Oligonukleotid hat in seiner Position 11 einen mit TAMRA-Farbstoff modifizierten dT-Nukleotid (als T* in der Sequenz angezeigt). Das Oligonukleotid wurde chemisch von einem kommerziellen Anbieter synthetisiert. Der Primer kann mit seiner Sequenz komplementär an C1-Oligonukleotide binden. Dank dieser Bindung zwischen dem Primer-1 und C1-Oligonukleotid sind bestimmte Sequenzsegmente des jeweiligen C1-Oligonukleotides während der Assemblierung der Reaktionsmischung (durchgeführt unter Raumtemperatur) doppelsträngig und können nicht mit dem einzelsträngigen Überhang des A1-Stranges interagieren.

### Vorbereitung eines komplementären Doppelstranges (A1 und B1)

Die beiden jeweils komplementäre Stränge von Nukleinsäuren (A1 und B1) wurden in Konzentration von je 1 µmol/l in der Puffer-Lösung 1 (ohne EvaGreen-Farbstoff) zusammengeführt, kurzfristig erhitzt und bei Raumtemperatur zur Abkühlung stehen gelassen, so dass ein Doppelstrang (A1:B1) über die komplementäre Bereich ausgebildet werden kann. Es resultierten zwei Varianten von Doppelstrang:
Variante 1 enthält Sequenzen mit SEQ ID NO: 1 und SEQ ID NO: 2
Variante 2 enthält Sequenzen mit SEQ ID NO: 1 und SEQ ID NO: 3

**Vorbereitung von** einzelsträngigen Nukleinsäurekette (C1), **(ohne Primer-1 Komplex)** Die jeweilige einzelsträngige Nukleinsäurekette (C1), wurde in Puffer-Lösung 1 (ohne EvaGreen-Farbstoff) in Konzentration von 10 µmol/l vorgelegt.

### Vorbereitung von C1:Primer-1 Komplexen aus einzelsträngigen Nukleinsäurekette (C1) und 3Primer-1

Um den ersten und zweiten Bereich der einzelsträngigen Nukleinsäurekette (C1) reversibel zu blockieren wurde Primer-1 in equimolarer Konzentration zur jeweiligen C1-Oligonukleotid gegeben, so dass sich ein partiell doppelstranger C1:Primer-1 Komplex ausbilden konnte. Der Primer-1 bindet spezifisch an den ersten und zweiten Bereich des C1-Oligonukleotides und blockiert somit Sequenzsegment, welches mit dem Überhang des A1-Stranges während der Vorbereitungsphase der Reaktionsmischung (unter Raumtemperatur) interagieren kann. Dieser Komplex ist bei Raumtemperatur stabil und kann erst unter Reaktionsbedinungen in seine Einzelteile dissoziieren, so dass der erste und der zweite Bereiche des C1-Oligonukleotides einzelsträngig werden und somit mit entsprechenden Strukturen des Doppelstranges (A1:B1) interagieren können. Die Komplexe aus C1-Oligonukleotid und Primer 1 wurden ebenfalls in Puffer-Lösung 1 (ohne EvaGreen-Farbstoff) in Konzentration von 10 µmol/l vorgelegt.

### Vorbereitung der Reaktionsmischung, Reaktions-Start und Reaktionsbedinungen

Die Reaktionsmischung wurde durch Zusammenführung von gleichen Volumina (je 5 µl) einer Lösung mit einem Doppelstrang (A1:B1) und einer Lösung mit einem zu testenden C1-Oligonukleotid bzw. C1-Primer-1-Komplex bei Raumtemperatur vorbereitet. In der resultierenden Reaktionsmischung liegen die einzelnen Stränge in folgenden Konzentrationen vor:
Doppelstrang A1:B1 in Konzentration von 0,5 µmol/l
Einzelstrang (C1) bzw. Komplex C1-Primer-1 in Konzentration von 5 µmol/l
Einzelstrang B1 (als FAM-Signal-Kontrolle) in Konzentration von 0,5 µmol/l

### Beobachtung des Verlaufs der Strangverdrängung unter isothermalen Bedinungen von 65°C

Die so vorbereitete Reaktionsmischung wurde in einem entsprechenden Reaktionsgefäß (Mikrotiter-Platte) in ein Thermonstat mit mit einer Fluoreszenz-Detektions-Funktion überführt. Als Thermostat wurde ein Real-Time-PCR Gerät ("StepOne Plus", StepOne Software v2.1 ABI, Themorfischer) verwendet. Die Reaktionsmischung wurde auf Temperatur von zunächst 50°C für ca. 50 sec vorgewärmt (Zyklus 1 bis 5) und anschließend auf 65°C erwärmt. Diese Temperatur wurde über ca. 10 min aufrechterhalten (Zyklus 6 bis 30 wurde aufgezeichnet), so dass eine Strangverdrängungsreaktion unter isothermalen Bedinungen verlaufen und beobachtet werden konnte. Das Fluoreszenzsignal von FAM wurde in Abhängigkeit von der Zeit (Real-Time Funktion des Geräts) aufgezeichnet. Dank Verwendung von Real-Time-PCR Gerät konnte ein vom Hersteller mitgeliefertes Software-Programm mit entsprechenden FAM-Filter-Einstellungen verwendet werden. Der minimale vom Hersteller vorgesehene Zeitinterval zwischen zwei Messungen betrug 10 sec (minimaler einstellbarer Zeitinterval), was insgesamt zu einem gerätebedingten Mess-Zyklus von ca. 15 bis 18 sec führte. Der Verlauf des FAM-Signals wurde mit dieser maximalen Aufzeichnungsgeschwindigkeit aufgenommen.

### Beobachtung der Trennung von A1 und B1 während eines thermalen Gradienen (50°C - 95°C) bei Anwesenheit / Abwesenheit von C1-Oligonukleotiden.

In einem weiteren Experiment wurde die Reaktionsmischung kontinuierlich von 50°C auf 95°C erwärmt (ein Temperatur-Gradient). Während dieser Aufwärmfphase wurde ebenfalls FAM-Signal detektiert, wobei pro 0,5° C Temperatur-Zuwachs eine FAM-Signalmessung erfolgte. Es resultierte ein Thermo-Profil der Reaktionsmischung, bei welchem Signal-Intensität des FAM-Reportes in Abhängigkeit von Temperatur aufgezeichnet wurde. Dieses Experiment wurde ebenfalls mit Hilfe von Real-Time PCR Gerätes (StepOne Plus) durchgeführt, wobei das Programm zur Schmelzkurvenbestimmung verwendet wurde (vorinstalliert vom Hersteller, Software Version StepOne v2.1).

Beide Experimente wurden für jeweilige Reaktionsmischung ausgewertet und Ergebnisse werden nachfolgend vorgestellt. Die Zusammensetzung einzeler Reaktionen wird an entsprechenden Stellen angegeben.

### Ergebnis:

### 1. Testung des Detektionssystems:

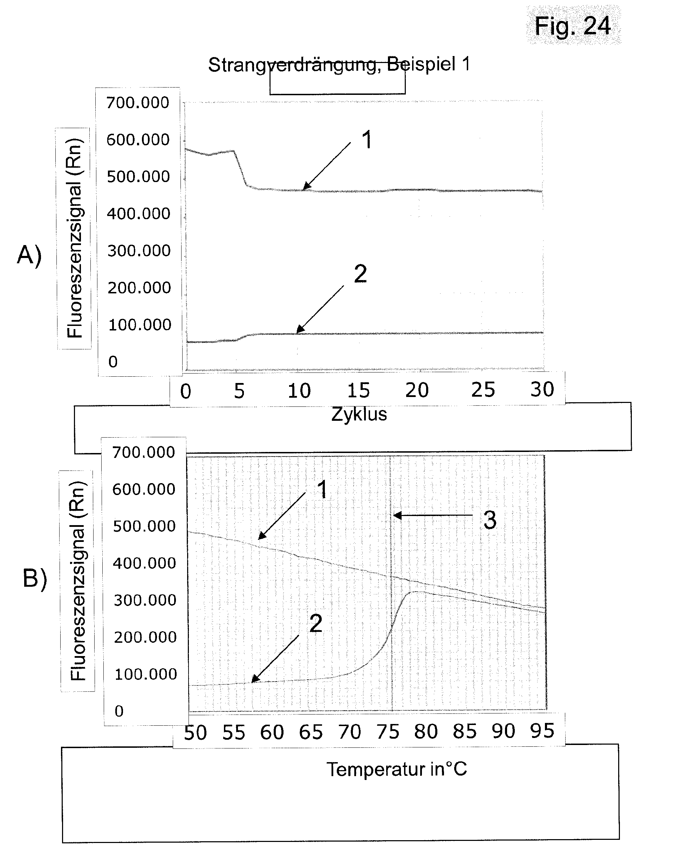

Die Funktionsfähigkeit des FRET-Paares aus FAM und TAMRA wurde in zwei Kontrollansätzen überprüft. Der erste Kontrollansatz enthielt nur einen B1-Strang markiert mit einem FAM-Farbstoff (Fig.24 Pfeil 1), der zweite Kontroll-Anzsatz enthielt sowohl einen mit TAMRA markierten A1-Strang als auch einen mit FAM markierten B1-Strang (A1:B1-Doppelstrang) (Fig. 24 Pfeil 2). Sowohl zeitlicher Verlauf des Signals unter isothermalen Bedingungen (Fig. 24 A) als auch das Verhalten des Signals im Temperatur-Gradient wurden aufgezeichnet (Fig. 24 B). Man erkennt jeweils eine stabile Signal-Linie bei isothermalen Bedinungen (65°C) für beide Kontroll-Ansätze (Fig. 24 A). Bei Aufzeichnung des Temperatur-Gradienten (Fig. 24 B) des Doppelstranges (Pfeil 2) sieht man einen Anstieg der Fluoreszenz des FAM-Reporters ab ca. 70°C. Das Signal erreicht bei ca. 80°C sein Maximum und verhält sich anschließend wie ein Einzelstrang (vergleiche Pfeil 1). Die errechnete Schmelztemperatur dieses Stranges (Variante 2 des Doppelstranges A1:B1 mit 9 Nukleotiden im 5'-Überhang A1-Stranges) liegt bei ca. 75°C (Pfeil 3). Bei dieser Temperaur ist definitionsgemäß die Hälfte des Doppelstranges geschmolzen, so dass Stränge A1 und B1 je zu Hälfte einzelsträngig vorliegen. Die Kontroll-Ansätze für Variante 1 des Doppelstranges A1:B1 mit 6 Nukleotiden im 5'-Überhang des A1-Stranges verhalten sich ähnlich, die Tm dieser Variante 1 liegt bei ca. 77°C.

Pfeile 1 und 2 zeigen auch in folgenden Figuren 24 bis Fig. 29 die jeweiligen Kontroll-Ansätze mit einem einzelsträngigen FAM-markierten B1-Strang (Pfeil 1) und einem markierten Doppelstrang A1:B1 (Pfeil 2).

### 2. Strangverdrängung durch ein einzelsträngiges komplementäres Oligonukleotid.

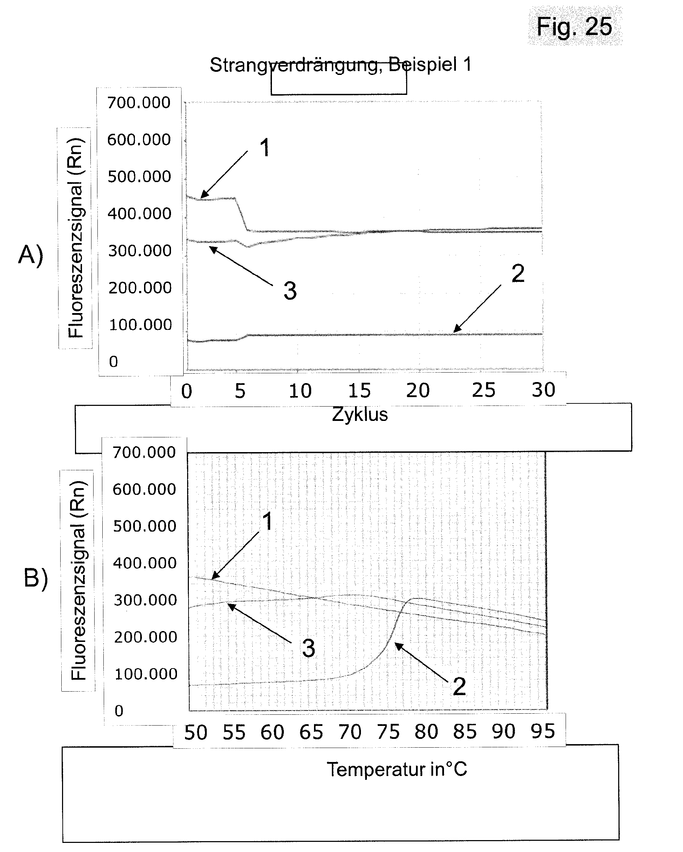

Bei durchgehend komlementären Zusammensetzung der Sequenzen der C1-Oligonukleotide und A1-Stränge (z.B. Reaktion zwischen A1:B1 Variante 2 und C1-Oligonukleotid SEQ ID NO 5 A6 8504) verlief die Strangverdrängung des B1-Strangesunter teilweise dermaßen schnell, dass ein signifikanter Anteil der B1-Stränge bereits während der Vorbereitung der Raktionsmischung bzw. noch vor Start der Aufzeichnung einzelsträngig vorlag. Das sieht man anhand der gestiegenen Signalintensität in Fig. 25 (Pfeil 3). Bereits die erste Messung (Zyklus 1) weist einen deutlich höheres Signalniveau als die Kontroll-Reaktion mit einem A1:B1-Doppelstrang ohne C1-Oligonukleotid. Somit liegt B1-Strang größtenteils bereits vor der ersten Messung in einzelsträngiger Form. Ein ähnliches Verhalten zeigen auch Reaktionen mit A1:B1-Doppelstrang Variante 1.

Ein Austausch des C1-Oligonukleotids durch ein anderes durchgehend komplementäres C1-Oligonukleotid (z.B. SEQ ID NO 4, 6 oder 8) zeigen grundsätzlich ähnliches Verhalten. Die hohe Intensität des Fluoreszenz-Signals von FAM-Reporter bereits zu Beginn der Messung deutet auf Vorliegen von B1-Stranges in einzelsträngiger Form noch vor der ersten Messung. Dieses Verhalten von entsprechenden C1-Oligonukleotide zeigt, dass die ggf. vorhandenen Unterschiede in Zusammensetzung des Zucker-Phosphat-Rückgrades (DNA vs. 2'-O-Methyl Modifikationen) im vorliegenden Beispiel nur unwesentliche Unterschiede im Ergebnis der Strangverdrängung durch einzelne C1-Oligonukleotide bewirkten. Sowohl Oligonukleotide mit längeren 2'-O-Me Segmenten als auch komplett aus DNA bestehende Oligonukleotide führten zum gleichen Ergebnis. Die durchgehende Reihenfolge von komplementären Basen insbesondere in Bereichen zwei und drei von C1-Oligonukleotiden sorgte für eine Strangverdrängung mit hohen Ausbeuten.

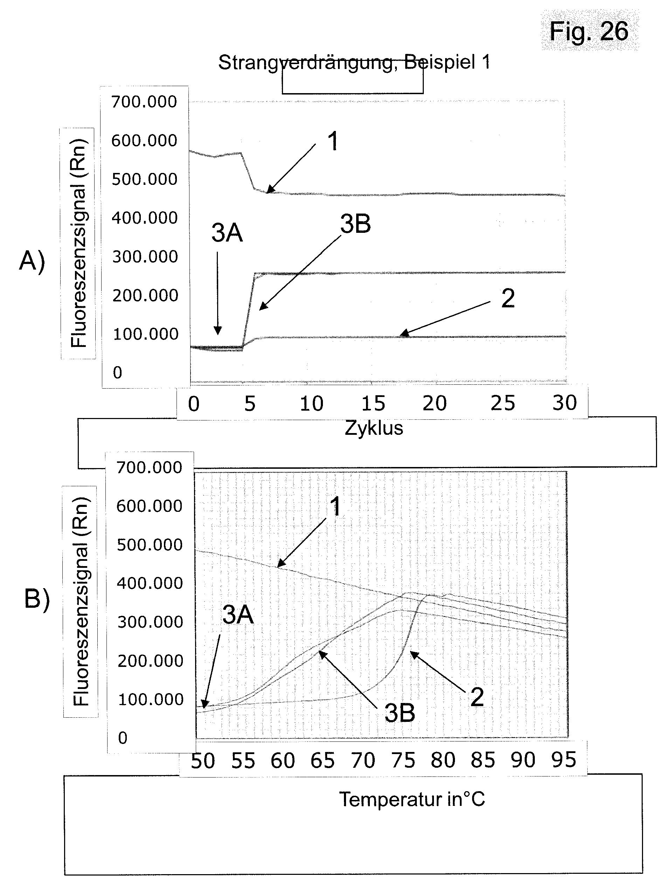

Verwendung von C1-Strang mit SEQ ID NO: 4 (8504 kurz) mit einer durchgehenen komplementären Zusammensetzung einzelner Bereiche allerdings mit einem kürzeren komplementären dritten Bereich führte zum Ergebnis dargestellt in Fig. 26. Ansätze mit C1 und A1:B1-Doppelstrang Varianten 1, sowie C1 und A1:B1-Doppelstrang Variante 2 verhalten sich ähnlich (Pfeil 3): erst nach Erhöhung der Temperatur auf 65°C kommt es zu einer Strangverdrängung.

Eine Verkürzung der C1-Sequenz am seinem 5'-Ende (entspricht dem dritten Bereich eines Aktivator-Oligonukleotides) (SEQ ID NO: 4) führte dazu, dass ein längerer Sequenzabschnitt im 3'-Segement des A1-Stranges nicht von C1-Oligonukleotid komplementär gebunden wird, so dass dieses 3'-ständige Segment des A1-Stranges in Kontakt mit 5'-Segment des B1-Strang bleiben kann (Positionen 1 bis 18 des 5'-Segment des B1 Stranges). Dieses doppelsträngige Fragment ist bei 50°C hinreichend stabil, so dass A1:B1-Doppelstrang an seinem markierten Ende auch bei 50°C hinreichend Kontakt behält, und das Signal von FAM bei dieser Temperatur hinreichend gequenched (Pfeil 3, Zyklus 1 bis 5, Fig. 26) ist. Mit Erhöhung der Temperatur auf 65°C (Pfeil 3B Fig. 26) führte die Anwesenheit von C1-Oligonukleotid dazu, dass ca. die Hälfte der Population von A1:B1-Doppelstrangen in Einzelstränge zerfiel. Es bildet sich ein Gleichgewicht zwischen einer A1:B1-Doppelstrang-Form und Einzelstrangform von B1-Strang. Die Fluoreszenzintensität von FAM-Reporter im Ansatz entspricht ca. 50% der Intensität eines freien Stranges. Dieses Verhalten wird darauf zurückgeführt, dass bei angenommenen voll ausgeprägten Strangverdrängung durch C1-Oligonukleotid (d.h. vollständige Bindung zwischen komplementären Bereichen von A1-Strang und C1-Oligonukleotid) der restlich verbleibende Kontakt zwischen dem 3'-Segment von A1-Strang und 5'-Segment des B1-Stranges bei dieser Temperatur nicht mehr hinreichend stabil, so dass es zu einer spontanen, temperatur-induzierten Trennung von A1 und B1 Strängen in diesem Segment kommt. Unter Reaktionsbedinungen kann ein einzelsträngiger B1-Strang wiederum an das 3'-Segment des A1-Strang erneut binden und zur Strangverdrängung von C1-Oligonukleotid führen. Das System befindet sich somit im Gleichgewicht zwischen A1:B1-Doppelstrang, C1:A1-Doppelstrang und ihren Zwischenformen.

### 3. Strangverdrängung durch ein einzelsträngiges Oligonukleotid mit Abweichungen in der Sequenzzusammensetzung.

Die Testung des Einflusses von Sequenzabweichungen wurde mit C1-Oligonukleotiden ausgeführt, welche in ihrer Zusammensetzung der Nukleobasen zwei oder mehr nicht-komplementären Basen umfassten, welche im zweiten oder dritten Bereich des C1-Oligonukleotides lagen. Diese abweichende Basenzusammensetzung von C1 führte dazu, dass keine durchgehend komplementäre Reihenfolge von Basenpaarungen in einem zu erwartenden Doppelstrang aus A1 und C1 entstehen würde.

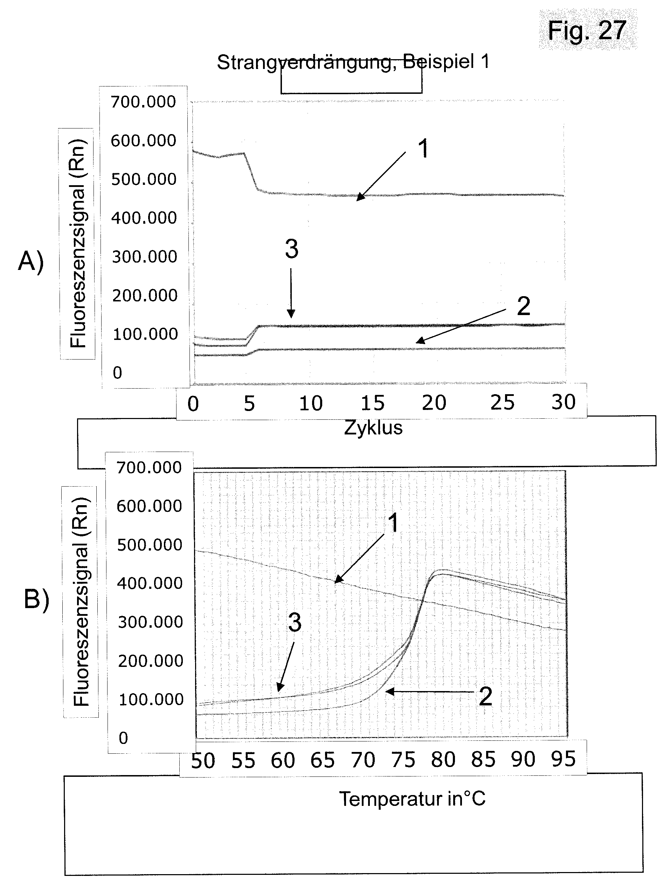

In Fig. 27 ist Ergebnis der Interaktion zwischen C1-Oligonukleotid SEQ ID NO 5 und A1:B1-Doppelstrang Variante 1 gezeigt. Die Abweichungen von der durchgehend komplementären Sequenz sind in Positionen 18 und 36 (SEQ ID NO 9) und Deletion zwischen Positionen 26 und 27 (SEQ ID NO 10) zu sehen. Beide C1-Oligonukleotide führen zu einer geringfügig ausgeprägten Änderung in der Fluoreszenz des FAM-Reporters (Fig. 27, Pfeil 3). Eine solch geringe Signalintensität des FAM-Reportes ist assoziiert mit einer vorwiegend gebundenen Form des B1-Stranges an A1-Strang. Mit anderen Worten, ein C1-Oligonukleotid mit Abweichungen in der Nukleobasenzusammensetzung im zweiten und dritten Bereich führte zu Verringerung von Ausbeuten bei der Strangverdrängung: der A1:B1-Doppelstrang wurde kaum geöffnet und nur geringer Anteil von B1-Strängen wurde in einzelsträngige Form überführt. Das Gleichgewicht der Reaktion lag überwiegend auf der Seite des A1:B1-Doppelstranges.

Eine weitere Erhöhung der Anzahl von Abweichungen in Nukleobasenzusammensetzung führte zu einer vollständigen Aufhebung der Strangverdrängung. Das C1-Oligonukleotid mit SEQ ID NO: 7 umfasst durchgehend komplementäre Nukleobasensequenz im ersten und zweiten Bereich, weist allerdings signifikante Abweichungen im dritten Bereich seiner Sequenz auf. Diese Sequenzabweichungen liegen teilweise im 3'-Segement des dritten Bereichs des C1-Oligonukleotids (Positionen 26 - 32) und sind somit unmittelbar am zweiten Bereich lokalisiert. Die Zugabe eines solchen C1-Oligonukleotid zum vorgelegten A1:B1-Doppelstrang führte zu keiner messbaren Änderung in Fluoreszenz des FAM-Reporters unter 65°C; das Signal ist auf dem Niveau des intakten A1:B1-Doppelstranges geblieben. Das bedeutet, dass obwohl C1-Oligonukleotid in der Lage war, mit seinem ersten und zweiten Bereich an das A1-Strang komplementär zu binden (diese Bereiche umfassen komplementäre Sequenzen zwischen C1 und A1 Strängen), führte Abweichung der Sequenzzusammensetzung im Dritten Bereich zu vollständigen Aufhebung einer erfolgreichen Strangverdrängung unter gegebenen Reaktionsbedinungen, der A1:B1-Doppelstrang blieb weitgehend intakt. Dies wird im Zusammenhang mit der Stabilität des restlichen A1:B1-Doppelstrang gesehen. Im Fall einer komplementären Bindung des ersten und zweiten Bereichs des C1-Oligonukleotides an A1-Strang bleiben A1-Strang und B1-Strang über ein signifikant langes Segment aneinander komplementär gebunden. Unter gegebenen Bedinungen ist diese Stabilität dieses restlichen, nicht verdrängten doppelsträngigen Segmentes (A1:B1) hinreichend hoch, um beide Stränge zusammenzuhalten und eine messbare Trennung des B1 von A1 zu verhindern. Das Gleichgewicht der Reaktion liegt in diesem Fall vollständig auf der Seite der A1:B1-Doppelstranges, es findet keine messbare Trennung von diesen Strängen statt.

### 4. Kinetik der Strangverdrängung

Zur Messung der Kinetik der Strangverdrängung wurden C1:Primer-1 Komplexe und vorbereitete A1:B1-Doppelstränge verwendet. Der Vorteil bei der Verwendung von C1-Primer-1 Komplexen bestand darin, dass Primer 1 an komplementäre Bereiche des C1-Oligonukleotides bereits im Vorfeld binden konnte, was zu einem bei Raumtemperatur stabilen Doppelstrang führte. Dank dieser Reaktionsanordnung konnte eine Strangverdrängung erst gestartet werden, wenn Primer-1 von C1-Strängen unter Reaktionsbedinungen getrennt war, z.B. im Rahmen der Erhöhung der Reaktionstemperatur auf 65°C (entspricht in etwa der Tm von Primer-1 (Tm plus / minus 5°C). Durch Freisetzung der komplementären Bereiche im C1-Oligonukleotid konnte dieses nun unter Reaktionsbedinungen mit dem A1:B1-Doppelstrang interagieren und ggf. zu Strangverdrängung führen.

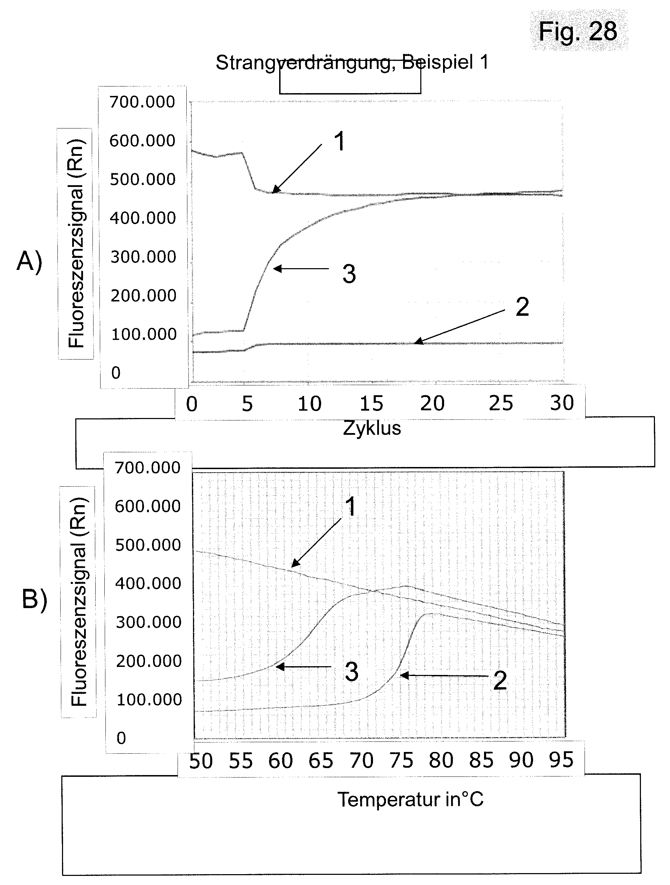

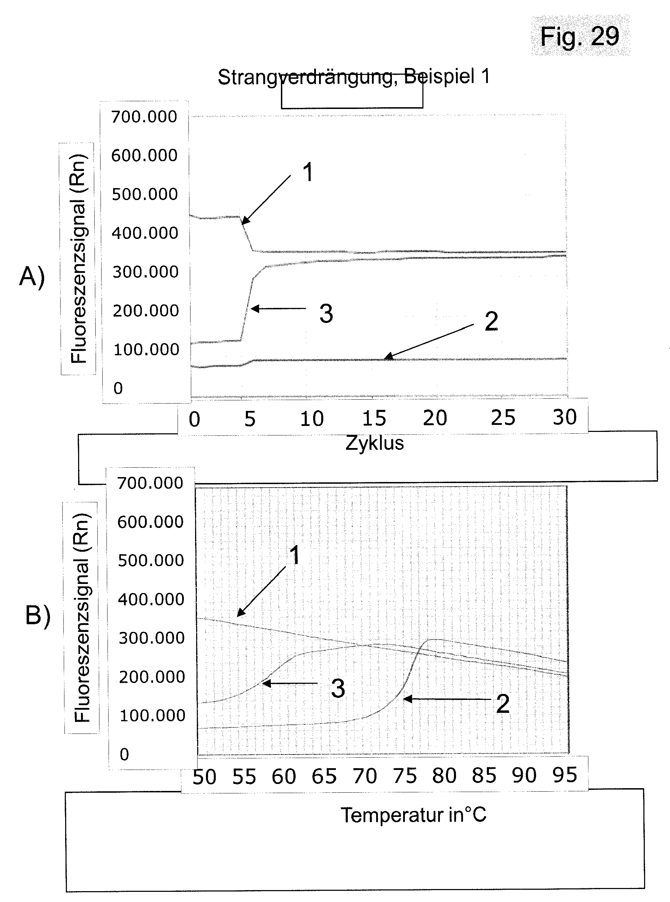

Zu Demonstration der Kinetik der sequenzabhängigen Strangverdrängung wurde Variante 1 des A1:B1-Stranges mit C1-Primer-1 Komplex (bestehend aus SEQ ID NO 5 und 11) in Kontakt gebracht und zunächst auf 50°C (Zyklus 1 - 5) und anschließend auf 65°C (Zyklus 6 und weiter) erhitzt, wie oben dargestellt. Der Verlauf des Signals vom FAM-Reporter ist in Fig. 28 zu sehen. Man beobachtet eine nahe konstante Linie zwischen Zyklus 1 und 5 (50°C) und danach eine sprunghafte Zunahme der Fluoreszenz von FAM-Reporter ab dem 6. Mess-Zyklus (65°C). In darauf folgenden 10 Zyklen (6 bis 16) erreicht die Signalintensität des FAM-Reporters ca. 90% der maximal möglichen Fluoreszenz. Die Zunahme der Signalintensität spricht für Trennung von Strängen A1 und B1. Die daraus errechnete Halbwertzeit der Trennung des A1:B1-Doppelstranges lag bei ca. 40 - 50 sec. Im Vergleich dazu, erfolgte der Anstieg der Fluoreszenz von FAM-Reporter bei Verwendung der Variante 2 des A1:B1-Stranges mit gleichem C1:Primer-1 Komplex (bestehend aus SEQ ID NO 5 und 11) wesentlich schneller (Fig. 29, Pfeil 3). Die errechnete Halbwertzeit der Trennung des A1:B1-Doppelstranges lag in diesem Ansatz unter 10 sec bei gegebenen Reaktionsbedinungen.

Der Unterschied zwischen Variante 1 und 2 des A1:B1-Doppelstrangs besteht in erster Linien in der Länge des 5'-Überhangs des A1-Stranges, mit welchem A1-Strang in den initialen Kontakt mit C1-Oligonukleotid kommt. Der B1-Strang der Variante 1 ist länger als in Variante 2, der dabei resultierende Unterschied ergibt 6-Nukleotide langen 5'-Überhang in der Variante 1 und 9-Nukleotide langen 5'-Überhang in der Variante 2. Durch komplementäre Zusammensetzung entsprechender Segmente im C1-Oligonukleotid ist davor auszugehen, dass die Bindung an 9-Nukleotid-Überhang etwas stabiler ist als die Bindung an einen 6-Nukleotid-Überhang.

Vergleich der Kinetik der Fluoreszenzzunahme zwischen Variante 1 und Variante 2 des A1:B1-Doppelstranges führte zu Schlußfolgerung, dass die Länge des 5'-Überhangs am A1-Strang und somit auch Stabilität eines während der Reaktion geformten Zwischenkomplexes, bestehend aus A1-Überhang und C1-Oligonukleotid, eine Rolle spielen kann: je stabiler dieser Komplex aus A1-Überhang und C1-Oligonukleotid ist, umso effektiver bzw. schneller die Strangverdrängung bei sonst gleichen Parametern erfolgen kann. Dieser Zwischenkomplex aus A1-Überhang und dem ersten Bereich des C1-Oligonukleotides bringt das C1-Oligonukleotid in hinreichende räumliche Nähe zum entsprechenden Duplex-Ende des A1:B1-Oligonukleotids, so dass eine Strangverdrängung erfolgen kann. Im vorliegenden Ansatz resultiert eine Komplex-Bildung zwischen C1-Oligonukleotid und 5'-Überhang des A1-Stranges in annehrend identischer räumlicher Nähe des komplexierten C1-Oligonukleotides zum Duplex-Ende des A1:B1-Doppelstranges sowohl bei Variante 1 als auch bei Variante 2. Die Unterschiede in der Kinetik der Strangverdrängung zeigen jedoch, dass höhere Stabilität der Bindung des Zwischenkomplexes bei gleicher räumlicher Nähe zu Effizienzsteigerung des Vorgangs der Strangverdrängung führen kann. Das Gleichgewicht kann schneller verschoben werden.

In folgenden Beispielen werden einzelne Ausführungsformen einer exponentiellen Amplifikation veranschaulicht. Diese Beispiele dienen zur Veranschaulichtung der Funktion einzelner Elemente der exponentiellen Amplifikation und sollen nicht als Einschränkung verstanden werden. Während einer exponentiellen Amplifikation läuft eine polymerasenabhängige Nukleinsäuresynthese zeitlich gleichzeitig mit der nukleinsäureabhängigen Strangverdrängung ab. Die Reaktionsansätze umfassen Polymerasen und dNTP's als Substrate. Statt eines C1-Oligonukleotides werden Aktivator-Oligonukleotide verwendet, welche in ihren Strängen Nukleotid-Modifikationen umfassen, die zu lokalen Blockade der Polymerase-Tätigkeit führen, so dass Aktivator-Oligonukleotide nicht als Matrize während der Amplifikation auftreten. Es werden je zwei Primer in einer Amplifikation eingesetzt: das erste Primer-Oligonukleotid und das zweite Primer-Oligonukleotid. Das erste Primer-Oligonukleotid umfasst einen Polynukleotid-Schwanz, welcher Aktivator-Oligonukleotide spezifisch binden kann und damit räumliche Nähe zum Duplex-Ende einer zu amplifizierenden Nukleinsäure herbeiführen, so dass eine Strangverdrändung des zweiten Primer-Verlängerungsproduktes stattfinden kann. Das zweite Primer-Oligonukleotid kann an das 3'-Segment des ersten Primer-Verlängerungsproduktes binden und initiiert somit die Synthese des zweiten Primer-Verlängerungsproduktes.

### Beispiel 2:

Dieses Beispiel demonstriert eine exponentiell ablaufende Amplifikation eines artifiziellen DNA-Fragments unter isothermalen Bedinungen. Dabei dienen die neu synthetisierten Stränge als Matrizen für die spätere Synthese der komplementären Partner-Stränge. Die Synthese findet in Abhängigkeit von Präsenz einer spezifischen Matrize statt. Die Vermehrung von zu amplifizierenden Nukleinsäuren erfolgte um ca. Faktor 10⁹ bezogen auf die Ausgangsmenge der eingesetzten Matrize.

### Ausgangsmaterial:

### Matrize:

Als Teil einer zu amplifizierenden Nukleinsäure wurde eine einzelsträngie DNA-Matrize genommen. Die Sequenz der Matrize-001 (SEQ ID NO 12) wurde zufällig ausgewählt und wurde im Hinblick auf Vermeidung von Strecken (mehr als 6 Basen), welche zu einer Doppelstrangbildung innerhalb des gleichen Strangs überprüft, um die Formierung von sog. Hairpins zu vermeiden. Diese DNA Sequenz wurde künstlich von einem kommerziellen Anbieter synthetisiert. Die DNA Sequenz trägt keine Nukleotid-Modifikationen. Das 3'-Ende des Matrizenstrangs ist offen und ist nicht blockiert. Diese Matrize soll als Beispiel für eine einzelsträngige zu amplifizierende Nukleinsäure dienen.
Matrize 001: (SEQ ID NO 12) T35-4401-601 5'- ATACTACAATGTCACTTACGTCCTCTCCACACTCTGAGTTCCTGCGAG - 3' (Reihenfolge der Nummerierung: 1 - 48)

Die gemessene Schmelztemperatur eines DNA-Doppelstranges, welcher Matrize-001 als ein Strang einschließt, beträgt 77°C (Tm gemessen in Lösung 1).

### Primer-1 (erstes Primer-Oligonukleotid)

Primer 001-01:
SEQ ID NO: 13 A6P-10-1001 5' - GTA[CCGAAGC]T*[CG]CAGGAAC - 3' Nummerierung von 5' zu 3'

Das erste Primer-Oligonukleotid umfasst 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides ist frei. Das Oligonukleotid hat in seiner Position 11 einen mit TAMRA-Farbstoff modifizierten dT-Nukleotid (als T* in der Sequenz angezeigt).

### Primer-2 (zweites Primer-Oligonukleotid)

Primer 001-02: (SEQ ID NO 14)
PA6-35-4401-608 5'- AAAAAAAC ATACTACAATGTCACTTAC-3' (Reihenfolge der Nummerierung: 1 - 27)

Dieser Primer ist für die Initiierung der Synthese des 2. Stranges notwendig. Die Nukleotide in Positionen 9 - 27 sind mit der Matrize-001 identisch.

### Aktivator-Oligonukleotid-001-01

Aktivator-Olgonukleotid-001-01: (SEQ ID NO 15)
A6 8505 5'- [CUAGCUAUCGUCCUCUCCACACUCUGA GUUCC]TGCGAGCTTCGGTACAAGX -3' (Reihenfolge der Nummerierung: 1 - 50)

Das Aktivator-Oligonukleotid umfaßt 2'-O-Me Nukleotid-Modifikationen (angegeben in [ ]-Klammern) und DNA Nukleotide (im 3'-Segment). Die 3'-OH-Gruppe des 3'-endständigen Nukleotides war durch Phosphat-Rest blockiert (hier mit X gekennzeichnet). Oligonukleotid wurde chemisch von einem kommerziellen Anbieter synthetisiert. Das unterstrichene Segmente der Sequenz kann komplementär an das erste Primer-Verlängerungsprodukt binden. Diese durchgehende Anordnung von komplementären Nukleobasen soll eine Strangverdrängung unterstützen. Die Nukleotide und Nukleotid-Modifikationen sind untereinander mit Phosphodiesterbindung verknüpft.

Das Aktivator-Oligonukleotid schließt drei Bereiche ein:

| | |
|---|---|
| Erster Bereich | = Position 35 - 50 |
| Zweiter Bereich | = Position 28 - 34 |
| Dritter Bereich | = Position 1 - 27 |

Diese Komponenten wurden wie folg in der Reaktionsmischung eingesetzt:
- Matrize-001 2 fmol/l
- Aktivator-Oligonukleotid-001-01: 10 µmol/l
- Primer 001-01: 10 µmol/l
- Primer 001-02: 10 µmol/l

Weitere Komponenten der Reaktion wurden als Amplifikationsreaktion Lösung 2 eingesetzt:
- Kalium Glutamat, 50 mmol/l, pH 8,0
- Magnesium Acetat, 10 mmol/l
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1 % (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

### Start der Reaktion und Reaktionsbedingungen

Die Vorbereitung der Reaktionsmischung erfolgte bei Raumtemperatur. Die Matrize wurde im Reaktionsansatz als letzte Komponente zugegeben. Ein Kontroll-Ansatz enthielt keine Matrize.

Die so vorbereiteten Reaktionsmischung wurden in je einem entsprechenden Reaktionsgefäß (Mikrotiter-Platte) in ein Thermonstat mit mit einer Fluoreszenz-Detektions-Funktion überführt. Als Thermostat wurde ein Real-Time-PCR Gerät ("StepOne Plus", StepOne Software v2.1 ABI, Themorfischer) verwendet. Der Start der Reaktion erfolgte durch Erhitzung der Reaktionsmischung auf 65°C.

Die Reaktionstemperatur wurde während der gesamten Amplifikation (40 min) bei 65°C konstant gehalten (isothermale Amplifikation). Anschließend wurde Reaktion auf 95°C für 10 min erhitzt. Während der Reaktion wurde die Intensität des Fluoreszenz-Signal von EvaGreen Farbstoff aufgezeichnet. Die Mess-Intervale zwischen benachbarten Messungen betrugen je eine Minute.

### Auswertung:

### Die zu erwartenden Primer-Verlängerungsprodukte:

Das zu erwartende erste Primer-Verlängerungsprodukt-001-01 nach der ersten Primer-Verlängerung an der Matrize-001: (SEQ ID NO 16) 
5´- GTACCGAAGCTCGCAGGAACTCAGAGTGTGGAGAGGACGTAAGT
GACATTGTAGTAT -3´
 (Reihenfolge der Nummerierung: 1 - 57)

Unterstrichene Bereiche sind durch 2'-O-Me Modifikationen des ersten Primer-Oligonukleotid dargestellt. Nicht unterstrichene Bereiche sind 2'-deoxy-Nukleotide (DNA). Dieses erste Primer-Verlängerungsprodukt kann das zweite Primer-Oligonukleotid im 3'-Bereich komplementär binden und damit selbst als Matrize für die Synthese des zweiten Primer-Produktes dienen.

Die Synthese des zweiten Primer-Verlängerungsproduktes erfolgt bis hin zum modifizierten Bereich des ersten Primer-Oligonukleotides. Polymerase kann dabei entweder unmittelbar vor dem modifizierten Bereich gestoppt werden oder erster nach Kopierung von einigen Nukleotid-Modifikationen. Das zu erwartende zweite Primer-Verlängerungsprodukt 001-02 (SEQ ID NO 17) unter Verwendung des ersten Primer-Verlängerungsproduktes als Matrize: 
5′- AAAAAAACATACTACAATGTCACTTACGTCCTCTCCACACTC
TGAGTTCCTGCGAG - 3′
 (Reihenfolge der Nummerierung: 1 - 56)

Die vier unterstrichenen 3'-ständigen Nukleotide werden hier angezeigt als angenommene maximale Verlängerung des zweiten Primer-Verlängerungsproduktes an der Sequenz des ersten Primer-Verlängerungsproduktes. Diese Nukleotide bilden komplementäre Positionen zu einigen 2'-O-Me modifizierten Nukleotiden des ersten Primer-Oligonukleotids. Polymerase kann auch kürzere Stränge unter Reaktionsbedinungen synthetisieren, so dass es eine Mischung aus Primer-Verlängerungsprodukten resultieren kann, da Stopp der Polymerase-Synthese bereits vor dem modifizierten Bereich des ersten Primer-Oligonukleotides oder erst nach Kopierung von einigen Modifikationen erfolgen kann. Solche Primer-Verlängerungsprodukte sind entsprechend kürzer (sie schließen beispielsweise Positionen 1 bis 52 oder 1 bis 53 oder 1 bis 54 oder 1 bis 55 ein). Die genaue Position der Terminierung der Synthese des zweiten Primer-Verlängerugnspdoruktes und somit seine die exakte Länge des zweiten Primer-Verlängerungsproduktes an seinem 3'-Ende ist an dieser Stelle von untergeorneter Bedeutung, solange alle amplifikationsfähigen zweiten Primer-Verlängerungsprodukte in der Lage sind, das erste Primer-Oligonukleotid zu binden und selbst als Matrize für die Synthese des ersten Primer-Verlängerungsproduktes auftreten. Die polymeraseabhängige Kopierung des zweiten Primer-Verlängerungsproduktes führt zu Generierung eines weiteren ersten Primer-Verlängerungsproduktes, welches auch komplementäre Sequenz zum 5'-Segment des zweiten Primer-Oligonukleotids einschließt.

Als Ergebnis einer exponentiellen Amplifikation entstehen zwei Primer-Verlängerungsprodukte, welche einen komplementären Doppelstrang bilden können.

Die dabei zu erwartende Struktur des Doppelstranges, welcher aus beiden maximal verlängerten Primer-Verlängerungsprodukten besteht, ist nachfolgend abgebildet:

5´- GTACCGAAGCTCGCAGGAACTCAGAGTGTGGAGAGGACGTAAGTGACA
TTGTAGTATGTTTTTTT -3´ (SEQ ID NO 18)

3´-                GAGCGTCCTTGAGTCTCACACCTCTCCTGCATTCACTGT
AACATCATACAAAAAAA -5´   (SEQ ID NO 19)

Im Laufe der exponentiellen Amplifikation kommt es zu Akkumulierung von diesen Primer-Verlängerungsprodukten, welche das Hauptprodukt der exponentiellen Amplifikation darstellen. Die so amplifizierten Stränge stellen die zu amplifizierende Nukleinsäure dar. Sie schließen die Sequenz der in die Reaktion eingesetzten Matrize ein. Unterstrichene Bereiche sind durch 2'-O-Me Modifikationen dargestellt. Nicht unterstrichene Bereiche sind 2'-deoxy-Nukleotide (DNA).

Der während der Reaktion entstehende Doppelstrang kann EvaGreen Farbstoff binden, so dass die Signal-Intensität des Farbstoffs unter fortschreitender Reaktion ansteigen kann.

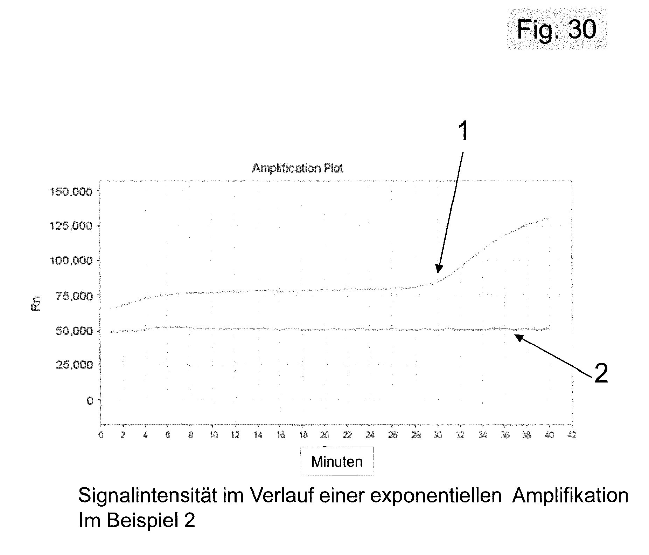

Das Ergebnis des Verlaufs der Signalintensität ist in Fig. 30 zu sehen. Pfeil 1 zeigt den Verlauf des Signals in Reaktion mit Matrize. Pfeil 2 zeigt den Verlauf der Reaktion ohne Matrize. Nach einer anfänglichen Plato-Phase wurde ein Signalanstieg des Fluoreszenzsignals ab ca. 30 min in der Reaktion beobachtet, welche Matrize-001 enthielt. Die Kontroll-Reaktion ohne Matrize-001 zeigte keine Änderung in der Signal-Intensität über den gesamten aufgezeichneten Zeitraum von 40 min. Das durchgehend hohe Niveau der Grundfluoreszenz hängt mit der Tatsache zusammen, dass der Primer-001-01 einen Fluoreszenzmarker enthält (dT-TMR in Position 11).

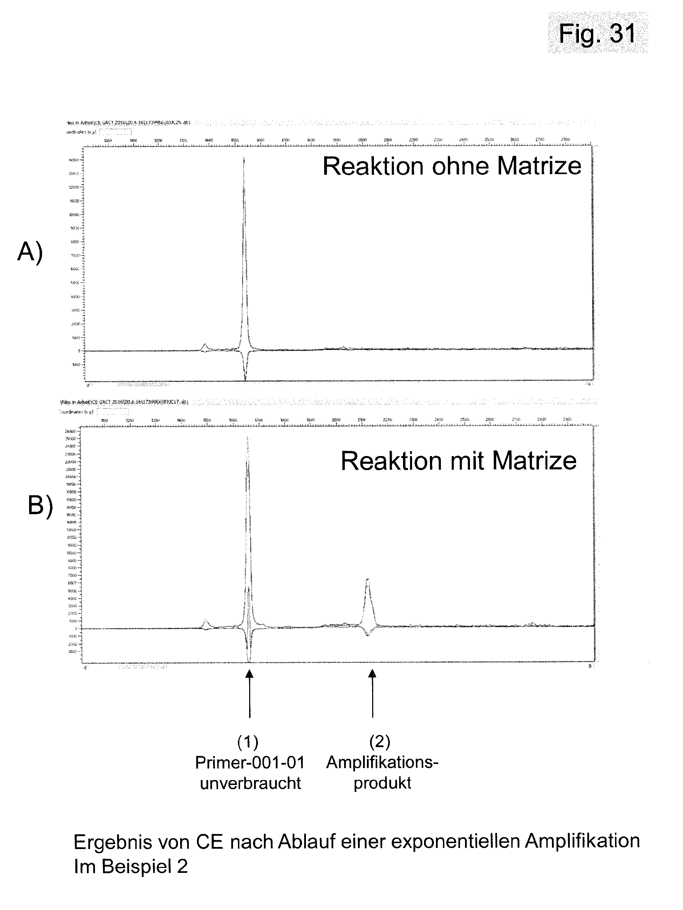

Zur Überpfüfung der Identität des Signals wurde je ein Aliquot von beiden Reaktionen mittels CE analysiert. Da das erste Primer-Oligonukleotid einen Fluoreszenzmarker einschließt (dT-TMR in Position 11) konnte das Signal während CE aufgezeichnet werden. Das Ergebnis der CE ist in Fig. 31 zu sehen.

In Fig. 31 (A) ist CE-Verlauf der Kontroll-Reaktion zu sehen. Man sieht einen Peak, welcher dem nicht verbrauchten Primer-001-01 zugeordnet wurde. In Fig. 31 (B) ist CE-Verlauf der Reaktion mit Matrize zu sehen. Man sieht einen Peak (1), welcher dem unverbrauchten Primer-001-01 entspricht, sowie einen weiteren Peak (2), welcher um ca. 460 CE-Einheiten nach rechts verschoben ist (was einem längeren Molekül entspricht). In parallelen Kontroll-Experimenten wurde festgestellt, dass unter gegebenen CE-Bedingungen 10,2 bis ca. 10,3 CE-Längen-Einheiten einer Sequenzdifferenz von einem Nukleotid entsprechen. Somit entspricht die in CE gemessene Distanz zwischen dem Peak (1) und dem Peak (2) einer Differenz in der Länge von ca. 44 - 45 Nukleotiden zwischen beiden aufgetrennten markierten Sequenzen. Diese Sequenzdifferenz stimmt mit der Länge des zu erwartenden Verlängerungsproduktes des ersten Primer-Verlängerungsproduktes (SEQ ID NO 20) in einer exponentiellen Amplifikation überein, sie beträgt 45 Nukleotide. 
5´-     GTACCGAAGCTCGCAGGAAC
TCAGAGTGTGGAGAGGACGTAAGTGACATTGTAGTATGTTTTTTT -3´ (SEQ ID NO 20)
 (das zu erwartete erste Verlängerungsprodukt des ersten Primer-Oligonukleotids ist unterstrichen)

Die Intensität des Signals in Peak (2) zeigt, dass weniger als 50% des anfänglich eingesetzten Primer-001-01 in der Reaktion verlängert wurde. Die Konzentration des entstandenen Produktes wurde auf etwa 1 µmol/l bis 2 µmol/l geschätzt, ausgehend von der Signalintensität des verbrauchten Primers. Somit betrug die Ausbeute ca. 10 - 20% gemessen nach dem Primer-001-01 Verbrauch.

In der exponentiellen Amplifikation erfolgte somit eine Matrizen-induzierte Vermehrung der zu amplifizierenden Sequenz, wobei auf die Ausgangskonzentration der Matrize-001 von 2 fmol/l entsprach und die beobachtete Konzentration des Produktes der Amplifikationsreaktion auf ca. 1 bis 2 µmol/l geschätzt wurde. Der daraus errechnete Vermehrungsfaktor beträgt somit ca. 5x10^8 bis 10^9. Die exponentielle Amplifikation verlief unter isothermalen Bedinungen.

Das Ergebnis der exponentiellen Reaktion ist abhängig von Anwesenheit der Matrize und Aktivator-Oligonukleotid, sowie Primer-1, Primer-2, Polymerase, dNTP's und passenden Puffer-Bedinungen. Weiterhin ist die Reaktion abhängig von der SequenzZusammensetzung einzelner beteiligten Nukleinsäurekomponenten: Aktivator-Oligonukleotid und Primer-1 und Primer-2. Im Folgenden wird auf das Struktur-Wirkung-Prinzip dieser Komponenten eingegangen.

### Erläuterungen zum Design von Sequenzen

### Matrize:

Die Sequenz der zu amplifizierenden Nukleinsäure dient als Ausgangspunkt für Design der Sequenzen von Primer-1, Primer-2 und Aktivator-Oligonukleotid. Diese zu amplifizierende Nukleinsäure umfasst eine Zielsequenz, welche spezifisch amplifiziert werden muss.

Die Sequenzlänge des zu erwartenden Verlängerungsprodukts des ersten Primer-Oligonukleotids beträgt 45 Nukleotide. Die Gesamtlänge des ersten Primer-Verlängerungsproduktes beträgt 65 Nukleotide. Die zu erwartende Länge des Verlängerungsproduktes liegt zwischen 25 und 29 Nukleotide. Die Gesamtlänge des zweiten Primer-Verlängerungsproduktes liegt zwischen 52 und 56 Nukleotiden (je nach Position des Stopps der Polymerase während der Synthese des zweiten Primer-Verlängerungsproduktes). Die Länge des komplementären Doppelstranges der zu amplifizierenden Nukleinsäure, bestehend aus dem ersten Primer-Verlängerungsprodukt und dem zweiten Primer-Verlängerungsprodukt, liegt somit zwischen 52 und 56 Nukleotide. Das erste Primer-Verlängerungsprodukt hat in seinem 5'-Segment einen einzelsträngigen Bereich, welcher dem Polynukleotid-Schwanz des ersten Primer-Oligonukleotides entspricht.

Die Sequenzlänge der zu amplifizierenden Nukleinsäure ist im vorliegenden Beispiel hinreichend groß, so dass die Schmelztemperatur des während der Reaktion gebildeten spezifischen Doppelstranges aus dem ersten und dem zweiten Primer-Verlängerungsprodukt ca. 78°C beträgt und somit über der Reaktionstemperatur von 65°C liegt. Es wird angenommen, dass während der Reaktion gebildeter Doppelstrang im Wesentlichen stabil ist und nicht spontan in seine Einzelstränge zerfällt.

### Primer 1

Das erste Primer-Oligonukleotid schließt einen ersten Primer-Bereich und einen zweiten Bereich mit einem Polynukleotid-Schwanz, welcher nicht von Polymerase kopiert wird und in der Lage ist, Aktivator-Oligonukleotid zu binden.

### Erster Primer-Bereich:

Positionen 14 - 20 bilden den ersten Primer-Bereich des ersten Primer-Oligonukleotid. Diese '-ständigen Nukleotide von Primer-001-01 (Positionen 14 - 20) sollen an die Matrize spezifisch binden und eine Primer-Verlängerungs-Reaktion ermöglichen. Daher wird die Sequenz dieser Positionen der Matrize 001 angepasst (Positionen 38 - 44). Diese sieben 3'-endständigen Nukleotide sind DNA-Nukleotide und sind mittels Phosphodiester-Verbindungen verknüpft. Dieses Segment initiiert die Primer-Verlängerung durch eine Polymerase bei der Synthese des ersten Stranges und dient als Matrize bei der Synthese des zweiten Stranges durch die Polymerase, so dass ein zu diesem Segment komplementärer Strang gebildet werden kann.

### Zweiter Primer-Bereich:

Der zweite Bereich ist an das 5'-Seite an den ersten Primer-Bereich angeschlossen (Positionen 1 - 13). Die Strukturen im zweiten Bereich sollen unter anderem Polymerase bei der Synthese des zweiten Stranges am Kopieren des Polynukleotid-Schwanzes hindern. Weiterhin sollen diese Strukturen in der Lage sein, Aktivator-Oligonukleotid zu binden und es bei Strangverdrängung unterstützen. Aus diesem Grund wurden vorwiegend 2'-O-Methyl Nukleotid-Modifikationen verwendet (Positionen 4 bis 10 und 12 bis 13) welche untereinander via 5'-3' Phosphodiester-Bindung verknüpft sind. Diese 2'-O-Methyl Nukleotid-Modifikationen stellen Beispiel für Modifikationen dar, welche in der Lage sind, Polymerase am Kopieren des Polynukleotid-Schwanzes zu hindern.

Die Sequenz von 5'-ständigen Nukleotide von Primer-001-01 (Positionen 1 - 9) ist zufällig gewählt, wobei darauf geachtet wurde, dass diese Sequenz keine extensive sebstkomplementäre Segmente, weiterhin keine komplementäre Bindungsstellen in der zu amplifizierenden Matrize hat, sowie keine multiple G-Nukleotide hat, welche in benachbarten Positionen stehen und somit G-Quadruplexe bilden könnten. Die Positionen 10 bis 13 sind komplementär zu Matrize-001 und sollen unter anderem Bindung des ersten Bereichs an die Matrize unterstützen.

Die Sequenz (Positionen 1 - 9) dient als Polynukleotid-Schwanz und soll während der Reaktion in der Lage sein, Aktivator-Oligonukleotid zu binden und dadurch räumliche Nähe zum Duplex-Ende herbeiführen, so dass Strangverdrängung stattfinden kann. Positionen 1 bis 3 sind durch DNA-Nukleotide dargestellt, Positionen 4 bis 9 sind 2'-O-Me Nukleotid-Modifikationen. Alle Nukleotide und Nukleotid-Modifikationen sind unter einander via Phosphodiester-Bindung verknüpft.

Der Übergang zwischen dem ersten Primer-Bereich und dem zweiten Bereich mit Polynukleotid-Schwanz ist in diesem Beispiel nahtlos: der zweite Bereich ist direkt an den ersten Bereich via Phosphordiester-Bindung angekoppelt. Das schafft günstige Voraussetzung für die Initiierung einer Strangverdrängung an einem Duplex-Ende durch ein Aktivator-Oligonukleotid. Beide Bereiche sind in gleicher Richtung angeordnet: von 5' zu 3'. Der zweite Bereich mit Polynukleotid-Schwanz liegt im 5'-Segment, der erste Primer-Bereich liegt im 3'-Segment.

Bei der Synthese des zweiten komplementären Stranges ist Polymerase ab Position 13 des ersten Primer-Oligonukleotids mit 2'-O-Methyl-Nukleotid-Modifikationen konfrontiert, welche weitere Synthese blockieren bzw. erschweren. Zwar führt eine einzelne 2'-O-Me-Nukleotid-Modifikation (wenn im Matrizenstrang eingefügt) nicht zwingend zur vollständigen Blockade der Polymerase-Synthese, so kommt es zur Erschwerung bzw. Verlangsamung der weiteren Synthese. Dank sequenzieller Kopplung von mehreren Nukleotid-Modifikationen wird Polymerase in ihrer Synthesfähigkeit erfolgreich gehemmt und kann den restlichen Polynukleotid-Schwanz nicht kopieren. Ob die vollständige Hemmung der Polymerase im Bereich des zweiten Bereichs des Primer-Oligonukleotid bereits nach einer Kopierung einer einzigen 2'-O-Methyl-Nukleotid-Modifikation auftritt oder erst nach Kopierung von mehreren 2'-O-Methyl-Nukleotid-Modifikationen ist zweitrangig, solange genügend nicht kopierte Nukleotide bzw. Nukleotid-Modifikationen im zweiten Bereich bleiben (Polynukleotid-Schwanz) und eine Bindung mit Aktivator-Oligonukleotid unter Reaktionsbedinugnen eingehen können, um eine erfolgreiche Strangverdrängung zu initiieren.

Aus Gründen der Anschaulichkeit und nicht als Einschränkung wird hiermit festgehalten, dass die Polymerase bis einschließlich Position 10 des ersten Primer-Oligonukleotides kopieren kann. Das bedeutet, dass das an den ersten Primer-Bereich angrenzende Segment des zweiten Bereichs von Polymerase teilweise kopiert wird. Bei der schematischen Darstellung werden deshalb vier Nukleotid-Positionen des zweiten Bereichs als von Polymerase kopiert betrachtet. Die restlichen Nukleotid-Positionen des zweiten Bereichs bleiben unkopiert und ermöglichen eine spezifische Bindung von Aktivator-Oligonukleotid.

### Primer 2:

Dieser Primer-2 ist für die Initiierung der Synthese des zweiten Stranges notwendig. Die Nukleotide in Positionen 9 - 27 sind mit der Matrize-001 identisch. Diese Positionen sollten zu Beginn der Amplifikation an das 3'-Segment des ersten Primer-Verlängerungsproduktes komplementär binden und damit die Synthese des zweiten Stranges initiieren. Der Primer 2 besteht aus DNA-Nukleotiden, welche untereinander via konventionelle 5'-3'-Phospordiesterbindungen verknüpft sind. Das 5'-Segment des zweiten Primers ist in diesem Beispiel bildet zunächst einen Überhang (Position 1 bis 8), welcher aber von einer Polymerase kopiert werden kann. Im Laufe der Reaktion kommt es zu Verlängerung der zu amplifizierenden Nukleinsäurekette um den 5'-Überhang des zweiten Primers.

Primer 2 nutzt das erste Primer-Verlängerungsprodukt als Matrize, so dass kopierbare Anteile des ersten Primer-Verlängerungsproduktes von Polymerase kopiert werden. Dadurch kommt es zu Verlängerung des zweiten Primers und Bildung eines Doppelstranges im kopierbaren Segment des ersten Primer-Verlängerungsproduktes. Der Polynukleotid-Schwanz des zweiten Bereichs des ersten Primers bleibt allerdings unkopiert. Das Primer-Verlängerungsprodukt schließt in seinem 3'-Segment eine Sequenz ein, welche in der Lage ist, das erste Primer-Oligonukleotid komplementär zu binden.

### Aktivator-Oligonukleotid:

### Erster Bereich:

Der erste Bereich (Position 35 - 50) schließt eine komplementäre Sequenz (Position 35 - 47) zum zweiten Bereich des ersten Primer-Oligonukleotides (Positionen 1 - 13) ein. Die drei 3'-endständigen Nukleotide bilden einen 3'-Überhang und haben im vorliegenden Beispiel keine funktionelle Bedeutung. Der erste Bereich besteht in diesem Beispiel aus 2'-Deoxynukleotiden, welche via eine konventionelle 5'-2' Phosphodiester-Bindung gekoppelt sind. Die endständige 3'-OH-Gruppe ist durch Phosphat-Rest blockiert und kann somit nicht von Polymerase verlängert werden.

### Zweiter Bereich:

Der zweite Bereich des Aktivator-Oligonukleotids schließt eine komplementäre Sequenz (Position 28 - 34) zum ersten Bereich des ersten Primer-Oligonukleotides (Positionen 14 - 20) ein. Der zweite Bereich des Aktivator-Oligonukleotids schließt in seinem 5'-Segment 2'-O-Methyl-Nukleotid-Modifikationen ein (Position 28 - 32) ein. Positionen 34 und 35 sind durch 2'-Deoxynukleotiden gebildet. Die einzelnen Nukleotide und Nukleotid-Modifikationen sind untereinander via eine konventionelle 5'-3'- Phosphodiester-Bindung gekoppelt. Der erste und der zweite Bereich des Aktivator-Oligonukleotides sind nahtlos aneinander via eine konventionelle 5'-3'- Phosphodiester-Bindung gekoppelt.

Die Sequenz-Länge und die ihre Beschaffenheit des ersten und zweiten Bereichs des Aktivator-Oligonukleotids ist an komplementäre Bereiche des ersten Primer-Oligonukleotides abgestimmt, so dass unter Reaktionsbedingungen das erste Primer-Oligonukleotid reversibel an das Aktivator-Oligonukleotid binden kann. Somit besteht ein Gleichgewicht zwischen freier, einzelsträngiger und gebundener, doppelsträngiger Form zwischen diesen beiden Komponenten.

### Dritter Bereich:

Der dritte Bereich des Aktivator-Oligonukleotids schließt eine komplementäre Sequenz (Position 9 - 27) zu einem Segment des zu erwartenden ersten Primer-Verlängerungsprodukt (Positionen 21 - 39), welches durch Polymerase während der Amplifikation synthetisiert werden soll. Der dritte Bereich besteht auf seiner gesamten Länge aus 2'-O-Methyl-Nukleotid-Modifikationen (Position 1 - 27). Die einzelnen Nukleotid-Modifikationen sind untereinander via eine konventionelle 5'-3'-Phosphodiester-Bindung gekoppelt. Der zweite und der dritte Bereich des Aktivator-Oligonukleotides sind nahtlos aneinander via eine konventionelle 5'-3'- Phosphodiester-Bindung gekoppelt. Die acht 5'-endständigen Nukleotid-Modifikationen bilden einen 5'-Überhang, welcher nicht zum ersten Primer-Verlängerungsprodukt komplementär ist.

Die Sequenz des Aktivator-Oligonukleotids ist dermaßen zusammengesetzt, dass es mit seinen Positionen 9 - 47 mit einem Teil des ersten Primer-Verlängerungsprodukts (Positionen 1 bis 39) einen komplementären Doppelstrang bilden kann. Positionen 1 bis 9, sowie Positionen 48 - 50 nehmen an der Ausbildung eines Doppelstranges mit dem ersten Primer-Verlängerungsprodukt nicht teil. Gleichzeitig ist die Sequenz des Aktivator-Oligonukleotids dermaßen zusammengesetzt, dass es mit seinen Nukleotid-Positionen 28 - 47 mit dem ersten Primer-Oligonukleotid einen komplementären Doppelstrang bilden kann. Dabei kann 3'-Ende des ersten Primers an das Aktivator-Oligonukleotid komplementär bindem. Um die Verlängerung des 3'-Ende des ersten Primers-Oligonukleotides durch eine Polymerase zu verhindern, schließt das Aktivator-Oligonukleotid Nukleotid-Modifikationen ein, welche die synthetische Wirkung der Polymerase verhindern. In diesem Beispiel sind es 2'-O-Methyl-Nukleotid-Modifikationen. Das Aktivator-Oligonukleotid in diesem Beispiel besteht an Positionen 1 - 32 aus 2'-O-Methyl-Nukleotid-Modifikationen. Dadurch bindet das 3'-Ende des ersten Primers bei korrekter Hybridisierung im Sequenz-Segment, welcher Nukleotid-Modifikationen umfasst. Eine solche Anordnung der Interaktion zwischen dem Aktator-Oligonukleotid und dem ersten Primer-Oligonukleotid soll eine ungewollte Verlängerung des ersten Primer-Oligonukleotids verhindern, wenn es am Aktivator-Oligonukleotid komplementär gebunden hat. Dank Modifizierungen im dritten Bereich des Aktivator-Oligonukleotides wird auch vorbeugend die Möglichkeit einer unspezifischen Verlängerung durch Polymerase generell reduziert. Um die Strandverdrängung zu fördern, wurden in entsprechenden Segmenten des Aktivator-Oligonukleotides Sequenzen eingesetzt, welche ihren Bindungspartnern im ersten Primer-Verlängerungsprodukt komplementär sind. Da sowohl Sequenz-Mismatches als auch Kettenunterbrechung durch Modifikationen ohne Nukleobasen (z.B. C3-Linker oder C18-Linker) die Effektivität einer Strandverdrängung und / oder ihre Geschwindigkeit reduzieren können, wurden solche Struktur-Varianten in diesem Beispiel vermieden.

Aktivator-Oligonukleotid soll unter Reaktionsbedingungen in Kontakt mit dem Doppelstrang der zu amplifizierenden Nukleinsäure kommen, über eine Bindung an den Polynukleotid-Schwanz des ersten Primer-Verlängerungsprodukts. Die Bindung ist vorzugsweise unter Reaktionbedingungen reversibel. Der Doppelstrang besteht dabei entweder am Beginn der Reaktion aus dem ersten Primer-Verlängerungsprodukt und der Matrize-001 und im späteren Verlauf der Reaktion aus dem ersten und dem zweiten Primer-Verlängerungsprodukt.

Die Beschaffenheit und die Länge des Aktivator-Oligonukleotids sollte hinreichend sein, um eine Strangverdrängung durchzuführen, welche zu einer Trennung der Primer-Verlängerungsprodukte in hinreichenden Ausbeuten führt. Die hinreichende Trennung der beiden Primer-Verlängerungsprodukte soll gewährleisten, dass Sequenzsegmente der beiden Primer-Verlängerungsprodukte für die Bindung von jeweils komplementären Primer-Oligonukleotide zugängig werden. Im vorliegenden Beispiel beträgt die Länge des Aktivator-Oligonukleotides 50 Nukleotide (Deoxynukleotide plus Nukleotid-Modifikationen), davon können 39 eine komplementäre Bindung mit dem ersten Primer-Verlängerungsprodukt einhergehen. Aktivator-Oligonukleotid war dadurch in der Lage, zunächst die Matrize-001 und im späteren Verlauf der Reaktion auch das zweite Primer-Verlängerungsprodukt unter Reaktionsbedingungen mittels Strangverdrängung zumindest teilweise aus der Bindung mit dem ersten Primer-Verlängerungsprodukt zu verdrängen.

Dabei wurden Matrize-001 bzw. das zweite Primer-Verlängerungsprodukt vollständig oder teilweise einzelsträngig, so dass ein neues erstes Primer-Oligonukleotid an das entsprechend komplementäres Sequenzsegment binden konnte und mittels Polymerase verlängert werden konnte. Im vorliegenden Beispiel bindet der dritte Bereich nur ein 5'-ständiges Segment des ersten Primer-Verlängerungsproduktes. Im vorliegenden Beispiel beträgt die Länge dieses komplementären Segments 19 Nukleotide.

Die 3'-ständigen Sequenzanteile des ersten Primer-Verlängerungsproduktes wurden von Aktivator-Oligonukleotid nicht gebunden. Dieses 3'-ständige Segment des ersten Primer-Verlängerungsprodukts soll in seiner einzelsträngiger Form in der Lage sein, das zweite Primer-Oligonukleotid zu binden, um eine Primer-Verlängerungsreaktion zu ermöglichen. Damit im Verlauf der Reaktion ein weiteres zweites Primer-Oligonukleotid an das erste Primer-Verlängerungsfragment binden kann, muss das 3'-ständiges Segment des ersten Primer-Verlängerungsproduktes in einzelsträngige Form überführt werden und aus der Bindung mit der Matrize oder mit einem zweiten Primer-Verlängerungsprodukt getrennt werden. Im vorliegenden Beispiel wird dies durch Trennung der beiden synthetisierten Stränge unter Reaktionsbedingungen während einer isothemalen Reaktion erreicht. Während der Strangverdrängung durch das Aktivator-Oligonukleotids kommt es zu Ausbildung eines Zwischenprodukts der Strangverdrängungsreaktion, welches aus einem ersten Primer-Verlängerungsprodukt, dem Aktivator-Oligonukleotid und einem zweiten Primer-Verlängerungsprodukt besteht. Das zweite Primer-Verlängerungsprodukt ist in diesem Zwischenprodukt an das 3'-ständige Segment des ersten Primer-Verlängerungsproduktes komplementär gebunden. Die Stabilität dieses resultierenden Doppelstranges ist allerdings im Vergleich zu einem vollständig gebundenen zweiten Primer-Verlängerungsprodukt deutlich herabgesetzt. Unter Reaktionsbedingungen kommt es zu einer spontanen Trennung dieses Doppelstrangs, so dass das zweite Primer-Verlängerungsprodukt vollständig vom ersten Primer-Verlängerungsprodukt abgelöst wird. Das 3'-Segment des ersten Primer-Verlängerungsproduktes ist unmittelbar nach der Trennung vom zweiten Primer-Verlängerungsprodukt einzelsträngig.

Das einzelsträngige 3'-endständige Segment des ersten Primer-Verlängerungsproduktes kann sowohl in Kontakt mit der Matrize bzw. mit dem zweiten Primer-Verlängerungsprodukt, als auch eine neue Bindung mit dem zweiten Primer-Oligonukleotid eingehen. Aufgrund eines molaren Überschusses des eingesetzten zweiten Primer-Oligonukleotid ist die Wahrscheinlichkeit des Kontaktes mit dem zweiten Primer-Oligonukleotid wesentlich höher. Dies ist insbesondere zu Beginn der Reaktion der Fall: die Matrize wird im vorliegenden Beispiel in Konzentration von 1 fmol/l eingesetzt. Während der ersten Verdoppelungszyklen der exponentiellen Amplifikation bleibt die Konzentration von synthetisierten zweiten Primer-Verlängerungsprodukten ebenfalls längere Zeit deutlich unter dem Wert von 1 µmol/l. Das zweite Primer-Oligonukleotid dagegen in Konzentration 10 µmol/l. Es resultiert somit ein 10¹⁰ Überschuss an zweiten Primer-Oligonukleotiden über die Matrize und über die synthetisierten zweiten Primer-Verlängerungsprodukte insbesondere während der anfänglichen Phasen der exponentiellen Amplifikation. In späteren Phasen der Amplifikation konkurriert das nun in höhreren Konzentrationen vorliegende zweite Primer-Verlängerungsprodukt um die Bindung an das 3'-Segment des ersten Primer-Verlängerungsproduktes, so dass es zu einer Verlangsamung der Reaktion kommen kann.

### Beispiel 3:

### Überprüfung der Spezifität der Amplifikations-Reaktion

In diesem Beispiel wurde Einfluss einer Sequenzänderung in der Matrize auf die Amplifikation untersucht. Drei Nukleotide aus dem mittleren Bereich der Matrize wurden entfernt. Die randständigen Bereiche, welche für die Bindung von Primern entscheidend sind wurden identisch gestaltet. Auf diese Weise sollte überprüft werden, welche Auswirkung ein solcher Mismatch zwischen einem dabei entstehenden ersten Primer-Verlängerungsprodukt und einem Aktivator-Oligonukleotid auf die Amplifikation hat. Die Gestaltung von Sequenzen erfolgte in ähnlicher Art und Weise wie im Beispiel 1.

Folgende Matrizen wurden verwendet:
- Matrize (SEQ ID NO 21) mit Sequenzzusammensetzung, welche zu einem ersten Primer-Verlängerungsprodukt mit einem Perfekt-Match Übereinstimmung mit Aktivator-Oligonukleotid führt:

T-A6P-15-4401 5′ GTAGTTCTCACTTACGTCCTCTCCACACTCTGAGTTCCTGCGAG
3′

Bindungssequenz für das erste Primer-Oligonukleotid ist unterstrichen.
- Matrize (SEQ ID NO 22) mit Sequenzzusammensetzung, welche zu einem ersten Primer-Verlängerungsprodukt mit einem MisMatch mit Aktivator-Oligonukleotid führt:
   T-A6P-15-4401-5 5' GTAGTTCTCACTTACGTCCTCTCCCTCTGAGTTCCTGCGAG 3' Bindungssequenz für das erste Primer-Oligonukleotid ist unterstrichen.

Bei beiden Matrizen sind jeweilige Primer-Bindungsstellen identisch, so dass zu erwarten ist, dass bei Primer-Oligonukleotiden jeweils eine erfolgreiche erste Primer-Verlängerungsreaktion initiieren können.

Beide Matrizen unterscheiden sich nur an einer Stelle:
T-A6P-15-4401 5' GTAGTTCTCACTTACGTCCTCTCCACACTCTGAGTTCCTGCGAG 3' (SEQ ID NO 21)
T-A6P-15-4401-5 5'GTAGTTCTCACTTACGTCCTCTCC_CTCTGAGTTCCTGCGAG 3' (SEQ ID NO 22)

### Die Differenz-Stelle ist unterstrichen.

Somit sind auch Unterschiede in jeweiligen Primer-Verlängerungsprodukten des ersten und des zweiten Primer-Oligonukleotide zu erwarten. Die Differenzstelle liegt im 5'-Segment des Verlängerungsprodukt des ersten Primer-Oligonukleotides.

Folgende Primer wurden verwendet:
- Das erste Primer-Oligonukleotid (SEQ ID NO 23):
   A6P-10-1002 TMR7 5' 7765567876 CAGGAAC 3'
   A = 2'-deoxy-Adenosin
   C = 2'-deoxy-Cytosin
   G = 2'-deoxy-Guanosin
   T = 2'-deoxy-Thymidin (Thymidin)
   Modifikationen:
   5 = 2'-O-Me A (2'-O-Methyl-Adenosine)
   6 = 2'-O-Me G (2'-O-Methyl-Guanosine)
   7 = 2'-O-Me C (2'-O-Methyl-Cytosine)
   8 = dT-TMR
- Das zweite Primer-Oligonukleotid (SEQ ID NO 24):
   PA6-15-4401-10 5' CACGAGTAGTTCTCACTTAC 3'

Folgendes Aktivator-Oligonukleotid-002-01 (SEQ ID NO 25) wurde verwendet:
A6 8504 
5′- CTAGCTAT CGTCCTCT CCACACT CTGA GTTCCTGCGAG CTTC GGTAC AAG  X 3′
68576858  67866868  6656568 6875  788668 7CGAG CTTC GGTAC AAG
A = 2'-deoxy-Adenosin
C = 2'-deoxy-Cytosin
G = 2'-deoxy-Guanosin
T = 2'-deoxy-Thymidin (Thymidin
5 = 2'-O-Me A (2'-O-Methyl-Adenosine)
6 = 2'-O-Me C (2'-O-Methyl-Cytosine)
7 = 2'-O-Me G (2'-O-Methyl-Guanosine)
8 = 2'-O-Me U (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindung verknüpft. Das 3'-Ende ist mit einer Phosphat-Gruppe blockiert, um eine mögliche Verlängerung durch die Polymerase zu verhindern.

Die Matrize mit Perfekt-Match Sequenzzusammensetzung wurde in Konzentration von 1 nmol/l, 1 pmol /l und 1 fmol/l eingesetzt.

Die Matrize mit Mismatch Sequenzzusammensetzung wurde in Konzentration von 1 nmol/l verwendet.

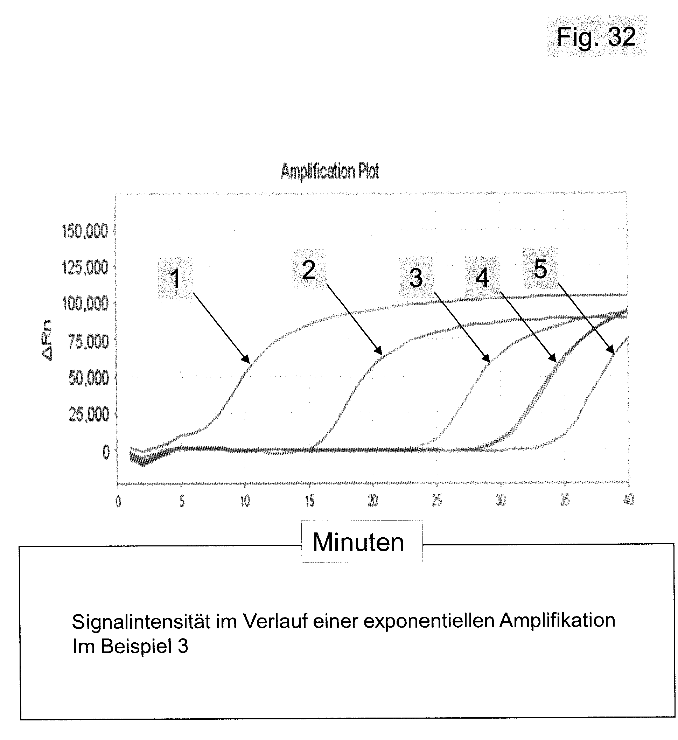

In der Kontroll-Reaktion wurde keine Matrize eingesetzt. Sonstige Reaktionsbedinungen wurden wie im Beispiel 2 gehalten. Fig. 32 zeigt das Ergebnis der Reaktion.

Bei Verwendung einer Perfect-Match Matrize (SEQ ID NO 21) kommt es zur Synthese eines komplementären Stranges eines Primer-Verlängerungsproduktes. Dieses Verlängerungsprodukt ist sowohl zu Perfect-Match-Matrize komplementär als auch zum verwendeten Aktivator-Oligonukleotid. Diese Konstellation wurde im Beispiel 1 ausführlich dargestellt. Sie dient als Grundlage für eine erfolgreiche Amplifikation.

Im Gegensatz dazu, unter Verwendung einer Mismatch-Sequenz (SEQ ID NO 22) kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Fehlen von drei Nukleotiden in entsprechenden Positionen eine Strangverdrängung durch das Aktivator-Oligonukleotid.

Die Fig. 32 zeigt diesen Einfluss der Sequenzunterschiede zwischen Aktivator-Oligonukleotid und dem Verlängerungsprodukt des ersten Primer-Oligonukleotids. Abweichungen an 3 Positionen in der Sequenz des Aktivator-Oligonukleotides von der Sequenz des an der Mismatch-Matrize synthetisierten Primer-Verlängerungsproduktes zeigt die Abhängigkeit der Amplifikation von der Übereinstimmung in der Reihenfolge der komplementären Basen im Aktivator-Oligonukleotid und Matrize bzw. dem ersten Primer-Verlängerungsprodukt. Eine Wegnahme von nur 3 Basen aus dem Bereich des Verlängerungsproduktes führte bereits zu einer Unterdrückung der Amplifikation auf ein Niveau, was im vorliegenden Beispiel sogar unter der Negativkontrolle liegt.

Die Kontrollreaktionen mit einer Perfect Match Matrize (Pfeile 1 - 3) zeigten eine Konzentrationsabhängigkeit der Amplifikation. Mit sinkender Konzentration brauchte Reaktion längere Zeit, um eine ausreichende Menge der zu amplifizierenden Nukleinsäure zu synthetisieren, damit das Signal über Niveau der Base-line ansteigt.

Die Negativ-Kontroll Reaktion ohne Matrize zeigte ebenfalls ein Anstieg im Signal, allerdings in einem signifikant späteren Zeitintervall zu Reaktionen mit einer Perfect-Match Matrize.

Die Pfeile in Fig. 32 zeigen:
Perfect Match Matrize (SEQ ID NO 21) 1 nmol/l Konzentration (Pfeil 1)
Perfect Match Matrize (SEQ ID NO 21) 1 pmol/l Konzentration (Pfeil 2)
Perfect Match Matrize (SEQ ID NO 21) 1 fmol/l Konzentration (Pfeil 3)
Negativ-Kontrolle: Keine Matrize in der Reaktion (Pfeil 4) (angezeigt sind 2 KontrollReaktionen, beide ohne Matrize)
Mismatch Matrize (SEQ ID NO 22) 1 nmol/l Konzentration (Pfeil 5)

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zur Unterbrechung der Amplifikation führen.

In diesem Beispiel wurde gezeigt, dass obwohl Sequenzenden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall fehlten drei Nukleotide im 5'-Segment des Verlängerungsproduktes des ersten Primers) führte zur starken Unterdrückung der Amplifikation.

### Beispiel 4:

### Verwendung einer anderen Matrizen-Sequenz mit einem anderen passenden Aktivator-Oligonukleotid

Zunächst wurde eine künstliche Sequenz von 49 Nukleotiden synthetisch (Eurofins) generiert, welche eine Sequenz des MIP-Gens von *Legionella pneumophila* zwischen dem Primer-1 und Primer-2 einschließt (SEQ ID NO 26), Matrize-003-01. Als Kontrolle wurde Matrize-003-02 (SEQ ID NO 27) mit Sequenzzusammensetzung synthetisch generiert (Eurofins), welche zu einem ersten Primer-Verlängerungsprodukt mit einem MisMatch mit Aktivator-Oligonukleotid führt (5 Nukleotide). Zur Amplifikation einer solchen neuen Matrize-003 wurden zwei Primer (Primer-1, erstes Primer-Oligonukleotid (identisch mit SEQ ID NO 13 im Beispiel 2) und Primer-2, zweites Primer-Oligonukleotid (SEQ ID NO 28) sowie ein zur Matrize-003-01 passendes Aktivator-Oligonukleotid (SEQ ID NO 29) generiert. Die Gestaltung von Sequenzen erfolgte in ähnlicher Art und Weise wie im Beispiel 2 beschrieben.

Folgende Matrizen wurden verwendet:
- Matrize-003-01 (SEQ ID NO 26) mit Sequenzzusammensetzung, welche zu einem ersten Primer-Verlängerungsprodukt mit einem Perfekt-Match Übereinstimmung mit Aktivator-Oligonukleotid führt:
   LP-T1-1000-103 5' CTACAATGTCACTTACACGTTCTTAACAAGTTTCAG**CC**GTTCCTGCGAG 3' Sequenzsegment des MIP-Gens von *L.pneumonia* ist unterstrichen. Die Matrize besteht aus DNA Nukleotiden.
- Matrize (SEQ ID NO 27) mit Sequenzzusammensetzung, welche zu einem ersten
   Primer-Verlängerungsprodukt mit einem MisMatch mit Aktivator-Oligonukleotid führt:
   LP-T1-1001-118 5'CTACAATGTCACTTACACGTTCTTAACAAGTTTGTTCCTGCGAG 3' Sequenzsegment des MIP-Gens von *L.pneumonia* ist unterstrichen (dieses Segment ist um 5 Nukleotide kürzer als in der SEQ ID NO 26). Die Matrize besteht aus DNA Nukleotiden.

Bei beiden Matrizen sind jeweilige Primer-Bindungsstellen identisch, so dass zu erwarten ist, dass eine erfolgreiche erste Primer-Verlängerungsreaktion durch Primer-Oligonukleotide jeweils initiieren können.
LP-T1-1000-103 5' CTACAATGTCACTTACACGTTCTTAACAAGTTTCAG**CC**GTTCCTGCGAG 3'(SEQ ID NO 26)
LP-T1-1001-118 5' CTACAATGTCACTTACACGTTCTTAACAAGTTT GTTCCTGCGAG 3'(SEQ ID NO 27)

### Bindungsstelle für Primer-1 unterstrichen.

Beide Matrizen unterscheiden sich nur an einer Position:
LP-T1-1000-103 5' CTACAATGTCACTTACACGTTCTTAACAAGTTTCAGCCGTTCCTGCGAG 3'(SEQ ID NO 26)
LP-T1-1001-118 5' CTACAATGTCACTTACACGTTCTTAACAAGTTT_GTTCCTGCGAG 3' (SEQ ID NO 27)

### Die Differenz-Stelle zwischen Matrizen ist unterstrichen.

Diese Position liegt im Bereich, welcher bei der Synthese des ersten Primer-Verlängerungsproduktes durch Polymerase synthetisiert wird. Die Differenzstelle liegt im 5'-Segment des Verlängerungsprodukt des ersten Primer-Oligonukleotides. Somit sind auch Unterschiede in jeweiligen Primer-Verlängerungsprodukten des ersten und des zweiten Primer-Oligonukleotide zu erwarten.

### Folgende Primer wurden verwendet:

Das erste Primer-Oligonukleotid (SEQ ID NO 13) ist identisch mit Primer 1 in Beispiel 2:

### Primer 1

Primer001-01 (20 Nukleotide):
Die Sequenz des ersten Primer-Oligonukleotides:
   A6P-10-1001 5' GTACCGAAGC**T**CGCAGGAAC 3' (SEQ ID NO 13) (Reihenfolge der Nummerierung: 1 - 20)

Unterstrichene Bereiche sind durch 2'-O-Me Modifikationen dargestellt. Nicht unterstrichene Bereiche sind 2'-deoxy-Nukleotide (DNA).
- Das zweite Primer-Oligonukleotid (SEQ ID NO 28):
   LP-P2-1000-211 5' CTACAATGTCACTTAC ACGTTCTTAACAAGTTT 3' (SEQ ID NO 17):

Das erste und das zweite Primer-Oligonukleotide können beide Matrizen (SEQ ID NO 26 und 27) an entsprechenden Primer-Bindungsstellen binden.

Folgendes Aktivator-Oligonukleotid-003 (SEQ ID NO 29) wurde verwendet:
LP-A1-1000-104

5´-
A*A*A*A*C*A*A*A[AACCGAACAACAAAUGAAAGACGUUCUUAACAAGUUUCAGCCGUU
CCUG ]CGAGCTTCGGTACAAG X 3´

Sequenz am 3'-ständigen Segment besteht aus DNA-Nukleotiden
A = 2'-deoxy-Adenosin
C = 2'-deoxy-Cytosin
G = 2'-deoxy-Guanosin
T = 2'-deoxy-Thymidin (Thymidin)

Sequenz zwischen eckigen Klammern [], Positionen 9 - 58, besteht aus 2'-O-Me-Nukleotid-Modifikationen, welche vie Phosphordiester-Bindung verknüpft sind: 2'-O-Me A (2'-O-Methyl-Adenosine), 2'-O-Me C (2'-O-Methyl-Cytosine), 2'-O-Me G (2'-O-Methyl-Guanosine), 2'-O-Me U (2'-O-Methyl-Uridine).

Verbindungen zwischen 5'-ständigen Nukleotiden (Positionen 1 - 8) sind via Phosphorothioat-Verbindungen (PTO) verknüpft; entsprechende Positionen sind mit (*) markiert.

X = 3'-Phosphat-Gruppe zur Blockade einer möglichen Verlängerung durch Polymerase. Das 3'-Ende ist mit einer Phosphat-Gruppe blockiert, um eine mögliche Verlängerung durch die Polymerase zu verhindern.

Die Konzentration von Matrizen zu Beginn der Reaktion betrug 10 pmol/l (für perfect match und mismatch). In einer weiteren Kontroll-Reaktion wurde keine Matrize eingesetzt. Die Reaktion wurde 60 min bei 65°C durchgeführt. Sonstige Reaktionsbedinungen wurden wie im Beispiel 1 gehalten.

### Ergebnisse

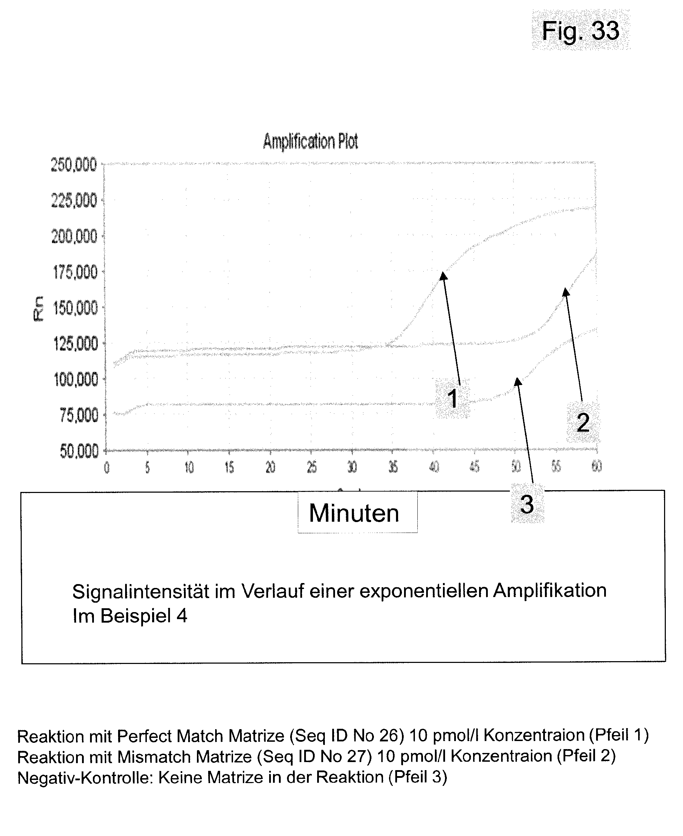

Der Verlauf der Reaktion ist in Fig. 33 abgebildet. Man erkennt, dass die Reaktion, welche eine perfekt-Match Matrize enthielt, führt ab 35 min zum Anstieg des Fluoreszenzsignals (Pfeil 1). Reaktion mit Mismatch und Negativ-Kontrolle führen zwar ebenfalls zu einem Signal, welches allerdings signifikant später kommt (ca. 45 min bzw. 50 min).

### Auswertung:

Der Signalanstieg in Fig. 33 korreliert mit der Anwesenheit einer Perfect-Match Matrize-003-01. Eine sehr ähnliche Sequenz, mit gleichen Primer-Bindungsstellen und einem signifikant ähnlichen Abschnitt zwischen den Primern, allerdings mit einer Deletion von 5 Nukleotiden, unterscheidet sich im Verlauf der Amplifikation und liegt im Bereich der Negativ-Kontrolle.

Dieser Unterschied wird auf folgende Sachverhältnisse zurückgeführt:
Bei Verwendung einer Perfect-Match Matrize (SEQ ID NO 26) kommt es zur Synthese eines komplementären Stranges eines Primer-Verlängerungsproduktes. Dieses Verlängerungsprodukt ist sowohl zu Perfect-Match-Matrize komplementär als auch zu entsprechenden Positionen des verwendeten Aktivator-Oligonukleotids (SEQ ID NO 29). Diese Konstellation wurde im Beispiel 1 ausführlich dargestellt. Sie dient als Grundlage für eine erfolgreiche Amplifikation.

Nachfolgend sind Sequenzen gegenübergestellt und jeweilige Segmente markiert, welche unter der Reaktion einen komplementären Doppelstrang mit entsprechenden Reaktionspartnern bilden:

### Matrizen

LP-T1-1000-103 5' CTACAATGTCACTTACACGTTCTTAACAAGTTTCAG**CC**GTTCCTGCGAG 3'(SEQ ID NO 26)
LP-T1-1001-118 5' CTACAATGTCACTTACACGTTCTTAACAAGTTT GTTCCTGCGAG 3' (SEQ ID NO 27)

### Bindungsstelle für Primer-1 ist unterstrichen.

### Primer-1 und Primer 2:

Primer 1: A6P-10-1001 5' GTACCGAAGC**T**CGCAGGAAC 3' (SEQ ID NO: 13) Bindungsstelle an Matrize ist unterstrichen.
Primer 2: LP-P2-1000-211 5' CTACAATGTCACTTAC ACGTTCTTAACAAGTTT 3' (SEQ ID NO 28):
   (unterstrichen ist 5'-Segment des zweiten Primer-Oligonukleotides, welches an das 3'-Segment des ersten Primer-Verlängerungsproduktes komplementär binden kann, wobei dieses 3'-Segment des ersten Primer-Verlängerungsproduktes nicht komplementär zum dritten Bereich des Aktivator-Oligonukleotid ist.)

### Aktivator-Oligonukleotid

LP-A1-1000-104 (SEQ ID NO 29)

5´-
A*A*A*A*C*A*A*A[AACCGAACAACAAAUGAAAGACGUUCUUAACAAGUUUCAGCCGUU
CCUG ]CGAGCTTCGGTACAAG X 3´

(Positionen des dritten Bereichs des Aktivator-Oligonukleotides, welche mit dem Verlängerungsprodukt des ersten Primer-Oligonukleotides an der Matrize-003-01 (SEQ ID NO 14) perfekt übereinstimmen sind unterstrichen.)
LP-A1-1000-104 (SEQ ID NO 29)

5´-
A*A*A*A*C*A*A*A[AACCGAACAACAAAUGAAAGACGUUCUUAACAAGUUUCAGCCGUU
CCUG ]CGAGCTTCGGTACAAG X 3´

(Positionen des Aktivator-Oligonukleotides, welche mit dem Primer-Verlängerungsprodukt des ersten Primer-Oligonukleotides an der Matrize-003-01 (SEQ ID NO 14) perfekt übereinstimmen sind unterstrichen.) Dieses Fragment ist länger als das Fragment, welches mit Verlängerungsprodukt des ersten Primer-Oligonukleotides interagiert, da das gesamte Primer-Verlängerungsprodukt aus dem ersten Primer-Oligonukleotid und dem Verlängerungsprodukt besteht.

Man erkennt, dass es einen nahtlosen Übergang in der Komplementarität zwischen dem ersten Primer-Verlängerungsprodukt (welches an der perfect-match Matrize-003-01 synthetisiert wurde) und den Segmenten des zweiten Bereiches und des dritten Bereich des Aktivator-Oligonukleotides gibt. Diese Konstellation unterstützt die Ausbildung des neuen Doppelstrangs zwischen dem Aktivator-Oligonukleotid und dem ersten Primer-Verlängerungsprodukt, so dass eine hinreichende Strangverdrängung des Matrizenstranges, bzw. einen zweiten Primer-Verlängerungsproduktes stattfinden kann. Insgesamt führt das zu einem positiven Amplifikationsergebnis.

Im Gegensatz dazu, unter Verwendung einer Mismatch-Sequenz (SEQ ID NO 15) kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Fehlen von fünf Nukleotiden in entsprechenden Positionen eine Strangverdrängung durch das Aktivator-Oligonukleotid, was zu Störung der Amplifikation führt.
LP-A1-1000-104 (SEQ ID NO 29)

5´-
A*A*A*A*C*A*A*A[AACCGAACAACAAAUGAAAGACGUUCUUAACAAGUUUCAGCCGUU
CCUG ]CGAGCTTCGGTACAAG X 3´

(Positionen des dritten Bereichs des Aktivator-Oligonukleotides, welche mit dem Verlängerungsprodukt des ersten Primer-Oligonukleotides an der Matrize-003-02 (SEQ ID NO 26) perfekt übereinstimmen sind unterstrichen.
LP-A1-1000-104 (SEQ ID NO 29)

5´-
A*A*A*A*C*A*A*A[AACCGAACAACAAAUGAAAGACGUUCUUAACAAGUUUCAGCCGUU
CCUG ]CGAGCTTCGGTACAAG X 3´

(Positionen des Aktivator-Oligonukleotides, welche mit dem Primer-Verlängerungsprodukt des ersten Primer-Oligonukleotides an der Matrize-003-01 (SEQ ID NO 27) perfekt übereinstimmen sind unterstrichen.

Man erkennt eine zu erwartende Unterbrechung in der Bindung eines komplementären Doppelstranges zwischen dem Aktivator-Oligonukleotid-003 und dem ersten Primer-Verlängerungsprodukt, welches an der Mismatch Matrize gebildet wurde. Diese Unterbrechung entspricht dem Anteil des Primer-Verlängerungsproduktes der Matrize-003-02 (SEQ ID NO 27), welcher dem weggelassenen Segment der Matrize-003-02 entspricht. Diese Position liegt zwischen dem zweiten und dem dritten Bereich des Aktivator-Oligonukleotides.

Eine solche Differenz in der Komplementarität der potenziell zu erwartenden Doppelstränge zwischen Aktivator-Oligonukleotid-003 (SEQ ID NO 29) und dem ersten Primer-Verlängerungsprodukt einer perfect-match Matrize-003-01 bzw. dem ersten Primer-Verlängerungsprodukt einer mismatch Matrize-003-02 kann zu Unterschieden in der Strangverdrängungsreaktion führen, welche in einer Hinderung der Amplifikation resultieren.

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zur Unterbrechung der Amplifikation führen.

In diesem Beispiel wurde gezeigt, dass obwohl Sequenzenden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall fehlten fünf Nukleotide im 5'-Segment des Verlängerungsproduktes des ersten Primers) führte zur Unterdrückung der Amplifikation.

### Beispiel 5:

In diesem Beispiel wird gezeigt, dass weitere Struktur-Varianten des ersten Primer-Oligonukleotides in der Lage sind, eine exponentielle Amplifikation einer zu amplifizierenen Nukleinsäurekette zu unterstützen. Diese Strukturvarianten unterscheiden sich in der zum Stopp der Polymerase verwendeten Gruppe bei der Synthese des zweiten Primer-Verlängerungsproduktes. Es wurden je eine Modifikation als eine zum Stopp der Polymerase führende Grupp verwendet: C3-Linker, C18-Linker (auch als HEG-Linker, Hexaethylenglycol-Linker, bekannt) oder Iso-dC-5-Me.

Weiterhin wird in diesem Beispiel gezeigt, dass ein Primer-2 mit seinem zum ersten Primer-Verlängerungsprodukt komplementären 3'-Segment das Aktivator-Oligonukleotid aus der Bindung mit dem ersten Primer-Verlängerungsprodukt teilweise verdrängen kann und eine erfolgreiche Amplifikation unterstützen kann.

Zu Testzwecken wurde die gleiche zu amplifizierende Nukleinsäure genommen, wie in Beispiel 2 beschrieben.
Matrize 001-01: (SEQ ID NO 12) T35-4401-601 5'- ATACTACAATGTCACTTACGTCCTCTCCACACTCTGAGTTCCTGCGAG - 3' (Reihenfolge der Nummerierung: 1 - 48)

Aktivator-Oligonukleotid:
Es wurde das gleiche Aktivator-Oligonukleotid-001-01 verwendet, wie im Beispiel 2 beschrieben:
   Aktivator-Olgonukleotid-001-01: (SEQ ID NO: 15) A6 8505 
5´- [CUAGCUAUCGUCCUCUCCACACUCUGA GUUCC] TGCGAGCTTCGGTACAAG X -
3´
 (Reihenfolge der Nummerierung: 1 - 50)

Das Aktivator-Oligonukleotid umfaßt 2'-O-Me Nukleotid-Modifikationen (angegeben in [ ]-Klammern) und DNA Nukleotide (im 3'-Segment). Die 3'-OH-Gruppe des 3'-endständigen Nukleotides war durch Phosphat-Rest blockiert (hier mit X gekennzeichnet).

Folgende Varianten des ersten Primer-Oligonukleotids wurden verwendet:
P-1001-100 TMR C3 (SEQ ID NO 30) 5'- 6T57765568 CTCGCAGGAAC - 3' 8 = C3-Linker
P-1001-103 TMR C18 (SEQ ID NO 31) 5'- 6T57765568 7T76CAGGAAC - 3' 8 = C18-Linker (HEG-Linker, Hexaethylenglycol)
P-10-1002-IsoC4 (SEQ ID NO 32) 5'- 7765568TCGCAGGAAC - 3' 8 = Iso-dC-5-Me (Modifikation)
weitere Positionen wurden wie folgt belegt:
A = 2'-deoxy-Adenosin
C = 2'-deoxy-Cytosin
G = 2'-deoxy-Guanosin
T = 2'-deoxy-Thymidin (Thymidin)

Modifikationen:
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
5'- wurde von ersten Primer-Oligonukleotiden wurden mit TAMRA-Farbstoff modifiziert. (TMR -Modifikation).

Der jeweilige zweite Bereich eines jeden ersten Primer-Oligonukleotid ist unterstrichen.

Es wurden jeweils konvetionelle 5'-3'-Verknüpfung zwischen benachbarten Nukleotiden bzw. Nukleotid-Modifikationen gewählt. Auch Modifikationen wurden in 5'-3'- Anordnung angebracht. Alle Sequenzen wurden durch kommerzielle Anbieter mit einer Standard-Synthese Chemie (Phosphoroamidit-Chemie) synthetisiert.

Das folgende zweite Primer-Oligonukleotid wurde verwendet:
Primer 001-02: (SEQ ID NO: NN 33) PA6-35-4401-604 5'- GCTCATACTACAATGTCACTTACGTCCTCT - 3' (Reihenfolge der Nummerierung in 5'-3': 1 - 30)

Der zweite Primer besteht aus DNA.

Zusammensetzung der Reaktionsmischung und Start der Reaktion wurde durchgeführt wie im Beispiel 2 beschrieben.

Diese Komponenten wurden wie folg in der Reaktionsmischung eingesetzt:
- Matrize 001-01: 10 pmol/l
- Aktivator-Oligonukleotid-001-01: 10 µmol/l
- Der jeweilige Primer-1: 10 µmol/l
- Primer 001-02: 10 µmol/l

Weitere Komponenten der Reaktion wurden als Amplifikationsreaktion Lösung 2 eingesetzt:
- Kalium Glutamat, 50 mmol/l, pH 8,0
- Magnesium Acetat, 10 mmol/l
- dNTP (dATP, dCTP, dTTP, dGTP), je 200 µmol/l
- Polymerase (Bst 2.0 WarmStart, 120.000 U/ml NEB), 12 Units /10 µl
- Triton X-100, 0,1% (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

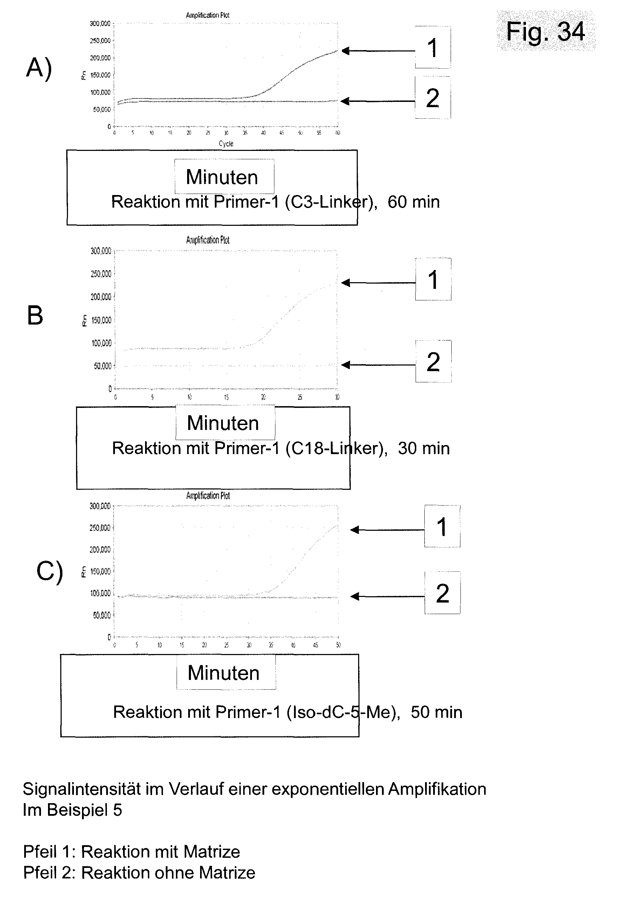

Die Reaktion wurde in der Amplifikationsreaktion Lösung 2 unter jeweils isothermalen Reaktionsbedingungen bei 65°C über die Zeitdauer (wie jeweils angegeben) durchgeführt. Das Fluoreszenzsignal von EvaGreen wurde während Reaktion aufgezeichnet, wie im Beispiel 2 beschrieben. Das Ergebnis der Aufzeichnungen der Signalintensität während der Reaktion ist in Fig. 34 dargestellt.

### Auswertung:

Unter Einsatz von angeführten ersten Primer-Oligonukleotiden ist Entstehung des folgenden ersten Primer-Verlängerungsprodukten zu erwarten:
(SEQ ID NO: NN 34) 5'-XXXXXXX TCAGAGTGTGGAGAGGACGTAAGTGACATTGTAGTATGAGC - 3'

Mit "XXXXXXX" ist die jeweilige Sequenz des entsprechenden ersten Primer-Oligonukleotides markiert. Direkt anschließend ist die Sequenz des zu erwartenden ersten Verlängerungsproduktes angegeben (41 NT). Das Verlängerungsprodukt ist unterstrichen.

Es wurden unterschiedliche Primer-1 Strukturen auf ihre Fähigkeit getestet, eine exponentielle Amplifikation unter isothermalen Bedingungen zu unterstützen. Dabei wurden im zweiten Bereich des ersten Primer-Oligonukleotides unterschiedliche zum Stopp der Polymerase führende Gruppen integriert: C3-Linker, C18-Linker (auch als HEG-Linker, Hexaethylenglycol-Linker, bekannt) oder Iso-dC-5-Me. Bei der Synthese des zweiten Primer-Verlängerungsproduktes kommt es zu einer positionsspezifischen Unterbrechung der Synthese des zweiten Primer-Verlängerungsproduktes, so dass Polynukleotid-Schwanz des zweiten Bereichs unkopiert bleibt.

Das jeweilige Signalprofil der Reaktion ist in Fig.34 abgebildet.
Die Reaktion mit Primer-1 (C3-Linker) mit SEQ ID NO: 30 ist in Fig. 34 (A) abgebildet.
Die Reaktion mit Primer-1 (C18-Linker) mit SEQ ID NO: 31 ist in Fig. 34 (B) abgebildet.
Die Reaktion mit Primer-1 (Iso-dC-5-Me) mit SEQ ID NO: 32 ist in Fig. 34 (C) abgebildet.

Alle angeführten Struktur-Varianten des ersten Primer-Oligonukleotides führten zu einer erfolgreichen exponentiellen Amplifikation. Beim Design der ersten Primer-Oligonukleotide wurden folgende Aspekte berücksichtigt:
- Erster Primer-Bereich des ersten Primer-Oligonukleotides muss spezifisch an die zu amplifizierende Nukleinsäure binden können und eine messbare Primer-Verlängerungsreaktion durch die verwendete Polymerase initiieren können.
- Erster Primer-Bereich des ersten Primer-Oligonukleotides muss mit dem zweiten Bereich des Aktivator-Oligonukleotides komplementär binden, damit eine Strangverdrängungsreaktion effektiv stattfinden kann.
- Zweiter Bereich des ersten Primer-Oligonukleotides umfasst einen Polynukleotid-Schwanz, welcher den ersten Bereich des Aktivator-Oligonukleotids spezifisch binden soll
- Zwischen dem ersten Bereich und dem Polynukleotid-Schwanz des zweiten Bereichs des ersten Primer-Oligonukleotides ist jeweils eine zur Blockade der Polymerase-abhängigen Synthese bzw. zur Termination bzw. zum Stopp der Polymerase-abhängigen Synthese führende erste Blockieungs-Einheit integriert, welche die Polymerase am Kopieren des Polynukleotid-Schwanzes hindert.
- Bindung zwischen dem ersten Primer-Oligonukleotid und dem Aktivator-Oligonukleotid soll unter verwendeten isothermalen Bedinungen reversiblel sein. Insgesamt soll unter gewählten Reaktionsbedingungen ein Gleichgewicht zwischen einer einzelsträngigen Form des ersten Primer-Oligonukleotids und einer an das Aktivator-Oligonukleotid komplementär gebundenen Form des ersten Primer-Oligonukleotid bestehen.

Bei der Wahl der Sequenzlänge und Sequenzkomposition des zweiten Primer-Oligonukleotides wurden folgende Aspekte berücksichtigt:
- Die Primer-Länge umfasst 30 Nukleotide. Positionen 5 - 30 der Sequenz des zweiten Primer-Oligonukleotides sind komplementär zum zu erwartenden sequenzspezifischen ersten Primer-Verlängerungsprodukt, welches bereits nach der ersten Primer-Verlängerungsreaktion entsteht. Der Primer-2 kann somit eine Primer-Verlängerungsreaktion für das zweite Primer-Verlängerungsprodukt initiieren.
- Das 3'-Segment (Positionen 23 - 30) des zweiten Primer-Oligonukleotides konkurriert im vorliegenden Beispiel um die Bindung an das erste sequenzspezifische Primer-Verlängerungsprodukt mit dem Aktivator-Oligonukleotid. Dieses 3'-Segment kann zu einer Nukleinsäure-abhängigen Strangverdrängung des entsprechenden Segmentes des Aktivator-Oligonukleotides führen. Bei einer solchen Strangverdrängung kommt das 3'-endständige Nukleotid des zweiten Primer-Oligonukleotides in Kontakt mit dem ersten sequenzspezifischen Primer-Verlängerungsprodukt, so dass die Polymerase eine effektive sequenzspezifische zweite Primer-Verlängerungsreaktion initiieren kann, welche zur Synthese des zweiten sequenzspezifischen Stranges führt. Diese Synthese findet unter gleichzeitiger Strangverdrängung des Aktivator-Oligonukleotides durch die Polymerase statt. Die Länge dieses 3'-Segmentes wurde in diesem Beispiel dermassen gewählt, dass keine Überlappung von komplementären Sequenzabschnitten mit dem ersten Primer-Oligonukleotid stattfinden kann.
- Im vorliegenden Beispiel kann das 5'-Segment (22 Nukleotide, Position 1 bis 22) des zweiten Primer-Oligonukleotides an das 3'-Segment des ersten sequenzspezifischen Primer-Verlängerungsproduktes komplementär binden. Dieses 5'-Segment konkurriert nicht um die Bindung an das erste Primer-Verlängerungsprodukt mit dem Aktivator-Oligonukleotid und führt somit nicht zu einer Strangverdrängung des Aktivator-Oligonukleotides aus der Bindung mit dem ersten Primer-Verlängerungsprodukt. Die Tm dieses 5'-Segmentes des zweiten Primer-Oligonukleotides ist so gewählt, dass eine temperatur-induzierte spontane Strangtrennung zwischen einem ersten sequenzspezifischen Primer-Verlängerungsprodukt und einem zweiten sequenzspezifischen Primer-Verlängerungsprodukt im Falle einer vollständigen Bindung des Aktivator-Oligonukleotides an das erste sequenzspezifische Primer-Verlängerungsprodukt begünstigt wird. Die Trennung der beiden Stränge resultiert in einzelsträngigen Form des 3'-Segments des ersten Primer-Verlängerungsproduktes.

## Patentansprüche

1. Verfahren zur Amplifikation einer Nukleinsäure,das folgende Schritte umfasst:
a) Hybridisierung eines ersten Primer-Oligonukleotids an das 3'-Ende eines Stranges einer zur amplifizierenden Nukleinsäurekette, wobei die zu amplifizierende Nukleinsäurekette eine Zielsequenz umfasst,
wobei das erste Primer-Oligonukleotid umfasst:
• Einen ersten Primer-Bereich im 3'-Segment des ersten Primer-Oligonukleotides, der an einen Strang einer zu amplifizierenden Nukleinsäurekette sequenzspezifisch binden kann
• Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt.
b) Extension des ersten Primer-Oligonukleotids mit Hilfe einer Polymerase zur Bildung eines ersten Primer-Verlängerungsproduktes, das zur Zielsequenz der zu amplifizierenden Nukleinsäurekette (a) komplementär ist,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
• Einen ersten einzelsträngigen Bereich, welcher an den Polynukleotid-Schwanz des zweiten Bereichs des ersten Primer-Oligonukleotides binden kann
• Einen zweiten einzelsträngigen Bereich, welcher zu dem ersten Bereich des ersten Primer-Oligonukleotides im Wesentlichen komplementär ist und an diesen binden kann
• Einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette bis dieser komplementärer Strang der zu amplifizierenden Nukleinsäure vom ersten Primer-Verlängerungsprodukt abgelöst wird, wobei das 3'-Segment des ersten Primer-Verlängerungsproduktes einzelsträngig wird
f) Hybridisierung eines zweiten Oligonukleotid-Primers an das erste Primer-Verlängerungsprodukt, wobei das 3'-Segment des zweiten Oligonukleotid-Primers eine Ziel-Bindungssequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist.

2. Verfahren nach Anspruch 1, **dadurch gekennzeichnet, dass** das Verfahren im Wesentlichen isothermal bei einer Temperatur zwischen 15°C und 75°C durchgeführt wird.

3. Verfahren nach einem der Ansprüche 1 oder 2, **dadurch gekennzeichnet, dass** der dritte einzelsträngige Bereich des Aktivator-Oligonukleotids zum Segment des von der Polymerase synthetisierten Verlängerungsproduktes des ersten Primer-Verlängerungsprodukts, welches sich unmittelbar an den ersten Primer-Bereich anschließt, im Wesentlichen komplementär ist.

4. Verfahren nach Anspruch 1, **dadurch gekennzeichnet, dass** Schritt (f) des Verfahrens dahingehend modifiziert wird, dass es umfasst die Hybridisierung eines zweiten Oligonukleotid-Primers an das erste Primer-Verlängerungsprodukt, wobei gleichzeitig zumindest eine partielle Verdrängung des Aktivator-Oligonukleotides aus der Bindung mit dem ersten Verlängerungsprodukt durch Strangverdrängung erfolgt.

5. Verfahren nach Anspruch 1, **dadurch gekennzeichnet, dass** Schritt (g) des Verfahrens so modifiziert wird, dass er eine Verdrängung des Aktivator-Oligonukleotides aus der Bindung mit dem ersten Primer-Verlängerungsprodukt unter Mitwirkung der Polymerase umfasst.

6. Verfahren nach Anspruch 1, **dadurch gekennzeichnet, dass** Schritt (h) des Verfahrens dahingehend modifiziert wird, dass es die Bindung des Aktivator-Oligonukleotides an den unkopierten Polynukleotid-Schwanz des ersten verlängerten Primer-Oligonukleotids und eine Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oiigonukleotides umfasst.

7. Verfahren nach Anspruch 1, **dadurch gekennzeichnet, dass** die Wiederholung der Schritte unter solchen Bedingungen durchgeführt wird, die die Wiederholung der Schritte (a) bis (g) zulassen.

8. Verfahren nach einem der vorhergehenden Ansprüche, **dadurch gekennzeichnet, dass** es die gleichzeitige Amplifikation des ersten und zweiten Primer-Verlängerungsproduktes in einer exponentiellen Reaktion unter Verwendung der ersten und zweiten Primer-Oligonukleotide und des Aktivator-Oligonukleotides umfasst, wobei die gebildeten Primer-Verlängerungsprodukte als Matrizen für die gegenseitige Synthese fungieren.

9. Kit zur Durchführung eines Verfahrens nach einem der Ansprüche 1 bis 8, **dadurch gekennzeichnet, dass** es wenigstens ein erstes Primer-Oligonukleotid, wenigstens ein Aktivator-Oligonukleotid und wenigstens eine Polymerase zur isothermalen Amplifikation enthält.

10. Kit nach Anspruch 9, **dadurch gekennzeichnet, dass** es weiterhin einen zweiten Oligonukleotid-Primer enthält.


SEQUENCE LISTING

<110>  AGCT GmbH

<120>  Verfahren zur Amplifikation von Nukleinsäuren und Kit zu dessen
       Durchführung

<130>  pep02791Gen

<160>  34

<170>  PatentIn version 3.5

<210>  1
<211>  59
<212>  DNA
<213>  artificial sequence

<220>
<223>  target

<400>  1
ccgaagctcg caggaactca gagtgtggag aggacgatag ctagtcagat gatgaagtt        59


<210>  2
<211>  48
<212>  DNA
<213>  artificial sequence

<220>
<223>  complementary

<400>  2
catcatctga ctagctatcg tcctctccac actctgagtt cctgcgag                    48


<210>  3
<211>  45
<212>  DNA
<213>  artificial sequence

<220>
<223>  variant

<400>  3
catcatctga ctagctatcg tcctctccac actctgagtt cctgc                       45


<210>  4
<211>  42
<212>  DNA
<213>  artificial sequence

<220>
<223>  activator oligonucleotide

<400>  4
cguccucucc acacucugag uuccugcgag cttcggtaca ag                          42


<210>  5
<211>  58

<212>  DNA
<213>  artificial sequence

<220>
<223>  activator oligonucleotide

<400>  5
aaaacaaacu agcuaucguc cucuccacac ucugaguucc ugcgagcttc ggtacaag          58


<210>  6
<211>  50
<212>  DNA
<213>  artificial sequence

<220>
<223>  activator oligonucleotide

<400>  6
ctagctatcg tcctctccac acucugaguu ccugcgagct tcggtacaag                  50


<210>  7
<211>  56
<212>  DNA
<213>  artificial sequence

<220>
<223>  negative control

<400>  7
auucaaaugu guucucaacg uccucuacuc auguuccugc gagcttcggt acaaaa            56


<210>  8
<211>  55
<212>  DNA
<213>  artificial sequence

<220>
<223>  A6 8510

<400>  8
tttttctagc tatcgtcctc tccacactct gagttcctgc gagcttcggt acaag             55


<210>  9
<211>  55
<212>  DNA
<213>  artificial sequence

<220>
<223>  A6 8512

<400>  9
tttttctagc tatcgtcatc tccacactct gagttactgc gagcttcggt acaag             55


<210>  10
<211>  53
<212>  DNA
<213>  artificial sequence

<220>
<223>  A6-8514-1

<400>  10
tttttctagc tatcgtcctc tccacactga gttcctgcga gcttcggtac aag          53


<210>  11
<211>  20
<212>  DNA
<213>  artificial sequence

<220>
<223>  primer

<400>  11
gtaccgaagc tcgcaggaac                                               20


<210>  12
<211>  48
<212>  DNA
<213>  artificial sequence

<220>
<223>  target

<400>  12
atactacaat gtcacttacg tcctctccac actctgagtt cctgcgag                48


<210>  13
<211>  20
<212>  DNA
<213>  artificial sequence

<220>
<223>  primer

<400>  13
gtaccgaagc tcgcaggaac                                               20


<210>  14
<211>  27
<212>  DNA
<213>  artificial sequence

<220>
<223>  second primer

<400>  14
aaaaaaacat actacaatgt cacttac                                       27


<210>  15
<211>  50
<212>  DNA
<213>  artificial sequence

<220>
<223>  activator oligonucleotide

<400>  15
cuagcuaucg uccucuccac acucugaguu cctgcgagct tcggtacaag                50


<210>  16
<211>  57
<212>  DNA
<213>  artificial sequence

<220>
<223>  elongation product

<400>  16
gtaccgaagc tcgcaggaac tcagagtgtg gagaggacgt aagtgacatt gtagtat        57


<210>  17
<211>  56
<212>  DNA
<213>  artificial sequence

<220>
<223>  second primer elongation product

<400>  17
aaaaaaacat actacaatgt cacttacgtc ctctccacac tctgagttcc tgcgag         56


<210>  18
<211>  65
<212>  DNA
<213>  artificial sequence

<220>
<223>  amplification product

<400>  18
gtaccgaagc tcgcaggaac tcagagtgtg gagaggacgt aagtgacatt gtagtatgtt     60

ttttt                                                                 65


<210>  19
<211>  56
<212>  DNA
<213>  artificial sequence

<220>
<223>  amplification product

<400>  19
gagcgtcctt gagtctcaca cctctcctgc attcactgta acatcataca aaaaaa         56


<210>  20
<211>  65
<212>  DNA
<213>  artificial sequence

<220>
<223>  amplification product

<400>  20

gtaccgaagc tcgcaggaac tcagagtgtg gagaggacgt aagtgacatt gtagtatgtt 60

ttttt 65

<210> 21
<211> 44
<212> DNA
<213> artificial product

<220>
<223> target

<400> 21
gtagttctca cttacgtcct ctccacactc tgagttcctg cgag 44

<210> 22
<211> 41
<212> DNA
<213> artificial product

<220>
<223> target with one mismatch

<400> 22
gtagttctca cttacgtcct ctccctctga gttcctgcga g 41

<210> 23
<211> 7
<212> DNA
<213> artificial sequence

<220>
<223> first primer

<400> 23
caggaac 7

<210> 24
<211> 20
<212> DNA
<213> artificial sequence

<220>
<223> second primer

<400> 24
cacgagtagt tctcacttac 20

<210> 25
<211> 66
<212> DNA
<213> artificial sequence

<220>
<223> activator oligonucleotide

<400> 25
ctagctatcg tcctctccac actctgagtt cctgcgagct tcggtacaag cgagcttcgg 60

tacaag                                                                    66

<210>  26
<211>  49
<212>  DNA
<213>  artificial sequence

<220>
<223>  MIP Gene

<400>  26
ctacaatgtc acttacacgt tcttaacaag tttcagccgt tcctgcgag                     49

<210>  27
<211>  44
<212>  DNA
<213>  artificial sequence

<220>
<223>  target with one mismatch

<400>  27
ctacaatgtc acttacacgt tcttaacaag tttgttcctg cgag                          44

<210>  28
<211>  33
<212>  DNA
<213>  artificial sequence

<220>
<223>  second primer oligonucleotide

<400>  28
ctacaatgtc acttacacgt tcttaacaag ttt                                      33

<210>  29
<211>  73
<212>  DNA
<213>  artificial sequence

<220>
<223>  activator oligonucleotide

<400>  29
aaaacaaaaa ccgaacaaca aaugaaagac guucuuaaca aguuucagcc guuccugcga         60

gcttcggtac aag                                                            73

<210>  30
<211>  11
<212>  DNA
<213>  artificial sequence

<220>
<223>  first primer

<400>  30

ctcgcaggaa c                                                              11

<210>  31
<211>  7
<212>  DNA
<213>  artificial sequence

<220>
<223>  first primer variant

<400>  31
caggaac                                                                   7

<210>  32
<211>  10
<212>  DNA
<213>  artificial sequence

<220>
<223>  first primer variant

<400>  32
tcgcaggaac                                                               10

<210>  33
<211>  30
<212>  DNA
<213>  artificial sequence

<220>
<223>  second primer

<400>  33
gctcatacta caatgtcact tacgtcctct                                         30

<210>  34
<211>  41
<212>  DNA
<213>  artificial sequence

<220>
<223>  elongation product

<400>  34
tcagagtgtg gagaggacgt aagtgacatt gtagtatgag c                            41